# EUROPÄISCHE PATENTSCHRIFT

(11) **EP 1 163 240 B1**
(45) Veröffentlichungstag und Bekanntmachung des Hinweises auf die Patenterteilung: **14.03.2007**
(21) Anmeldenummer: 00915171.3
(22) Anmeldetag: 08.03.2000
(51) Int. Cl.: C07D 413/06, C07D 498/04, C07D 495/04, C07D 491/04, A01N 43/90, A01N 43/80, C07D 261/20

(54) **TRICYCLISCHE BENZOYLPYRAZOL-DERIVATE ALS HERBIZIDE**
TRICYCLIC BENZOYLPYRAZOLE DERIVATIVES USED AS A HERBICIDE
DERIVES DE BENZOYLPYRAZOL TRICYCLIQUES S'UTILISANT COMME HERBICIDES

(30) Priorität: 12.03.1999 DE 19911219
(43) Veröffentlichungstag der Anmeldung: 19.12.2001
(73) Patentinhaber: BASF Aktiengesellschaft, 67056 Ludwigshafen (DE)
(72) Erfinder: WITSCHEL, Matthias, D-67061 Ludwigshafen (DE); KUDIS, Steffen, D-68167 Mannheim (DE); LANGEMANN, Klaus, D-67551 Worms (DE); BAUMANN, Ernst, D-67373 Dudenhofen (DE); VON DEYN, Wolfgang, D-67435 Neustadt (DE); MAYER, Guido, D-67433 Neustadt (DE); MISSLITZ, Ulf, D-67433 Neustadt (DE); NEIDLEIN, Ulf, D-68165 Mannheim (DE); OTTEN, Martina, D-67069 Ludwigshafen (DE); WESTPHALEN, Karl-Otto, D-67346 Speyer (DE); WALTER, Helmut, D-67283 Obrigheim (DE)
(86) Internationale Anmeldenummer: PCT/EP2000/002010
(87) Internationale Veröffentlichungsnummer: WO 2000/055158

(56) Entgegenhaltungen:
- EP-A- 0 365 201
- EP-A- 0 860 441
- WO-A-97/08164
- WO-A-97/19087
- US-A- 5 049 564
- DATABASE CHEMABS [Online] CHEMICAL ABSTRACTS SERVICE, COLUMBUS, OHIO, US; KUBOTA, MINEYUKI ET AL: "Preparation of pyrazoles and their use as herbicides" retrieved from STN Database accession no. 129:41126 XP002142547 & JP 10 130267 A (IDEMITSU KOSAN CO., LTD., JAPAN) 19. Mai 1998 (1998-05-19)
- DATABASE COSSFIRE [Online] Beilstein Informationssysteme GmbH; XP002142548 & JOURNAL OF PHARMACEUTICAL SCIENCES, Bd. 82, Nr. 5, 1993, Seiten 521-525, AMERICAN PHARMACEUTICAL ASSOCIATION. WASHINGTON., US ISSN: 0022-3549
- DATABASE CROSSFIRE [Online] Beilstein Informationssysteme GmbH; XP002142549 & SYNTHETIC COMMUNICATIONS, Bd. 18, Nr. 5, 1988, Seiten 481-486, MARCEL DEKKER, INC., BASEL., CH ISSN: 0039-7911

## Beschreibung

Die vorliegende Erfindung betrifft neue tricyclische Benzoylpyrazol-Derivate der Formel I 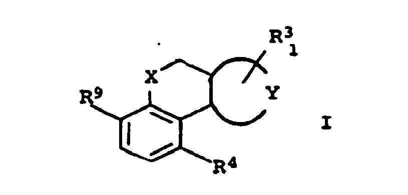 in der die Variablen folgende Bedeutungen haben:
- X: Sauerstoff, Schwefel oder eine Bindung;
- Y: bildet gemeinsam mit den beiden Kohlenstoffen, an die es gebunden ist,
nachfogende Heterocyclen aus : 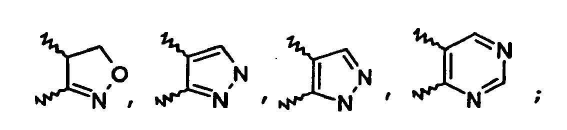
- R³: C₁-C₆-Alkyl;
- R⁴: Halogen, C₁-C₆-Alkoxy, C₁-C₆-Alkylthio, oder C₁-C₆-Alkylsulfonyl;
- l: 0 oder 1;
- R⁹: ein Rest IIa 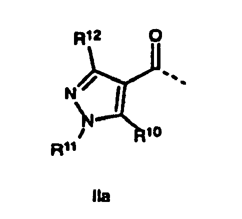 wobei
- R¹⁰: Hydroxy oder OR¹³,
- R¹¹: C₁-C₆-Alkyl oder C₃-C₆-Cycloalkyl;
- R¹²: Wasserstoff oder C₁-C₆-Alkyl;
- R¹³: Phenylcarbonyl, wobei der Phenyl-Rest partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
sowie deren landwirtschaftlich brauchbaren Salze.

Außerdem betrifft die Erfindung Verfahren und Zwischenprodukte zur Herstellung von Verbindungen der Formel I, Mittel welche diese enthalten, sowie die Verwendung dieser Derivate oder diese enthaltende Mittel zur Schadpflanzenbekämpfung.

Aus WO 97/19087 und EP-A 860 441 sind tricyclische Verbindungen bekannt, die dadurch charakterisiert sind, daß die jeweils enthaltene Benzoyleinheit über die Positionen 3 und 4 mit einem

Bicyclus anelliert ist. Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, biologisch, insbesondere herbizid wirksame, Verbindungen mit verbesserten Eigenschaften zu finden.

Demgemäß wurden die tricyclischen Benzoylpyrazol-Derivate der

Formel I sowie deren herbizide Wirkung gefunden.

Ferner wurden Verfahren und Zwischenprodukte zur Synthese der Verbindungen der Formel I gefunden. Ebenso wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im Allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)aulfonium und Sulfoxoniumionen, vorzugsweise Tri(C₁-C₄-alkyl)sulfoxonium, in Betracht.

Anionen von brauchbaren Saureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Im Falle von R¹⁰ = Hydroxy steht IIa auch stellvertretend für die tautomeren Formen IIa' und IIa' '

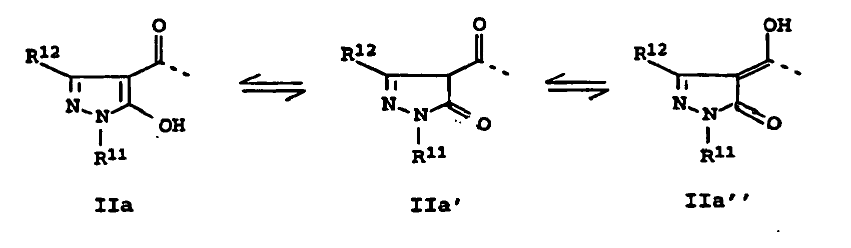

Die für die Substituenten R³, R⁴, R⁹- R¹³,R¹⁷ oder als Reste an Phenyl- und Heterocyclyl-Resten genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Hydroxyalkyl, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, N-Alkylaminosulfonyl-, N,N-Dialkylaminosulfonyl-, N-Alkylamino-, N,N-Dialkylamino-, N-Halogenalkylamino-, N,N-Dihalogenalkylamino, N-Alkylsulfonylamino-, N-Halogenalkylsulfonylamino-, N-Alkyl-N-alkylsulfonylamino-, N-Alkyl-N-halogenalkylsulfonylamino-, Alkylcarbonyl-, Alkoxycarbonyl-, Halogenalkyoxycarbonyl, Alkylthiocarbonyl-, Alkylcarbonyloxy-, Alkylaminocarbonyl-, Dialkylaminocarbonyl-, Dialkylaminothiocarbonyl-, Alkoxyalkyl-, Hydroxyalkoxyalkyl, Alkylcarbonylalkyl-, Alkoxyiminoalkyl-, N-(Alkylamino)-iminoalkyl-, N-(Dialkylamino)-iminoalkyl-, Phenylalkenylcarbonyl-, Heterocyclylalkenylcarbonyl-, N-Alkoxy-N-alkylaminocarbonyl-, N-Alkyl-N-phenylaminocarbonyl-, N-Alkyl-N-heterocyclylaminocarbonyl-, Phenylalkyl-, Heterocyclylalkyl-, Phenylcarbonylalkyl-, Heterocyclylcarbonylalkyl-, Dialkylaminoalkoxycarbonyl-, Alkoxyalkoxycarbonyl-, Alkenylcarbonyl-, Alkenyloxycarbonyl-, Alkenylaminocarbonyl-, N-Alkenyl-N-alkylaminocarbonyl-, N-Alkenyl-N-alkoxyaminocarbonyl-, Alkinylcarbonyl-, Alkinyloxycarbonyl-, Alkinylaminocarbonyl-, N-Alkinyl-N-alkylaminocarbonyl-, N-Alkinyl-N-alkoxyaminocarbonyl-, Alkenyl-, Alkinyl-, Halogenalkenyl-, Halogenalkinyl-, Alkenyloxy- und Alkinyloxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl sowie die Alkylteile von Hydroxy-C₁-C₄-alkyl: z.B. Methyl, Ethyl, Propyl, 1-Methylethyl, Butyl, 1-Methylpropyl, 2-Methylpropyl oder 1,1-Dimethylethyl;
- C₁-C₆-Alkyl, sowie die Alkylteile von C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl, N-(Di-C₁-C₆-alkylamino)-imino-C₁-C₆-alkyl, N(C₁-C₆-Alkoxy)-N-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkyl)-N-phenylaminocarbonyl, N-(C₁-C₆-Alkyl)-N-heterocyclylaminocarbonyl, Phenyl-C₁-C₆-alkyl, N-(C₁-C₆-Alkyl)-N-(C₁-C₆-alkylsulfonyl)-amino, N-(C₁-C₆-Alkyl)-N-(C₁-C₆-halogenalkylsulfonyl)-amino, Heterocyclyl-C₁-C₆-alkyl, Phenylcarbonyl-C₁-C₆-alkyl, Heterocyclylcarbonyl-C₁-C₆-alkyl: C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
   C₁-C₄-Halogenalkyl: einen C₁-C₄-Alkylrest, wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Halogenalkyl, sowie die Halogenalkylteile von N-C₁-C₆-Halogenalkylamino und N,N-(Di-C₁-C₆-halogenalkyl)amino: C₁-C₄-Halogenalkyl, wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₄-Alkoxy: z.B. Methoxy, Ethoxy, Propoxy, 1-Methylethoxy, Butoxy, 1-Methylpropoxy, 2-Methylpropoxy oder 1,1-Dimethylethoxy;
- C₁-C₆-Alkoxy, sowie die Alkoxyteile von C₁-C₆-Alkoxyimino-C₁-C₆-alkyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl und N-(C₃-C₆-Alkinyl)-N-(C₁-C₆-alkoxy)-aminocarbonyl: C₁-C₄-Alkoxy, wie voranstehend genannt, sowie z.B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
   C₁-C₄-Halogenalkoxy: einen C₁-C₄-Alkoxyrest, wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
- C₁-C₆-Halogenalkoxy: C₁-C₄-Halogenalkoxy, wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy;
- C₁-C₄-Alkylthio: z.B. Methylthio, Ethylthio, Propylthio, 1-Methylethylthio, Butylthio, 1-Methylpropylthio, 2-Methylpropylthio oder 1,1-Dimethylethylthio;
- C₁-C₆-Alkylthio, sowie die Alkylthioteile von C₁-C₆-Alkylthiocarbonyl: C₁-C₄-Alkylthio, wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₆-Halogenalkylthio: einen C₁-C₆-Alkylthiorest, wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio, Nonafluorbutylthio, 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio oder Dodecafluorhexylthio;
- C₁-C₆-Alkylsulfinyl (C₁-C₆-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-methylpropylsulfinyl;
   C₁-C₆-Halogenalkylsulfinyl: C₁-C₆-Alkylsulfinylrest, wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl oder Dodecafluorhexylsulfinyl;
   C₁-C₆-Alkylsulfonyl (C₁-C₆-Alkyl-S(=O)₂-), sowie die Alkylsulfonylreste von N-(C₁-C₆-Alkylsulfonyl)-amino und N-(C₁-C₆-Alkyl)-N-(C₁-C₆-alkylsulfonyl)-amino: z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl;
   C₁-C₆-Halogenalkylsulfonyl, sowie die Halogenalkylsulfonylreste von N-(C₁-C₆-Halogenalkylsulfonyl)-amino und N-(C₁-C₆-Alkyl)-N-(C₁-C₆-halogenalkylsulfonyl)-amino: einen C₁-C₆-Alkylsulfonylrest, wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl oder Dodecafluorhexylsulfonyl;
   C₁-C₆-Alkylamino, sowie die Alkylaminoreste von N-(C₁-C₆-Alkylamino)-imino-C₁-C₆-alkyl, also z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
   (C₁-C₆-Alkylamino)sulfonyl: z.B. Methylaminosulfonyl, Ethylaminosulfonyl, Propylaminosulfonyl, 1-Methylethylaminosulfonyl, Butylaminosulfonyl, 1-Methylpropylaminosulfonyl, 2-Methylpropylaminosulfonyl, 1,1-Dimethylethylaminosulfonyl, Pentylaminosulfonyl, 1-Methylbutylaminosulfonyl, 2-Methylbutylaminosulfonyl, 3-Methylbutylaminosulfonyl, 2,2-Dimethylpropylaminosulfonyl, 1-Ethylpropylaminosulfonyl, Hexylaminosulfonyl, 1,1-Dimethylpropylaminosulfonyl, 1,2-Dimethylpropylaminosulfonyl, 1-Methylpentylaminosulfonyl, 2-Methylpentylaminosulfonyl, 3-Methylpentylaminosulfonyl, 4-Methylpentylaminosulfonyl, 1,1-Dimethylbutylaminosulfonyl, 1,2-Dimethylbutylaminosulfonyl, 1,3-Dimethylbutylaminosulfonyl, 2,2-Dimethylbutylaminosulfonyl, 2,3-Dimethylbutylaminosulfonyl, 3,3-Dimethylbutylaminosulfonyl, 1-Ethylbutylaminosulfonyl, 2-Ethylbutylaminosulfonyl, 1,1,2-Trimethylpropylaminosulfonyl, 1,2,2-Trimethylpropylaminosulfonyl, 1-Ethyl-1-methylpropylaminosulfonyl oder 1-Ethyl-2-methylpropylaminosulfonyl;
   Di-(C₁-C₆-alkyl)-aminosulfonyl: z.B. N,N-Dimethylaminosulfonyl, N,N-Diethylaminosulfonyl, N,N-Di-(1-methylethyl)aminosulfonyl, N,N-Dipropylaminosulfonyl, N,N-Dibutylaminosulfonyl, N,N-Di-(1-methylpropyl)-aminosulfonyl, N,N-Di-(2-methylpropyl)-aminosulfonyl, N,N-Di-(1,1-dimethylethyl)-aminosulfonyl, N-Ethyl-N-methylaminosulfonyl, N-Methyl-N-propylaminosulfonyl, N-Methyl-N-(1-methylethyl)-aminosulfonyl, N-Butyl-N-methylaminosulfonyl, N-Methyl-N-(1-methylpropyl)-aminosulfonyl, N-Methyl-N-(2-methylpropyl)-aminosulfonyl, N-(1,1-Dimethylethyl)-N-methylaminosulfonyl, N-Ethyl-N-propylaminosulfonyl, N-Ethyl-N-(1-methylethyl)-aminosulfonyl, N-Butyl-N-ethylaminosulfonyl, N-Ethyl-N-(1-met hylpropyl)-aminosulfonyl, N-Ethyl-N-(2-methylpropyl)-aminosulfonyl, N-Ethyl-N-(1,1-dimethylethyl)-aminosulfonyl, N-(1-Methylethyl)-N-propylaminosulfonyl, N-Butyl-N-propylaminosulfonyl, N-(1-Methylpropyl)-N-propylaminosulfonyl, N-(2-Methylpropyl)-N-propylaminosulfonyl, N-(1,1-Dimethylethyl)-N-propylaminosulfonyl, N-Butyl-N-(1-methylethyl)-aminosulfonyl, N-(1-Methylethyl)-N-(1-methylpropyl)-aminosulfonyl, N-(1-Methylethyl)-N-(2-methylpropyl)-aminosulfonyl, N-(1,1-Dimethylethyl)-N-(1-methylethyl)-aminosulfonyl, N-Butyl-N-(1-methylpropyl)-aminosulfonyl, N-Butyl-N-(2-methylpropyl)-aminosulfonyl, N-Butyl-N-(1,1-dimethylethyl)-aminosulfonyl, N-(1-Methylpropyl)-N-(2-methylpropyl)-aminosulfonyl, N-(1,1-Dimethylethyl)-N-(1-methylpropyl)-aminosulfonyl, N-(1,1-Dimethylethyl)-N-(2-methylpropyl)-aminosulfonyl, N-Methyl-N-pentylaminosulfonyl, N-Methyl-N-(1-methylbutyl)-aminosulfonyl, N-Methyl-N-(2-methylbutyl)-aminosulfonyl, N-Methyl-N-(3-methylbutyl)-aminosulfonyl, N-Methyl-N-(2,2-dimethylpropyl)-aminosulfonyl, N-Methyl-N-(1-ethylpropyl)-aminosulfonyl, N-Methyl-N-hexylaminosulfonyl, N-Methyl-N-(1,1-dimethylpropyl)-aminosulfonyl, N-Methyl-N-(1,2-dimethylpropyl)-aminosulfonyl, N-Methyl-N-(1-methylpentyl)-aminosulfonyl, N-Methyl-N-(2-methylpentyl)-aminosulfonyl, N-Methyl-N-(3-methylpentyl)-aminosulfonyl, N-Methyl-N-(4-methylpentyl)-aminosulfonyl, N-Methyl-N-(1,1-dimethylbutyl)-aminosulfonyl, N-Methyl-N-(1,2-dimethylbutyl)-aminosulfonyl, N-Methyl-N-(1,3-dimethylbutyl)-aminosulfonyl, N-Methyl-N-(2,2-dimethylbutyl)-aminosulfonyl, N-Methyl-N-(2,3-dimethylbutyl)-aminosulfonyl, N-Methyl-N-(3,3-dimethylbutyl)-aminosulfonyl, N-Methyl-N-(1-ethylbutyl)-aminosulfonyl, N-Methyl-N-(2-ethylbutyl)-aminosulfonyl, N-Methyl-N-(1,1,2-trimethylpropyl)-aminosulfonyl, N-Methyl-N-(1,2,2-trimethylpropyl)-aminosulfonyl, N-Methyl-N-(1-ethyl-1-methylpropyl)-aminosulfonyl, N-Methyl-N-(1-ethyl-2-methylpropyl)-aminosulfonyl, N-Ethyl-N-pentylaminosulfonyl, N-Ethyl-N-(1-methylbutyl)-aminosulfonyl, N-Ethyl-N-(2-methylbutyl)-aminosulfonyl, N-Ethyl-N-(3-methylbutyl)-aminosulfonyl, N-Ethyl-N-(2,2-dimethylpropyl)-aminosulfonyl, N-Ethyl-N-(1-ethylpropyl)-aminosulfonyl, N-Ethyl-N-hexylaminosulfonyl, N-Ethyl-N-(1,1-dimethylpropyl)-aminosulfonyl, N-Ethyl-N-(1,2-dimethylpropyl)-aminosulfonyl, N-Ethyl-N-(1-methylpentyl)-aminosulfonyl, N-Ethyl-N-(2-methylpentyl)-aminosulfonyl, N-Ethyl-N-(3-methylpentyl)-aminosulfonyl, N-Ethyl-N-(4-methylpentyl)-aminosulfonyl, N-Ethyl-N-(1,1-dimethylbutyl)-aminosulfonyl, N-Ethyl-N-(1,2-dimethylbutyl)-aminosulfonyl, N-Ethyl-N-(1,3-dimethylbutyl)-aminosulfonyl, N-Ethyl-N-(2,2-dimethylbutyl)-aminosulfonyl, N-Ethyl-N-(2,3-dimethylbutyl)-aminosulfonyl, N-Ethyl-N-(3,3-dimethylbutyl)-aminosulfonyl, N-Ethyl-N-(1-ethylbutyl)-aminosulfonyl, N-Ethyl-N-(2-ethylbutyl)-aminosulfonyl, N-Ethyl-N-(1,1,2-trimethylpropyl)-aminosulfonyl, N-Ethyl-N-(1,2,2-trimethylpropyl)-aminosulfonyl, N-Ethyl-N-(1-ethyl-1-methylpropyl)-aminosulfonyl, N-Ethyl-N-(1-ethyl-2-methylpropyl)-aminosulfonyl, N-Propyl-N-pentylaminosulfonyl, N-Butyl-N-pentylaminosulfonyl, N,N-Dipentylaminosulfonyl, N-Propyl-N-hexylaminosulfonyl, N-Butyl-N-hexylaminosulfonyl, N-Pentyl-N-hexylaminosulfonyl oder N,N-Dihexylaminosulfonyl;
   Di-(C₁-C₄-alkyl)amino, sowie die Dialkylaminoreste von Di-(C₁-C₄-alkyl)amino-C₁-C₄-alkoxycarbonyl und N-(Di-C₁-C₄-alkylamino)-imino-C₁-C₆-alkyl, also z.B. N,N-Dimethylamino, N,N-Diethylamino, N,N-Dipropylamino, N,N-Di-(1-methylethyl)-amino, N,N-Dibutylamino, N,N-Di-(1-methylpropyl)amino, N,N-Di-(2-methylpropyl)amino, N,N-Di-(1,1-dimethylethyl)-amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl) amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl) amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)-amino, N-Butyl-N-(2-methylpropyl)amino, N-Butyl-N-(1,1-dimethylethyl)amino, N-(1-Methylpropyl)-N-(2-methylpropyl)-amino, N-(1,1-Dimethylethyl)-N-(1-methylpropyl)-amino oder N-(1,1-Dimethylethyl)-N-(2-methylpropyl)amino;
   Di-(C₁-C₆-alkyl)amino, sowie die Dialkylaminoreste von Di-(C₁-C₆-alkyl)amino-imino-C₁-C₆-alkyl: Di-(C₁-C₄-alkyl)amino wie voranstehend genannt, sowie N,N-Dipentylamino, N,N-Dihexylamino, N-Methyl-N-pentylamino, N-Ethyl-N-pentylamino, N-Methyl-N-hexylamino oder N-Ethyl-N-hexylamino.
- C₁-C₄-Alkylcarbonyl: z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₁-C₆-Alkylcarbonyl, sowie die Alkylcarbonylreste von C₁-C₆-Alkylcarbonyl-C₁-C₆-alkyl: C₁-C₄-Alkylcarbonyl, wie voranstehend genannt, sowie z.B. Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2,-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl;
- C₁-C₂₀-Alkylcarbonyl: C₁-C₆-Alkylcarbonyl, wie voranstehend genannt, sowie Heptylcarbonyl, Octylcarbonyl, Pentadecylcarbonyl oder Heptadecylcarbonyl;
- C₁-C₄-Alkoxycarbonyl, sowie die Alkoxycarbonylteile von Di-(C₁-C₄-alkyl)amino-C₁-C₄-alkoxycarbonyl, also z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- (C₁-C₆-Alkoxy)carbonyl: (C₁-C₄-Alkoxy)carbonyl, wie vorstehend genannt, sowie z.B. Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl;
- C₁-C₆-Halogenalkoxycarbonyl: ein C₁-C₆-Alkoxycarbonylrest, wie voranstehend genannt, der partiell oder vollständig durch Fluor, Chlor, Brom und/oder Iod substituiert ist, also z.B. Fluormethoxycarbonyl, Difluormethoxycarbonyl, Trifluormethoxycarbonyl, Chlordifluormethoxycarbonyl, Bromdifluormethoxycarbonyl, 2-Fluorethoxycarbonyl, 2-Chlorethoxycarbonyl, 2-Bromethoxycarbonyl, 2-Iodethoxycarbonyl, 2,2-Difluorethoxycarbonyl, 2,2,2-Trifluorethoxycarbonyl, 2-Chlor-2-fluorethoxycarbonyl, 2-Chlor-2,2-difluorethoxycarbonyl, 2,2-Dichlor-2-fluorethoxycarbonyl, 2,2,2-Trichlorethoxycarbonyl, Pentafluorethoxycarbonyl, 2-Fluorpropoxycarbonyl, 3-Fluorpropoxycarbonyl, 2-Chlorpropoxycarbonyl, 3-Chlorpropoxycarbonyl, 2-Brompropoxycarbonyl, 3-Brompropoxycarbonyl, 2,2-Difluorpropoxycarbonyl, 2,3-Difluorpropoxycarbonyl, 2,3-Dichlorpropoxycarbonyl, 3,3,3-Trifluorpropoxycarbonyl, 3,3,3-Trichlorpropoxycarbonyl, 2,2,3,3,3-Pentafluorpropoxycarbonyl, Heptafluorpropoxycarbonyl, 1-(Fluormethyl)-2-fluorethoxycarbonyl, 1-(Chlormethyl)-2-chlorethoxycarbonyl, 1-(Brommethyl)-2-bromethoxycarbonyl, 4-Fluorbutoxycarbonyl, 4-Chlorbutoxycarbonyl, 4-Brombutoxycarbonyl, Nonafluorbutoxycarbonyl, 5-Fluorpentoxycarbonyl, 5-Chlorpentoxycarbonyl, 5-Brompentoxycarbonyl, 5-Iodpentoxycarbonyl, 6-Fluorhexoxycarbonyl, 6-Bromhexoxycarbonyl, 6-Iodhexoxycarbonyl oder Dodecafluorhexoxycarbonyl;
- (C₁-C₄-Alkyl)carbonyloxy: Acetyloxy, Ethylcarbonyloxy, Propylcarbonyloxy, 1-Methylethylcarbonyloxy, Butylcarbonyloxy, 1-Methylpropylcarbonyloxy, 2-Methylpropylcarbonyloxy oder 1,1-Dimethylethylcarbonyloxy;
- (C₁-C₄-Alkylamino)carbonyl: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- (C₁-C₆-Alkylamino)carbonyl: (C₁-C₄-Alkylamino)carbonyl, wie vorstehend genannt, sowie z.B. Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl;
- Di-(C₁-C₄-alkyl)-aminocarbonyl: z.B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)-aminocarbonyl, N,N-Di-(2-methylpropyl)-aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)-aminocarbonyl, N-Butyl-N-methylaminocarbonyl, N-Methyl-N-(1-methylpropyl)-aminocarbonyl, N-Methyl-N-(2-methylpropyl)-aminocarbonyl, N-(1,1-Dimethylethyl)-N-methylaminocarbonyl, N-Ethyl-N-propylaminocarbonyl, N-Ethyl-N-(1-methylethyl)-aminocarbonyl, N-Butyl-N-ethylaminocarbonyl, N-Ethyl-N-(1-methylpropyl)-aminocarbonyl, N-Ethyl-N-(2-methylpropyl)-aminocarbonyl, N-Ethyl-N-(1,1-dimethylethyl)-aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)-aminccarbonyl, N-(1-Methylethyl)-N-(1-methylpropyl)-aminocarbonyl, N-(1-Methylethyl)-N-(2-methylpropyl)-aminocarbonyl, N-(1,1-Dimethylethyl)-N-(1-methylethyl)-aminocarbonyl, N-Butyl-N-(1-methylpropyl)-aminocarbonyl, N-Butyl-N-(2-methylpropyl)-aminocarbonyl, N-Butyl-N-(1,1-dimethylethyl)-aminocarbonyl, N-(1-Methylpropyl)-N-(2-methylpropyl)-aminocarbonyl, N-(1,1-Dimethylethyl)-N-(1-methylpropyl)-aminocarbonyl oder N-(1,1-Dimethylethyl)-N-(2-methylpropyl)-aminocarbonyl;
- Di-(C₁-C₆-alkyl)-aminocarbonyl: Di-(C₁-C₄-alkyl)-aminocarbonyl, wie voranstehend genannt, sowie z.B. N-Methyl-N-pentylaminocarbonyl, N-Methyl-N-(1-methylbutyl)-aminocarbonyl, N-Methyl-N-(2-methylbutyl)-aminocarbonyl, N-Methyl-N-(3-methylbutyl)-aminocarbonyl, N-Methyl-N-(2,2-dimethylpropyl)-aminocarbonyl, N-Methyl-N-(1-ethylpropyl)-aminocarbonyl, N-Methyl-N-hexylaminocarbonyl, N-Methyl-N-(1,1-dimethylpropyl)-aminocarbonyl, N-Methyl-N-(1,2-dimethylpropyl)-aminocarbonyl, N-Methyl-N-(1-methylpentyl)-aminocarbonyl, N-Methyl-N-(2-methylpentyl)-aminocarbonyl, N-Methyl-N-(3-methylpentyl)-aminocarbonyl, N-Methyl-N-(4-methylpentyl)-aminocarbonyl, N-Methyl-N-(1,1-dimethylbutyl)-aminocarbonyl, N-Methyl-N-(1,2-dimethylbutyl)-aminocarbonyl, N-Methyl-N-(1,3-dimethylbutyl)-aminocarbonyl, N-Methyl-N-(2,2-dimethylbutyl)-aminocarbonyl, N-Methyl-N-(2,3-dimethylbutyl)-aminocarbonyl, N-Methyl-N-(3,3-dimethylbutyl)-aminocarbonyl, N-Methyl-N-(1-ethylbutyl)-aminocarbonyl, N-Methyl-N-(2-ethylbutyl)-aminocarbonyl, N-Methyl-N-(1,1,2-trimethylpropyl)-aminocarbonyl, N-Methyl-N-(1,2,2-trimethylpropyl)-aminocarbonyl, N-Methyl-N-(1-ethyl-1-methylpropyl)-aminocarbonyl, N-Methyl-N-(1-ethyl-2-methylpropyl)-aminocarbonyl, N-Ethyl-N-pentylaminocarbonyl, N-Ethyl-N-(1-methylbutyl)-aminocarbonyl, N-Ethyl-N-(2-methylbutyl)-aminocarbonyl, N-Ethyl-N-(3-methylbutyl)-aminocarbonyl, N-Ethyl-N-(2,2-dimethylpropyl)-aminocarbonyl, N-Ethyl-N-(1-ethylpropyl)-aminocarbonyl, N-Ethyl-N-hexylaminocarbonyl, N-Ethyl-N-(1,1-dimethylpropyl)-aminocarbonyl, N-Ethyl-N-(1,2-dimethylpropyl)-aminocarbonyl, N-Ethyl-N-(1-methylpentyl)-aminocarbonyl, N-Ethyl-N-(2-methylpentyl)-aminocarbonyl, N-Ethyl-N-(3-methylpentyl)-aminocarbonyl, N-Ethyl-N-(4-methylpentyl)-aminocarbonyl, N-Ethyl-N-(1,1-dimethylbutyl)-aminocarbonyl, N-Ethyl-N-(1,2-dimethylbutyl)-aminocarbonyl, N-Ethyl-N-(1,3-dimethylbutyl)-aminocarbonyl, N-Ethyl-N-(2,2-dimethylbutyl)-aminocarbonyl, N-Ethyl-N-(2,3-dimethylbutyl)-aminocarbonyl, N-Ethyl-N-(3,3-dimethylbutyl)-aminocarbonyl, N-Ethyl-N-(1-ethylbutyl)-aminocarbonyl, N-Ethyl-N-(2-ethylbutyl)-aminocarbonyl, N-Ethyl-N-(1,1,2-trimethylpropyl)-aminocarbonyl, N-Ethyl-N-(1,2,2-trimethylpropyl)-aminocarbonyl, N-Ethyl-N-(1-ethyl-1-methylpropyl)-aminocarbonyl, N-Ethyl-N-(1-ethyl-2-methylpropyl)-aminocarbonyl, N-Propyl-N-pentylaminocarbonyl, N-Butyl-N-pentylaminocarbonyl, N,N-Dipentylaminocarbonyl, N-Propyl-N-hexylaminocarbonyl, N-Butyl-N-hexylaminocarbonyl, N-Pentyl-N-hexylaminocarbonyl oder N,N-Dihexylaminocarbonyl;
- Di-(C₁-C₆-alkyl)-aminothiocarbonyl: z.B. N,N-Dimethylaminothiocarbonyl, N,N-Diethylaminothiocarbonyl, N,N-Di-(1-methylethyl)aminothiocarbonyl, N,N-Dipropylaminothiocarbonyl, N,N-Dibutylaminothiocarbonyl, N,N-Di-(1-methylpropyl)-aminothiocarbonyl, N,N-Di-(2-methylpropyl)-aminothiocarbonyl, N,N-Di-(1,1-dimethylethyl)-aminothiocarbonyl, N-Ethyl-N-methylaminothiocarbonyl, N-Methyl-N-propylaminothiocarbonyl, N-Methyl-N-(1-methylethyl)-aminothiocarbonyl, N-Butyl-N-methylaminothiocarbonyl, N-Methyl-N-(1-methylpropyl)-aminothiocarbonyl, N-Methyl-N-(2-methylpropyl)-aminothiocarbonyl, N-(1,1-Dimethylethyl)-N-methylaminothiocarbonyl, N-Ethyl-N-propylaminothiocarbonyl, N-Ethyl-N-(1-methylethyl)-aminothiocarbonyl, N-Butyl-N-ethylaminothiocarbonyl, N-Ethyl-N-(1-methylpropyl)-aminothiocarbonyl, N-Ethyl-N-(2-methylpropyl)-aminothiocarbonyl, N-Ethyl-N-(1,1-dimethylethyl)-aminothiocarbonyl, N-(1-Methylethyl)-N-propylaminothiocarbonyl, N-Butyl-N-propylaminothiocarbonyl, N-(1-Methylpropyl)-N-propylaminothiocarbonyl, N-(2-Methylpropyl)-N-propylaminothiocarbonyl, N-(1,1-Dimethylethyl)-N-propylaminothiocarbonyl, N-Butyl-N-(1-methylethyl)-aminothiocarbonyl, N-(1-Methylethyl)-N-(1-methylpropyl)-aminothiocarbonyl, N-(1-Methylethyl)-N-(2-methylpropyl)-aminothiocarbonyl, N-(1,1-Dimethylethyl)-N-(1-methylethyl)-aminothiocarbonyl, N-Butyl-N-(1-methylpropyl)-aminothiocarbonyl, N-Butyl-N-(2-methylpropyl)-aminothiocarbonyl, N-Butyl-N-(1,1-dimethylethyl)-aminothiocarbonyl, N-(1-Methylpropyl)-N-(2-methylpropyl)-aminothiocarbonyl, N-(1,1-Dimethylethyl)-N-(1-methylpropyl)-aminothiocarbonyl, N-(1,1-Dimethylethyl)-N-(2-methylpropyl)-aminothiocarbonyl, N-Methyl-N-pentylaminothiocarbonyl, N-Methyl-N-(1-methylbutyl)-aminothiocarbonyl, N-Methyl-N-(2-methylbutyl)-aminothiocarbonyl, N-Methyl-N-(3-methylbutyl)-aminothiocarbonyl, N-Methyl-N-(2,2-dimethylpropyl)-aminothiocarbonyl, N-Methyl-N-(1-ethylpropyl)-aminothiocarbonyl, N-Methyl-N-hexylaminothiocarbonyl, N-Methyl-N-(1,1-dimethylpropyl)-aminothiocarbonyl, N-Methyl-N-(1,2-dimethylpropyl)-aminothiocarbonyl, N-Methyl-N-(1-methylpentyl)-aminothiocarbonyl, N-Methyl-N-(2-methylpentyl)-aminothiocarbonyl, N-Methyl-N-(3-methylpentyl)-aminothiocarbonyl, N-Methyl-N-(4-methylpentyl)-aminothiocarbonyl, N-Methyl-N-(1,1-dimethylbutyl)-aminothiocarbonyl, N-Methyl-N-(1,2-dimethylbutyl)-aminothiocarbonyl, N-Methyl-N-(1,3-dimethylbutyl)-aminothiocarbonyl, N-Methyl-N-(2,2-dimethylbutyl)-aminothiocarbonyl, N-Methyl-N-(2,3-dimethylbutyl)-aminothiocarbonyl, N-Methyl-N-(3,3-dimethylbutyl)-aminothiocarbonyl, N-Methyl-N-(1-ethylbutyl)-aminothiocarbonyl, N-Methyl-N-(2-ethylbutyl)-aminothiocarbonyl, N-Methyl-N-ethyl-N-(1,1,2-trimethylpropyl)-aminothiocarbonyl, N-Methyl-N-(1,2,2-trimethylpropyl)-aminothiocarbonyl, N-Methyl-N-(1-ethyl-1-methylpropyl)-aminothiocarbonyl, N-Methyl-N-(1-ethyl-2-methylpropyl)-aminothiocarbonyl, N-Ethyl-N-pentylaminothiocarbonyl, N-Ethyl-N-(1-methylbutyl)-aminothiocarbonyl, N-Ethyl-N-(2-methylbutyl)-aminothiocarbonyl, N-Ethyl-N-(3-methylbutyl)-aminothiocarbonyl, N-Ethyl-N-(2,2-dimethylpropyl)-aminothiocarbonyl, N-Ethyl-N-(1-ethylpropyl)-aminothiocarbonyl, N-Ethyl-N-hexylaminothiocarbonyl, N-Ethyl-N-(1,1-dimethylpropyl)-aminothiocarbonyl, N-Ethyl-N-(1,2-dimethylpropyl)-aminothiocarbonyl, N-Ethyl-N-(1-methylpentyl)-aminothiocarbonyl, N-Ethyl-N-(2-methylpentyl)-aminothiocarbonyl, N-Ethyl-N-(3-methylpentyl)-aminothiocarbonyl, N-Ethyl-N-(4-methylpentyl)-aminothiocarbonyl, N-Ethyl-N-(1,1-dimethylbutyl)-aminothiocarbonyl, N-Ethyl-N-(1,2-dimethylbutyl)-aminothiocarbonyl, N-Ethyl-N-(1,3-dimethylbutyl)-aminothiocarbonyl, N-Ethyl-N-(2,2-dimethylbutyl)-aminothiocarbonyl, N-Ethyl-N-(2,3-dimethylbutyl)-aminothiocarbonyl, N-Ethyl-N-(3,3-dimethylbutyl)-aminothiocarbonyl, N-Ethyl-N-(1-ethylbutyl)-aminothiocarbonyl, N-Ethyl-N-(2-ethylbutyl)-aminothiocarbonyl, N-Ethyl-N-(1,1,2-trimethylpropyl)-aminothiocarbonyl, N-Ethyl-N-(1,2,2-trimethylpropyl)-aminothiocarbonyl, N-Ethyl-N-(1-ethyl-1-methylpropyl)-aminothiocarbonyl, N-Ethyl-N-(1-ethyl-2-methylpropyl)-aminothiocarbonyl, N-Propyl-N-pentylaminothiocarbonyl, N-Butyl-N-pentylaminothiocarbonyl, N,N-Dipentylaminothiocarbonyl, N-Propyl-N-hexylaminothiocarbonyl, N-Butyl-N-hexylaminothiocarbonyl, N-Pentyl-N-hexylaminothiocarbonyl oder N,N-Dihexylaminothiocarbonyl;
- C₁-C₄-Alkoxy-C₁-C₄-alkyl sowie die Alkoxyalkylteile von Hydroxy-C₁-C₄-alkoxy-C₁-C₄-alkyl: durch C₁-C₄-Alkoxy, wie vorstehend genannt, substituiertes C₁-C₄-Alkyl, also z.B. für Methoxymethyl, Ethoxymethyl, Propoxymethyl, (1-Methylethoxy)-methyl, Butoxymethyl, (1-Methylpropoxy)methyl, (2-Methylpropoxy)-methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)-propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(Propoxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(Propoxy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)-butyl, 4-(Propoxy)butyl, 4-(1-Methylethoxy)butyl, 4-(Butoxy)-butyl, 4-(1-Methylpropoxy)butyl, 4-(2-Methylpropoxy)butyl oder 4-(1,1-Dimethylethoxy)butyl;
- C₁-C₄-Alkoxy-C₁-C₄-alkoxy als Alkoxyalkoxyteile von C₁-C₄-Alkoxy-C₁-C₄-alkoxycarbonyl: durch C₁-C₄-Alkoxy, wie vorstehend genannt, substituiertes C₁-C₄-Alkoxy, also z.B. für Methoxymethoxy, Ethoxymethoxy, Propoxymethoxy, (1-Methylethoxy)methoxy, Butoxymethoxy, (1-Methylpropoxy)methoxy, (2-Methylpropoxy)methoxy, (1,1-Dimethylethoxy)methoxy, 2-(Methoxy)ethoxy, 2-(Ethoxy)ethoxy,. 2-(Propoxy)ethoxy, 2-(1-Methylethoxy)ethoxy, 2-(Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(Propoxy)propoxy, 2-(1-Methylethoxy)propoxy, 2-(Butoxy)-propoxy, 2-(1-Methylpropoxy)propoxy, 2-(2-Methylpropoxy)propoxy, 2-(1,1-Dimethylethoxy)propoxy, 3-(Methoxy)-propoxy, 3-(Ethoxy)propoxy, 3-(Propoxy)propoxy, 3-(1-Methylethoxy)propoxy, 3-(Butoxy)propoxy, 3-(1-Methylpropoxy)-propoxy, 3-(2-Methylpropoxy)propoxy, 3-(1,1-Dimethylethoxy)propoxy, 2-(Methoxy)butoxy, 2-(Ethoxy)butoxy, 2-(Propoxy)butoxy, 2-(1-Methylethoxy)butoxy, 2-(Butoxy)-butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy)-butoxy, 3-(Propoxy)butoxy, 3-(1-Methylethoxy)butoxy, 3-(Butoxy)butoxy, 3-(1-Methylpropoxy)butoxy, 3-(2-Methylpropoxy)butoxy, 3-(1,1-Dimethylethoxy)butoxy, 4-(Methoxy)-butoxy, 4-(Ethoxy)butoxy, 4-(Propoxy)butoxy, 4-(1-Methylethoxy)butoxy, 4-(Butoxy)butoxy, 4-(1-Methylpropoxy)butoxy, 4-(2-Methylpropoxy)butoxy oder 4-(1,1-Dimethylethoxy)butoxy;
- C₃-C₆-Alkenyl, sowie die Alkenylteile von C₃-C₆-Alkenylcarbonyl, C₃-C₆-Alkenyloxy, C₃-C₆-Alkenyloxycarbonyl, C₃-C₆-Alkenylaminocarbonyl, N-(C₃-C₆-Alkenyl)-N-(C₁-C₆)alkylaminocarbonyl, N-(C₃-C₆-Alkenyl)-N-(C₁-C₆-alkoxy)aminocarbonyl: z.B. Prop-2-en-1-yl, But-1-en-4-yl, 1-Methylprop-2-en-1-yl, 2-Methyl-prop-2-en-1-yl, 2-Buten-1-yl, 1-Penten-3-yl, 1-Penten-4-yl, 2-Penten-4-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-2-en-1-yl, 1-Ethyl-prop-2-en-1-yl, Hex-3-en-1-yl, Hex-4-en-1-yl, Hex-5-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-2-en-1-yl, 1,2-Dimethyl-but-3-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-2-en-1-yl, 2,3-Dimethyl-but-3-en-1-yl, 3,3-Dimethyl-but-2-en-1-yl, 1-Ethyl-but-2-en-1-yl, 1-Ethyl-but-3-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl oder 1-Ethyl-2-methyl-prop-2-en-1-yl;
- C₂-C₆-Alkenyl, sowie die Alkenylteile von C₂-C₆-Alkenylcarbonyl, Phenyl-C₂-C₆-alkenylcarbonyl und Heterocyclyl-C₂-C₆-alkenylcarbonyl: C₃-C₆-Alkenyl, wie voranstehend genannt, sowie Ethenyl;
- C₂-C₂₀-Alkenyl als Alkenylteil von C₂-C₂₀-Alkenylcarbonyl, C₂-C₆-Alkenyl, wie vorstehend genannt, sowie Pentadecenyl oder Heptadecenyl;
- C₃-C₆-Halogenalkenyl: einen C₃-C₆-Alkenylrest, wie vorstehend genannt, der partiell oder vollständig durch Fluor, Chlor, Brom und/oder Iod substituiert ist, also z.B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- C₃-C₆-Alkinyl, sowie die Alkinylteile von C₃-C₆-Alkinylcarbonyl, C₃-C₆-Alkinyloxy, C₃-C₆-Alkinyloxycarbony, C₃-C₆-Alkinylaminocarbonyl, N-(C₃-C₆-Alkinyl)-N-(C₁-C₆-alkyl)-aminocarbonyl, N-(C₃-C₆-Alkinyl)-N-(C₁-C₆-alkoxyaminocarbonyl: z.B. Propargyl, But-1-in-3-yl, But-1-in-4-yl, But-2-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-3-yl, 3-Methyl-pent-1-in-4-yl, 3-Methyl-pent-1-in-5-yl, 4-Methyl-pent-2-in-4-yl oder 4-Methyl-pent-2-in-5-yl;
- C₂-C₆-Alkinyl, sowie die Alkinylteile von C₂-C₆-Alkinylcarbonyl: C₃-C₆-Alkinyl, wie voranstehend genannt, sowie Ethinyl;
- C₃-C₆-Halogenalkinyl: einen C₃-C₆-Alkinylrest, wie vorstehend genannt, der partiell oder vollständig durch Fluor, Chlor, Brom und/oder Iod substituiert ist, also z.B. 1,1-Difluor-prop-2-in-1-yl, 3-Iod-prop-2-in-1-yl, 4-Fluorbut-2-in-1-yl, 4-Chlorbut-2-in-1-yl, 1,1-Difluorbut-2-in-1-yl, 4-Iod-but-3-in-1-yl, 5-Fluorpent-3-in-1-yl, 5-Iod-pent-4-in-1-yl, 6-Fluor-hex-4-in-1-yl oder 6-Iod-hex-5-in-1-yl;
- C₃-C₆-Cycloalkyl, sowie die Cycloalkylteile von C₃-C₆-Cycloalkylcarbonyl: z.B. Cyclopropyl, Cyclobutyl, Cyclopentyl oder Cyclohexyl;
- Heterocyclyl, sowie Heterocyclylteile von Heterocyclyloxy, Heterocyclylcarbonyl, Heterocyclyl-C₁-C₆-alkyl, Heterocyclyloxycarbonyl, Heterocyclyloxythiocarbonyl, Heterocyclyl-C₂-C₆-alkenylcarbonyl, Heterocyclylcarbonyl-C₁-C₆-alkyl, N-(C₁-C₆-Alkyl)-N-(heterocyclyl)-aminocarbonyl, Heterocyclylaminocarbonyl: ein gesättigter, partiell gesättigter oder ungesättigter 5- oder 6-gliedriger, C-gebundener, heterocyclischer Ring, der ein bis vier gleiche oder verschiedene Heteroatome, ausgewählt aus folgender Gruppe: Sauerstoff, Schwefel oder Stickstoff, enthält, also z.B. 5-gliedrige Ringe mit beispielsweise einem Heteroatom, mit zwei Heteroatomen, mit drei Heteroatomen oder mit vier Heteroatomen oder z.B. 6-gliedrige Ringe mit beispielsweise einem Heteroatom, mit zwei Heteroatomen, mit drei Heteroatomen oder mit vier Heteroatomen, also 5-gliedrige Ringe, mit einem Heteroatom wie:
   Tetrahydrofuran-2-yl, Tetrahydrofuran-3-yl, Tetrahydrothien-2-yl, Tetrahydrothien-3-yl,Tetrahydropyrrol-2-yl, Tetrahydro-pyrrol-3-yl, 2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydrothien-2-yl, 2,3-Dihydrothien-3-yl, 2,5-Dihydrothien-2-yl, 2,5-Dihydro-thien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 2-Furyl, 3-Furyl, 2-Thienyl, 3-Thienyl, Pyrrol-2-yl oder Pyrrol-3-yl;
   5 gliedrige Ringe mit zwei Heteroatomen wie:
   Tetrahydropyrazol-3-yl, Tetrahydropyrazol-4-yl, Tetrahydro-isoxazol-3-yl, Tetrahydroisoxazol-4-yl, Tetrahydroisoxa-zol-5-yl, 1,2-Oxathiolan-3-yl, 1,2-Oxathiolan-4-yl, 1,2-Oxa-thiolan-5-yl, Tetrahydroisothiazol-3-yl, Tetrahydroisothia-zol-4-yl, Tetrahydroisothiazol-5-yl, 1,2-Dithiolan-3-yl, 1,2-Dithiolan-4-yl, Tetrahydroimidazol-2-yl, Tetrahydro-imidazol-4-yl, Tetrahydrooxazol-2-yl, Tetrahydrooxazol-4-yl, Tetrahydrooxazol-5-yl, Tetrahydrothiazol-2-yl, Tetrahydro-thiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihy-droisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydrothiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxathiol-4-yl, 1,3-Oxathiol-5-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl oder Thiazol-5-yl; 5-gliedrige Ringe mit drei Heteroatomen wie:
      1,2,3-Δ²-Oxadiazolin-4-yl, 1,2,3-Δ²-Oxadiazolin-5-yl,
      1,2,4-Δ⁴-Oxadiazolin-3-yl, 1,2,4-Δ⁴-Oxadiazolin-5-yl,
      1,2,4-Δ²-Oxadiazolin-3-yl, 1,2,4-Δ²-Oxadiazolin-5-yl,
      1,2,4-Δ³-Oxadiazolin-3-yl, 1,2,4-Δ³-Oxadiazolin-5-yl,
      1,3,4-Δ²-Oxadiazolin-2-yl, 1,3,4-Δ²-Oxadiazolin-5-yl,
      1,3,4-Δ³-Oxadiazolin-2-yl, 1,3,4-Oxadiazolin-2-yl,
      1,2,3-Δ²-Thiadiazolin-4-yl, 1,2,3-Δ²-Thiadiazolin-5-yl,
      1,2,4-Δ⁴-Thiadiazolin-3-yl, 1,2,4-Δ⁴-Thiadiazolin-5-yl,
      1,2,4-Δ³-Thiadiazolin-3-yl, 1,2,4-Δ³-Thiadiazolin-5-yl,
      1,2,4-Δ²-Thiadiazolin-3-yl, 1,2,4-Δ²-Thiadiazolin-5-yl,
      1,3,4-Δ²-Thiadiazolin-2-yl, 1,3,4-Δ²-Thiadiazolin-5-yl,
      1,3,4-Δ³-Thiadiazolin-2-yl, 1,3,4-Thiadiazolin-2-yl,
      1,3,2-Dioxathiolan-4-yl, 1,2,3-Δ²-Triazolin-4-yl,
      1,2,3-Δ²-Triazolin-5-yl, 1,2,4-Δ²-Triazolin-3-yl,
      1,2,4-Δ²-Triazolin-5-yl, 1,2,4-Δ³-Triazolin-3-yl,
      1,2,4-Δ³-Triazolin-5-yl, 1,2,4-Δ¹-Triazolin-2-yl, 1,2,4-Triazolin-3-yl, 3H-1,2,4-Dithiazol-5-yl, 2H-1,3,4-Dithiazol-5-yl, 2H-1,3,4-Oxathiazol-5-yl, 1,2,3-Oxadiazol-4-yl,
      1,2,3-Oxadiazol-5-yl, 1,2,4-Oxadiazol-3-yl,
      1,2,4,-Oxadiazol-5-yl, 1,3,4-Oxadiazol-2-yl,
      1,2,3-Thiadiazol-4-yl, 1,2,3-Thiadiazol-5-yl,
      1,2,4-Thiadiazol-3-yl, 1,2,4-Thiadiazol-5-yl, 1,3,4-Thiadiazolyl-2-yl, 1,2,3-Triazol-4-yl oder 1,2,4-Triazol-3-yl;
   5-gliedrige Ringe mit vier Heteroatomen wie:
   Tetrazol-5-yl;
   6-gliedrige Ringe mit einem Heteroatom wie:
      Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydropyran-6-yl, 2H-3,4-Dihydrothiopyran-5-yl, 2H-3,4-Dihydrothiopyran-4-yl, 2H-3,4-Dihydropyran-3-yl, 2H-3,4-Dihydropyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetrahydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydrothiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl,
      1,2-Dihydropyridin-3-yl, 1,2-Dihydropyridin-4-yl,
      1,2-Dihydropyridin-5-yl, 1,2-Dihydropyridin-6-yl,
      3,4-Dihydropyridin-2-yl, 3,4-Dihydropyridin-3-yl,
      3,4-Dihydropyridin-4-yl, 3,4-Dihydropyridin-5-yl,
      3,4-Dihydropyridin-6-yl, 2,5-Dihydropyridin-2-yl,
      2,5-Dihydropyridin-3-yl, 2,5-Dihydropyridin-4-yl,
      2,5-Dihydropyridin-5-yl, 2,5-Dihydropyridin-6-yl,
      2,3-Dihydropyridin-2-yl, 2,3-Dihydropyridin-3-yl,
      2,3-Dihydropyridin-4-yl, 2,3-Dihydropyridin-5-yl,
      2,3-Dihydropyridin-6-yl, Pyridin-2-yl, Pyridin-3-yl oder Pyridin-4-yl;
   6-gliedrige Ringe mit zwei Heteroatomen wie:
   1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6-Dihyclro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5,6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydropyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl oder 3,4-Dihydropyrimidin-6-yl, Pyridazin-3-yl, Pyridazin-4-yl, Pyrimidin-2-yl, Pyrimidin-4-yl, Pyrimidin-5-yl oder Pyrazin-2-yl;
   6-gliedrige Ringe mit drei Heteroatomen wie:
   1,3,5-Triazin-2-yl, 1,2,4-Triazin-3-yl, 1,2,4-Triazin-5-yl, 1,2,4-Triazin-6-yl;
   6-gliedrige Ringe mit vier Heteroatomen wie:
   1,2,4,5-Tetrazin-3-yl;
   wobei ggf. der Schwefel der genannten Heterocyclen zu S=O oder S(=O)₂ oxidiert sein kann
   und wobei mit einem ankondensierten Phenylring oder mit einem C₃-C₆-Carbocyclus oder mit einem weiteren 5- bis 6-gliedrigen Heterocyclus ein bicyclisches Ringsystem ausgebildet werden kann.
- N-gebundenes Heterocyclyl: ein gesättigter, partiell gesättigter oder ungesättigter 5- oder 6-gliedriger N-gebundener heterocyclischer Ring, der mindestens einen Stickstoff und gegebenenfalls ein bis drei gleiche oder verschiedene Heteroatome, ausgewählt aus folgender Gruppe: Sauerstoff, Schwefel oder Stickstoff enthält, also z.B.

N-gebundene 5-gliedrige Ringe wie:
Tetrahydropyrrol-1-yl, 2,3-Dihydro-1H-pyrrol-1-yl, 2,5-Dihydro-1H-pyrrol-1-yl, Pyrrol-1-yl, Tetrahydropyrazol-1-yl, Tetrahydroisoxazol-2-yl, Tetrahydroisothiazol-2-yl, Tetrahydroimidazol-1-yl, Tetrahydrooxazol-3-yl, Tetrahydrothiazol-3-yl, 4,5-Dihydro-1H-pyrazol-1-yl, 2,5-Dihydro-1H-pyrazol-1-yl, 2,3-Dihydro-1H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1H-imidazol-1-yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, Pyrazol-1-yl, Imidazol-1-yl, 1,2,4-Δ⁴-Oxadiazolin-2-yl, 1,2,4-Δ²-Oxadiazolin-4-yl, 1,2,4-Δ³-Oxadiazolin-2-yl, 1,3,4-Δ²-Oxadiazolin-4-yl, 1,2,4-Δ⁵-Thiadiazolin-2-yl, 1,2,4-Δ³-Thiadiazolin-2-yl, 1,2,4-Δ²-Thiadiazolin-4-yl, 1,3,4-Δ²-Thiadiazolin-4-yl, 1,2,3-Δ²-Triazolin-1-yl, 1,2,4-Δ²-Triazolin-1-yl, 1,2,4-Δ²-Triazolin-4-yl, 1,2,4-Δ³-Triazolin-1-yl, 1,2,4-Δ¹-Triazolin-4-yl, 1,2,3-Triazol-1-yl, 1,2,4-Triazol-1-yl, Tetrazol-1-yl;
sowie N-gebundene 6-gliedrige Ringe wie:
Piperidin-1-yl, 1,2,3,4-Tetrahydropyridin-1-yl, 1,2,5,6-Tetrahydropyridin-1-yl, 1,4-Dihydropyridin-1-yl, 1,2-Dihydropyridin-1-yl, Hexahydropyrimidin-1-yl, Hexahydropyrazin-1-yl, Hexahydropyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl, Tetrahydro-1,2-oxazin-2-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazin-2-yl, 2H-3,4-Dihydro-1,2-oxazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihdro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl;
sowie N-gebundene cyclische Imide wie:
Phthalsäureimid, Tetrahydrophthalsäureimid, Succinimid, Maleinimid, Glutarimid, 5-Oxo-triazolin-1-yl, 5-Oxo-1,3,4-oxadiazolin-4-yl oder 2,4-Dioxo-(1H,3H)-pyrimidin-3-yl;
wobei mit einem ankondensierten Phenylring oder mit einem C₃-C₆-Carbocyclus oder einem weiteren 5- bis 6-gliedrigen Heterocyclus ein bicyclisches Ringsystem ausgebildet werden kann.

Alle Phenylringe, Heterocyclyl- bzw. N-Heterocyclylreste sowie alle Phenylkomponenten in Phenoxy, Phenyl-C₁-C₆-alkyl, Phenylcarbonyl-C₁-C₆-alkyl, Phenylcarbonyl, Phenylalkenylcarbonyl, Phenoxycarbonyl, Phenyloxythiocarbonyl, Phenylaminocarbonyl und N-(C₁-C₆-Alkyl)-N-phenylaminocarbonyl bzw. Heterocyclylkomponenten in Heterocyclyloxy, Heterocyclyl-C₁-C₆-alkyl, Heterocyclylcarbonyl-C₁-C₆-alkyl, Heterocyclylcarbonyl, Heterocyclyloxythiocarbonyl, Heterocyclylalkenylcarbonyl, Heterocyclyloxycarbonyl, Heterocyclylaminocarbonyl und N(C₁-C₆-Alkyl)-N-heterocyclylaminocarbonyl sind, soweit nicht anders angegeben, vorzugsweise unsubstituiert oder tragen ein bis drei Halogenatome und/oder eine Nitrogruppe, einen Cyanorest und/oder einen oder zwei Methyl-, Trifluormethyl-, Methoxy- oder Trifluormethoxysubstituenten.

Weiterhin steht der Ausdruck "Y bildet gemeinsam mit den beiden Kohlenstoffen, an die es gebunden ist, einen gesättigten, partiell gesättigten oder ungesättigten Heterocyclus, der ein bis drei gleiche oder verschiedene Heteroatome, ausgewählt aus folgender Gruppe: Sauerstoff, Schwefel oder Stickstoff, enthält" beispielsweise für 5-gliedrige Ringe mit einem Heteroatom, wie:
Tetrahydrofurandiyl, Tetrahydrothiendiyl, Tetrahydropyrroldiyl, Dihydrofurandiyl, Dihydrothiendiyl, Dihydropyrroldiyl, Furandiyl, Thiendiyl oder Pyrroldiyl;
oder 5-gliedrige Ringe mit zwei Heteroatomen wie:
Tetrahydropyrazoldiyl, Tetrahydroisoxazoldiyl, 1,2-Oxathiolandiyl, Tetrahydroisothiazoldiyl, 1,2-Dithiolandiyl, Tetrahydroimidazoldiyl, Tetrahydrooxazoldiyl, Tetrahydrothiazoldiyl, 1,3-Dioxolandiyl, 1,3-Oxathiolandiyl, Dihydropyrazoldiyl, Dihydroisoxazoldiyl, Dihydroisothiazoldiyl, 1,2-Dithioldiyl, Dihydroimidazoldiyl, Dihydrooxazoldiyl, Dihydrothiazoldiyl, Dioxoldiyl, Oxathioldiyl, Pyrazoldiyl, Isoxazoldiyl, Isothiazoldiyl, Imidazoldiyl, Oxazoldiyl oder Thiazoldiyl;
oder 5-gliedrige Ringe mit drei Heteroatomen wie:
1,2,3-Oxadiazolindiyl, 1,2,3-Thiadiazolindiyl, 1,2,3-Triazolindiyl, 1,2,3-Oxadiazoldiyl, 1,2,3-Thiadiazoldiyl oder 1,2,3-Triazoldiyl;
oder 6-gliedrige Ringe mit einem Heteroatom wie:
Tetrahydropyrandiyl, Piperidindiyl, Tetrahydrothiopyrandiyl, Dihydropyrandiyl, Dihydrothiopyrandiyl, Tetrahydropyrindindiyl, Pyrandiyl, Thiopyrandiyl, Dihydropyrindiyl oder Pyridindiyl;
oder 6-gliedrige Ringe mit zwei Heteroatomen wie:
1,3-Dioxandiyl, 1,4-Dioxandiyl, 1,3-Dithiandiyl, 1,4-Dithiandiyl, 1,3-Oxathiandiyl, 1,4-Oxathiandiyl, 1,2-Dithiandiyl, Hexahydropyrimidindiyl, Hexahydropyrazindiyl, Hexahydropyridazindiyl, Tetrahydro-1,3-oxazindiyl, Tetrahydro-1,3-thiazindiyl, Tetrahydro-1,4-oxazindiyl, Tetrahydro-1,2-oxazindiyl, Dihydro-1,2-oxazindiyl, Dihydro-1,2-thiazindiyl, Tetrahydropyridazindiyl, Dihydro-1,3-oxazindiyl, Dihydro-1,3-oxazindiyl, Dihydro-1,3-thiazindiyl, Tetrahydropyrimidindiyl, Tetrahydropyrazindiyl, Dihydro-1,4-thiazindiyl, Dibydro-1,4-oxazindiyl, Dihydro-1,4-dioxindiyl, Dihydro-1,4-dithiindiyl, 1,2-Oxaziadiyl, 1,2-Thiazindiyl, 1,3-Oxazindiyl, 1,3-Thiazindiyl, 1,4-Oxaziadiyl, 1,4-Thiazindiyl, Dihydropyridazindiyl, Dihydropyrazindiyl, Dihydropyrimidincliyl, Pyridazindiyl, Pyrimidindiyl oder Pyrazindiyl;
oder 6-gliedrige Ringe mit 3 Heteroatomen wie:
1,2,4-Triazindiyl;
wobei ggf. der Schwefel der genannten Heteroyclen zu S=o oder S(=O)₂ oxidiert sein kann;
und wobei die Anellierung mit dem Grundkörper über zwei benachbarte Kohlenstoffatome erfolgt.

Die erfindungsgemäßen Verbindungen der Formel I mit R⁹ = IIa werden als Verbindungen der Formel Ia bezeichnet.

Bevorzugt werden die Verbindungen der Formel I, wobei
- R¹¹: für C₁-C₆-Alkyl
steht.

Ebenso bevorzugt werden die Verbindungen der Formel Ia.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- X: Sauerstoff, Schwefel oder eine Bindung;
- Y: bildet gemeinsam mit den beiden Kohlenstoffen, an das es gebunden ist,
nachfogende Heterocyclen aus : 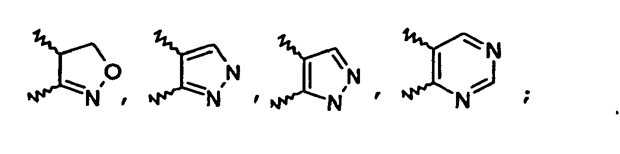
- R³: C₁-C₆-Alkyl ;
- R⁴: Halogen, C₁-C₆-Alkoxy, C₁-C₆-Alkylthio oder C₁-C₆-Alkylsulfonyl;
- l: 0 oder 1 ;
- R⁹: ein Rest IIa 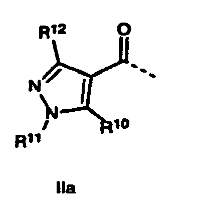 wobei
- R¹⁰: Hydroxy oder OR¹³,
- R¹¹: C₁-C₆-Alkyl oder C₃-C₆-Cycloalkyl;
- R¹²: Wasserstoff oder C₁-C₆-Alkyl ;
- R¹³: Phenylcarbonyl,
wobei der Phenyl-Rest partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy,

Besonders bevorzugt sind Verbindungen der Formel I, wobei die Variablen folgende Bedeutungen haben, und zwar für sich allein oder in Kombination:
- X: Sauerstoff, Schwefel oder eine Bindung;
- Y: bildet gemeinsam mit den beiden Kohlenstoffen, an die es gebunden ist, nachfolgende Heterocyclen aus: (Bei den nachfolgenden Ausführungen der Heterocyclen stellt jeweils die obere Wellenlinie die Verknüpfung zum Kohlenwasserstoff, der die Reste R¹ und R² trägt, und die untere Wellenlinie die Verknüpfung zum meta-Kohlenstoff des Benzoylteils dar). 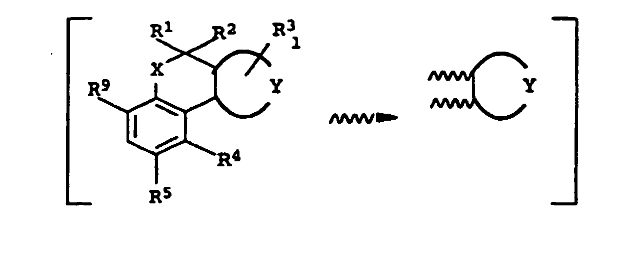 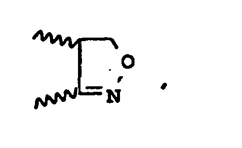 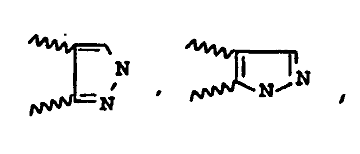 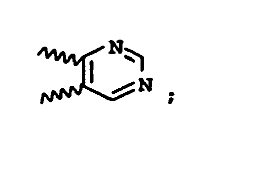 insbesondere bildet Y gemeinsam mit den beiden Kohlenstoffen, an die es gebunden ist, nachfolgende Heterocyclen aus: 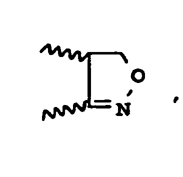 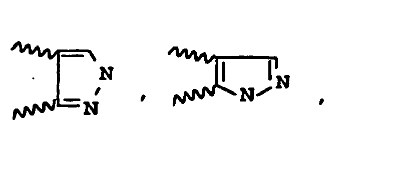 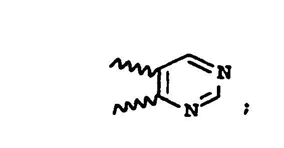
- R³: C₁-C₆-Alkyl, wie Methyl, Ethyl oder n-Propyl; insbesondere Methyl;
- R⁴: Halogen, C₁-C₆-Alkoxy, C₁-C₆-Alkylthio oder C₁-C₆-Alkylsulfonyl; insbesondere Halogen wie Fluor, Chlor oder Brom, C₁-C₆-Alkylthio wie Methylthio oder Ethylthio oder C₁-C₆-Alkylsulfonyl, wie Methylsulfonyl oder Ethylsulfonyl; besonders bevorzugt Chlor, Methylthio oder Methylsulfonyl;
- l: 0 oder 1;
- R⁹: ein Rest IIa 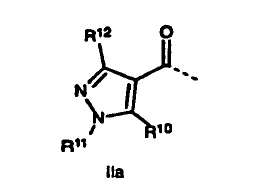 wobei
- R¹⁰: Hydroxy;
- R¹¹: C₁-C₆-Alkyl, wie Methyl, Ethyl, n-Propyl, 1-Methylethyl, n-Butyl, 2-Methylpropyl oder 1.1- Dimethylethyl oder Cyclopropyl;
insbesondere Methyl oder Ethyl; ebenso insbesondere bevorzugt Cyclopropyl;
- R¹²: Wasserstoff oder C₁-C₆-Alkyl, wie Methyl, Ethyl, n-Propyl oder 1-Methylethyl;
insbesondere Wasserstoff oder Methyl;

Insbesondere bevorzugt sind die Verbindungen Ia, wobei
- X: Sauerstoff, Schwefel oder eine Bindung;
- Y: gemeinsam mit den beiden Kohlenstoffen, an die es gebunden ist, folgende Heterocyclen ausbildet: 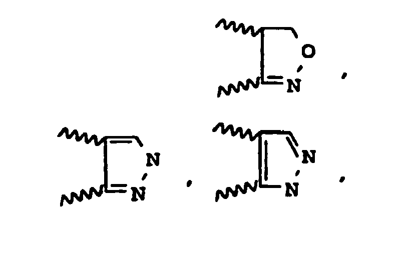 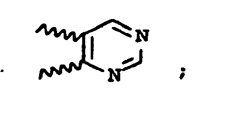
- R³: C₁-C₄-Alkyl;
- R⁴: Halogen, C₁-C₆-Alkoxy, C₁-C₆-Alkylthio oder C₁-C₆-Alkylsulfonyl;
- l: 0 oder 1 ;
- R⁹: ein Rest IIa;
- R¹⁰: Hydroxy;
- R¹¹: C₁-C₆-Alkyl oder Cyclopropyl; insbesondere C₁-C₆-Alkyl;
- R¹²: Wasserstoff oder C₁-C₆-Alkyl ;

Ebenso insbesondere bevorzugt sind die Verbindungen Ia, wobei X für Sauerstoff, Schwefel oder eine Bindung steht. Y bildet gemeinsam mit den beiden Kohlenstoffen, an die es gebunden ist, nachfolgende Hetercyclen aus:

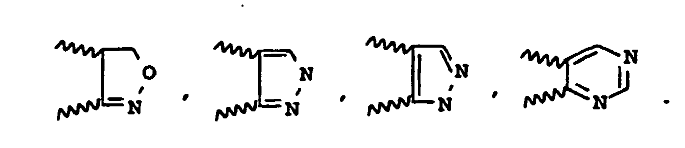

In den Verbindungen der Formel I bedeuten
- R³: C₁-C₆-Alkyl;
- R⁴: Halogen, C₁-C₆-Alkoxy, C₁-C₆-Alkylthio oder C₁-C₆-Alkylsulfonyl;
- l: 0 oder 1;

In den Verbindungen der Formel I bedeuten
- R¹⁰: Hydroxy oder Phenylcarbonyloxy, das unsubstituiert oder partiell oder vollständig halogeniert sein kann und/ oder einen bis drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
insbesondere Hydroxy;
- R¹¹: C₁-C₆-Alkyl oder C₃-C₆-Cycloalkyl; insbesondere C₁-C₆-Alkyl oder ebenso insbesondere Cyclopropyl;
- R¹²: Wasserstoff oder C₁-C₆-Alkyl; insbesondere Wasserstoff;

Ebenso insbesondere bevorzugt sind die Verbindungen der Formel Ia3, Ia5, ...

Die gegebenen Restedefinitionen R³,R⁴, R⁹-R¹², X, Y und l sowie die Bedeutung des anellierten Heterocyclus sind nicht nur in Kombination miteinander, sondern auch für sich allein betrachtet für die erfindungsgemäßen Verbindungen von besonderer Bedeutung. (Aus Gründen der klareren Darstellung gilt jeweils in den Formeln Ia3, Ia5, ... die Bedeutung des anellierten Heterocyclus wie jeweils in der zugehörigen Strukturformel angegeben.)

**Tabelle 1:**

| n | X | R⁴ | R¹⁰ | R¹¹ |
|---|---|---|---|---|
| 1 | Bindung | F | OH | CH₃ |
| 2 | Bindung | Cl | OH | CH₃ |
| 3 | Bindung | Br | OH | CH₃ |
| 5 | Bindung | SCH₃ | OH | CH₃ |
| 6 | Bindung | SO₂CH₃ | OH | CH₃ |
| 7 | Bindung | SO₂CH₂CH₃ | OH | CH₃ |
| 21 | O | F | OH | CH₃ |
| 22 | O | Cl | OH | CH₃ |
| 23 | O | Br | OH | CH₃ |
| 25 | O | SCH₃ | OH | CH₃ |
| 26 | O | SO₂CH₃ | OH | CH₃ |
| 27 | O | SO₂CH₂CH₃ | OH | CH₃ |
| 31 | S | F | OH | CH₃ |
| 32 | s | Cl | OH | CH₃ |
| 33 | S | Br | OH | CH₃ |
| 35 | S | SCH₃ | OH | CH₃ |
| 36 | S | SO₂CH₃ | OH | CH₃ |
| 37 | S | SO₂CH₂CH₃ | OH | CH₃ |
| 51 | Bindung | F | OH | CH₂CH₃ |
| 52 | Bindung | Cl | OH | CH₂CH₃ |
| 53 | Bindung | Br | OH | CH₂CH₃ |
| 55 | Bindung | SCH₃ | OH | CH₂CH₃ |
| 56 | Bindung | SO₂CH₃ | OH | CH₂CH₃ |
| 57 | Bindung | SO₂CH₂CH₃ | OH | CH₂CH₃ |
| 71 | O | F | OH | CH₂CH₃ |
| 72 | O | Cl | OH | CH₂CH₃ |
| 73 | O | Br | OH | CH₂CH₃ |
| 75 | O | SCH₃ | OH | CH₂CH₃ |
| 76 | O | SO₂CH₃ | OH | CH₂CH₃ |
| 77 | O | SO₂CH₂CH₃ | OH | CH₂CH₃ |
| 81 | S | F | OH | CH₂CH₃ |
| 82 | S | Cl | OH | CH₂CH₃ |
| 83 | S | Br | OH | CH₂CH₃ |
| 85 | S | SCH₃ | OH | CH₂CH₃ |
| 86 | S | SO₂CH₃ | OH | CH₂CH₃ |
| 87 | | SO₂CH₂CH₃ | OH | CH₂CH₃ |
| 101 | Bindung | F | OCOC₆H₅ | CH₃ |
| 102 | Bindung | Cl | OCOC₆H₅ | CH₃ |
| 103 | Bindung | Br | OCOC₆H₅ | CH₃ |
| 105 | Bindung | SCH₃ | OCOC₆H₅ | CH₃ |
| 106 | Bindung | SO₂CH₃ | OCOC₆H₅ | CH₃ |
| 107 | Bindung | SO₂CH₂CH₃ | OCOC₆H₅ | CH₃ |
| 121 | O | F | OCOC₆H₅ | CH₃ |
| 122 | O | Cl | OCOC₆H₅ | CH₃ |
| 123 | O | Br | OCOC₆H₅ | CH₃ |
| 125 | O | SCH₃ | OCOC₆H₅ | CH₃ |
| 126 | O | SO₂CH₃ | OCOC₆H₅ | CH₃ |
| 127 | O | SiO₂CH₂CH₃ | OCOC₆H₅ | CH₃ |
| 131 | S | F | OCOC₆H₅ | CH₃ |
| 132 | S | Cl | OCOC₆H₅ | CH₃ |
| 133 | S | Br | OCOC₆H₅ | CH₃ |
| 135 | S | SCH₃ | OCOC₆H₅ | CH₃ |
| 136 | S | SO₂CH₃ | OCOC₆H₅ | CH₃ |
| 137 | S | SO₂CH₂CH₃ | OCOC₆H₅ | CH₃ |
| 151 | Bindung | F | OCOC₆H₅ | CH₂CH₃ |
| 152 | Bindung | Cl | OCOC₆H₅ | CH₂CH₃ |
| 153 | Bindung | Br | OCOC₆H₅ | CH₂CH₃ |
| 155 | Bindung | SCH₃ | OCOC₆H₅ | CH₂CH₃ |
| 156 | Bindung | SO₂CH₃ | OCOC₆H₅ | CH₂CH₃ |
| 157 | Bindung | SO₂CH₂CH₃ | OCOC₆H₅ | CH₂CH₃ |
| 171 | O | F | OCOC₆H₅ | CH₂CH₃ |
| 172 | O | Cl | OCOC₆H₅ | CH₂CH₃ |
| 173 | O | Br | OCOC₆H₅ | CH₂CH₃ |
| 175 | O | SCH₃ | OCOC₆H₅ | CH₂CH₃ |
| 176 | O | SO₂CH₃ | OCOC₆H₅ | CH₂CH₃ |
| 177 | O | SO₂CH₂CH₃ | OCOC₆H₅ | CH₂CH₃ |
| 181 | S | F | OCOC₆H₅ | CH₂CH₃ |
| 182 | S | Cl | OCOC₆H₅ | CH₂CH₃ |
| 183 | S | Br | OCOC₆H₅ | CH₂CH₃ |
| 185 | S | SCH₃ | OCOC₆H₅ | CH₂CH₃ |
| 186 | S | SO₂CH₃ | OCOC₆H₅ | CH₂CH₃ |
| 187 | S | SO₂CH₂CH₃ | OCOC₆H₅ | CH₂CH₃ |
| 1001 | Bindung | F | OH | CH(CH₃)₂ |
| 1002 | Bindung | Cl | OH | CH(CH₃)₂ |
| 1003 | Bindung | Br | OH | CH(CH₃)₂ |
| 1005 | Bindung | SCH₃ | OH | CH(CH₃)₂ |
| 1006 | Bindung | SO₂CH₃ | OH | CH(CH₃)₂ |
| 1007 | Bindung | SO₂CH₂CH₃ | OH | CH(CH₃)₂ |
| 1021 | O | F | OH | CH(CH₃)₂ |
| 1022 | O | Cl | OH | CH(CH₃)₂ |
| 1023 | O | Br | OH | CH(CH₃)₂ |
| 1025 | O | SCH₃ | OH | CH(CH₃)₂ |
| 1026 | O | SO₂CH₃ | OH | CH(CH₃)₂ |
| 1027 | O | SO₂CH₂CH₃ | OH | CH(CH₃)₂ |
| 1031 | S | F | OH | CH(CH₃)₂ |
| 1032 | S | Cl | OH | CH(CH₃)₂ |
| 1033 | S | Br | OH | CH(CH₃)₂ |
| 1035 | S | SCH₃ | OH | CH(CH₃)₂ |
| 1036 | S | SO₂CH₃ | OH | CH(CH₃)₂ |
| 1037 | S | SO₂CH₂CH₃ | OH | CH(CH₃)₂ |
| 1051 | Bindung | F | OH | C(CH₃)₃ |
| 1052 | Bindung | Cl | OH | C(CH₃)₃ |
| 1053 | Bindung | Br | OH | C(CH₃)₃ |
| 1055 | Bindung | SCH₃ | OH | C(CH₃)₃ |
| 1056 | Bindung | SO₂CH₃ | OH | C(CH₃)₃ |
| 1057 | Bindung | SO₂CH₂CH₃ | OH | C(CH₃)₃ |
| 1071 | O | F | OH | C(CH₃)₃ |
| 1072 | O | Cl | OH | C(CH₃)₃ |
| 1073 | O | Br | OH | C(CH₃)₃ |
| 1075 | O | SCH₃ | OH | C(CH₃)₃ |
| 1076 | O | SO₂CH₃ | OH | C(CH₃)₃ |
| 1077 | O | SO₂CH₂CH₃ | OH | C(CH₃)₃ |
| 1081 | S | F | OH | C(CH₃)₃ |
| 1082 | S | Cl | OH | C(CH₃)₃ |
| 1083 | S | Br | OH | C(CH₃)₃ |
| 1085 | S | SCH₃ | OH | C(CH₃)₃ |
| 1086 | S | SO₂CH₃ | OH | C(CH₃)₃ |
| 1087 | S | SO₂CH₂CH₃ | OH | C(CH₃)₃ |
| 1101 | Bindung | F | OCOC₆H₅ | CH(CH₃)₂ |
| 1102 | Bindung | Cl | OCOC₆H₅ | CH(CH₃)₂ |
| 1103 | Bindung | Br | OCOC₆H₅ | CH(CH₃)₂ |
| 1105 | Bindung | SCH₃ | OCOC₆H₅ | CH(CH₃)₂ |
| 1106 | Bindung | SO₂CH₃ | OCOC₆H₅ | CH(CH₃)₂ |
| 1107 | Bindung | SO₂CH₂CH₃ | OCOC₆H₅ | CH(CH₃)₂ |
| 1121 | O | F | OCOC₆H₅ | CH(CH₃)₂ |
| 1122 | O | Cl | OCOC₆H₅ | CH(CH₃)₂ |
| 1123 | O | Br | OCOC₆H₅ | CH(CH₃)₂ |
| 1125 | O | SCH₃ | OCOC₆H₅ | CH(CH₃)₂ |
| 1126 | O | SO₂CH₃ | OCOC₆H₅ | CH(CH₃)₂ |
| 1127 | O | SO₂CH₂CH₃ | OCOC₆H₅ | CH(CH₃)₂ |
| 1131 | S | F | OCOC₆H₅ | CH(CH₃)₂ |
| 1132 | S | Cl | OCOC₆H₅ | CH(CH₃)₂ |
| 1133 | S | Br | OCOC₆H₅ | CH(CH₃)₂ |
| 1135 | S | SCH₃ | OCOC₆H₅ | CH(CH₃)₂ |
| 1136 | S | SO₂CH₃ | OCOC₆H₅ | CH(CH₃)₂ |
| 1137 | S | SO₂CH₂CH₃ | OCOC₆H₅ | CH(CH₃)₂ |
| 1151 | Bindung | F | OCOC₆H₅ | C(CH₃)₃ |
| 1152 | Bindung | Cl | OCOC₆H₅ | C(CH₃)₃ |
| 1153 | Bindung | Br | OCOC₆H₅ | C(CH₃)₃ |
| 1155 | Bindung | SCH₃ | OCOC₆H₅ | C(CH₃)₃ |
| 1156 | Bindung | SO₂CH₃ | OCOC₆H₅ | C(CH₃)₃ |
| 1157 | Bindung | SO₂CH₂CH₃ | OCOC₆H₅ | C(CH₃)₃ |
| 1171 | O | F | OCOC₆H₅ | C(CH₃)₃ |
| 1172 | O | Cl | OCOC₆H₅ | C(CH₃)₃ |
| 1173 | O | Br | OCOC₆H₅ | C(CH₃)₃ |
| 1175 | O | SCH₃ | OCOC₆H₅ | C(CH₃)₃ |
| 1176 | O | SO₂CH₃ | OCOC₆H₅ | C(CH₃)₃ |
| 1177 | O | SO₂CH₂CH₃ | OCOC₆H₅ | C(CH₃)₃ |
| 1181 | S | F | OCOC₆H₅ | C(CH₃)₃ |
| 1182 | S | Cl | OCOC₆H₅ | C(CH₃)₃ |
| 1183 | S | Br | OCOC₆H₅ | C(CH₃)₃ |
| 1185 | S | SCH₃ | OCOC₆H₅ | C(CH₃)₃ |
| 1186 | S | SO₂CH₃ | OCOC₆H₅ | C(CH₃)₃ |
| 1187 | S | SO₂CH₂CH₃ | OCOC₆H₅ | C(CH₃)₃ |

Ebenso insbesondere bevorzugt sind die Verbindungen der Formel Ia3 (≡ Ia mit R¹² = H, R³ = CH₃, l = 1), insbesondere die Verbindungen Ia3.n, wobei die Variablen X, R⁴, R¹⁰ und R¹¹ die in Tabelle 1 genannte Bedeutung haben.

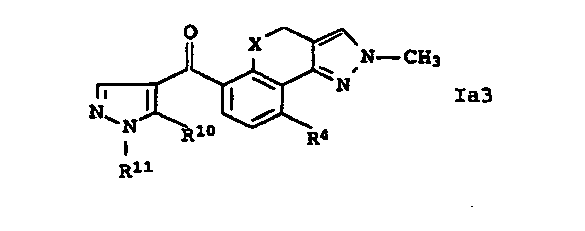

Ebenso insbesondere bevorzugt sind die Verbindungen der Formel Ia5 (≡ Ia mit R¹² = H, R³ = CH₃, l = 1), insbesondere die Verbindungen Ia5.n, wobei die Variablen X, R⁴, R¹⁰ und R¹¹ die in Tabelle 1 genannte Bedeutung haben.

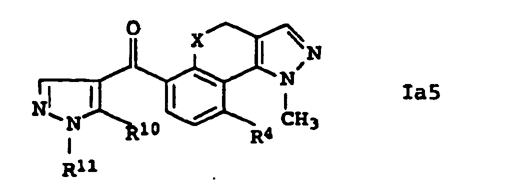

Ebenso insbesondere bevorzugt sind die Verbindungen der Formel Ia9 (≡ Ia mit R¹² = H, 1 = 0), insbesondere die Verbindungen Ia9.n, wobei die variablen X, R⁴, R¹⁰ und R¹¹ die in Tabelle 1 genannte Bedeutung haben.

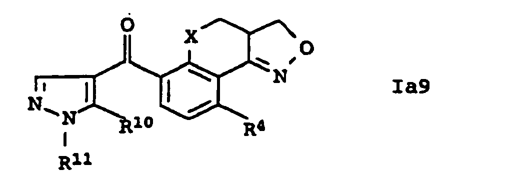

Ebenso insbesondere bevorzugt sind die Verbindungen der Formel Ia18 (≡ Ia mit R¹² = H, R¹³ = CH₃, l= 1), insbesondere die Verbindungen Ia18.n, wobei die Variablen X, R⁴, R¹⁰ und R¹¹ die in Tabelle 1 genannte Bedeutung haben.

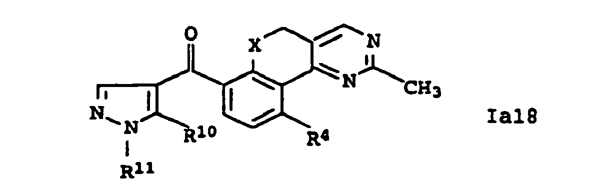

Die tricyclischen Benzoylpyrazol-Derivate der Formel I sind auf verschiedene Art und Weise erhältlich, beispielsweise nach folgenden Verfahren:

A. Darstellung von Verbindungen der Formel I mit R¹⁰ = Halogen durch Umsetzung eines tricyclischen Benzoylpyrazol-Derivats der Formel Iα (≡ I mit R¹⁰ = Hydroxy) mit einem Halogenierungsmittel: 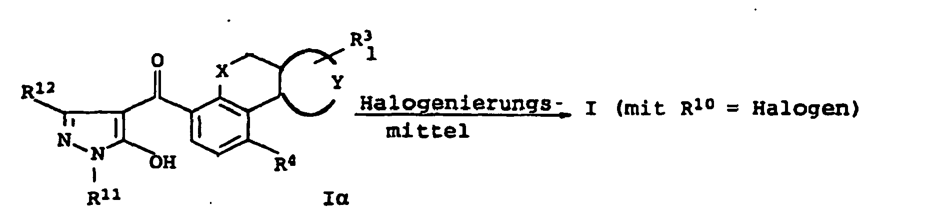 Als Halogenierungsmittel eignen sich beispielsweise Phosgen, Diphosgen, Triphosgen, Thionylchlorid, Oxalylchlorid, Phosphoroxychlorid, Phosphorpentachlorid, Mesylchlorid, Chlormethylen-N,N-dimethylammoniumchlorid, Oxylylbromid, Phosphoroxybromid etc.

Die Ausgangsverbindungen werden in der Regel äquimolaren Verhältnis eingesetzt. Es kann aber auch von Vorteil sein, die eine oder andere Komponente im Überschuß einzusetzen.

Als Lösungsmittel kommen z.B. chlorierte Kohlenwasserstoffe, wie Methylenchlorid oder 1,2-Dichlorethan, aromatische Kohlenwasserstoffe, z.B. Toluol, Xylol oder Chlorbenzol, polare aprotische Lösungsmittel wie Acetonitril, Dimethylformamid oder Dimethylsulfoxid oder Gemische hiervon in Betracht. Es ist aber auch möglich, die Reaktion in Substanz durchzuführen.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

B. Darstellung von Verbindungen der Formel I mit R¹⁰ = OR¹³, durch Umsetzung eines tricyclischen Benzoylpyrazol-Derivats der Formel Iα (≡ I mit R¹⁰ = Hydroxy) mit einem Alkylierungsmittel III.

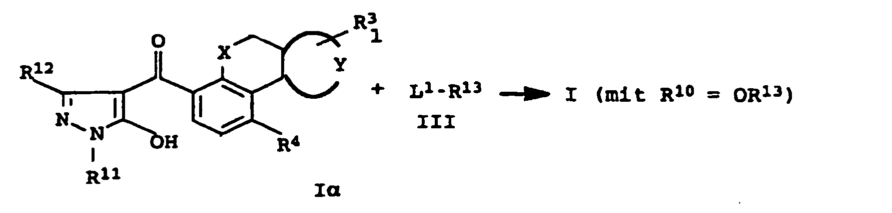

L¹ steht für eine nucleophil verdrängbare Abgangsgruppe, wie Halogen, z. B. Chlor oder Brom, Hetaryl, z. B. Imidazolyl, Carboxylat, z. B. Acetat, oder Sulfonat, z. B. Mesylat oder Triflat etc.

Die Verbindungen der Formel III können direkt eingesetzt werden wie z. B. im Fall der Carbonsäurehalogenide oder in situ erzeugt werden, z. B. aktivierte Carbonsäuren (mit Carbonsäure und Dicyclohexylcarbodiimid etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es auch von Vorteil sein, die Umsetzung in Gegenwart einer Base durchzuführen. Die Reaktanden und die Base werden dabei zweckmäßigerweise in äquimolaren Mengen eingesetzt. Ein Überschuß der Base kann z.B. 1,5 bis 3 Moläquivalente kann unter Umständen vorteilhaft sein.

Als Basen eignen sich tertiäre Alkylamine, wie Triethylamin, aromatische Amine, wie Pyridin, Alkalimetallcarbonate, z.B. Natriumcarbonat oder Kaliumcarbonat, Alkalimethallhydrogencarbonate, wie Natriumhydrogencarbonat und Kaliumhydrogencarbonat, Alkalimetallalkoholate wie Natriummethanolat, Natriumethanolat, Kalium-tert.-butanolat oder Alkalimetallhydride, z.B. Natriumhydrid. Bevorzugt verwendet werden Triethylamin oder Pyridin.

Als Lösungsmittel kommen z.B. chlorierte Kohlenwasserstoffe, wie Methylenchlorid oder 1,2-Dichlorethan, aromatische Kohlenwasserstoffe, z.B. Toluol, Xylol oder Chlorbenzol,

Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

Darstellung von Verbindungen der Formel I mit R¹⁰ = OR¹³, SR¹³, NR¹⁵R¹⁶ oder N-gebundenes Heterocyclyl durch Umsetzung von Verbindungen der Formel Iβ (≡ I mit R¹⁰ = Halogen) mit einer Verbindung der Formel IVα, IVβ, IVγ oder IVδ, gegebenenfalls in Gegenwart einer Base oder unter vorangehender Salzbildung.

| | | | | |
|---|---|---|---|---|
| | | HOR¹³ oder | IVα | |
| Iβ | + | HSR¹³ oder | IVβ → | I (mit R¹⁰ = OR¹³, SR¹³, NR¹⁵R¹⁶ oder N-gebundenes Heterocyclyl) |
| | | HNR¹⁵R¹⁶ oder | IVγ | |
| | | H(N-gebundenes Heterocyclyl) | IVδ | |

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es auch von Vorteil sein, die Umsetzung in Gegenwart einer Base durchzuführen. Die Reaktanden und die Base werden dabei zweckmäßigerweise in äquimolaren Mengen eingesetzt. Ein Überschuß der Base kann z.B. 1,5 bis 3 Moläquivalente, bezogen auf Iβ (mit R¹⁰ = Halogen), kann unter Umständen vorteilhaft sein. Als Basen eignen sich tertiäre Alkylamine, wie Triethylamin, aromatische Amine, wie Pyridin, Alkalimetallcarbonate, z.B. Natriumcarbonat oder Kaliumcarbonat, Alkalimethallhydrogencarbonate, wie Natriumhydrogencarbonat und Kaliumhydrogencarbonat, Alkalimetallalkoholate wie Natriummethanolat, Natriumethanolat, Kalium-tert.-butanolat oder Alkalimetallhydride, z.B. Natriumhydrid. Bevorzugt verwendet werden Natriumhydrid oder Kalium-tert.-butanolat.

Als Lösungsmittel kommen z.B. chlorierte Kohlenwasserstoffe, wie Methylenchlorid oder 1,2-Dichlorethan, aromatische Kohlenwasserstoffe, z.B. Toluol, Xylol oder Chlorbenzol, Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

D. Darstellung von Verbindungen der Formel I mit R¹⁰ = SO₂R¹⁴ durch Umsetzung von Verbindungen der Formel I mit R¹⁰ = SR¹⁰ (Iγ) mit einem oxidationsmittel.

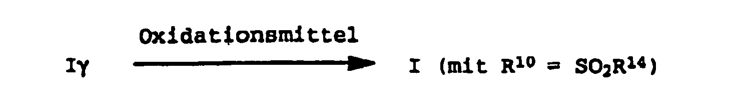

Als Oxidationsmittel kommen beispielsweise m-Chlorperbenzoesaure, Peroxyessigsäure, Trifluorperoxyessigsäure, Wasserstoffperoxid, ggf. in Gegenwart eines Katalysators wie Wolframat, in Betracht.

Die Ausgangsverbindungen werden in der Regel im äquimolaren Verhältnis eingesetzt. Es kann aber auch von Vorteil sein, die eine oder andere Komponente im Überschuß einzusetzen.

Als Lösungsmittel kommen z.B. chlorierte Kohlenwasserstoffe, wie Methylenchlorid oder 1,2-Dichlorethan, aromatische Kohlenwasserstoffe, z.B. Toluol, Xylol oder Chlorbenzol, Ether, wie Diethylether, Methyl-tert.-butylether, Tetrahydrofuran oder Dioxan, polare aprotische Lösungsmittel, wie Acetonitril oder Dimethylformamid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

E. Darstellung von Verbindungen der Formel I mit R⁹ = IIa (wobei R¹⁰ + Hydroxy oder Mercapto ist) durch Umsetzung eines metallierten Pyrazol-Derivats der Formel V mit einem tricyclischen Benzolsäure-Derivat der Formel VIα:

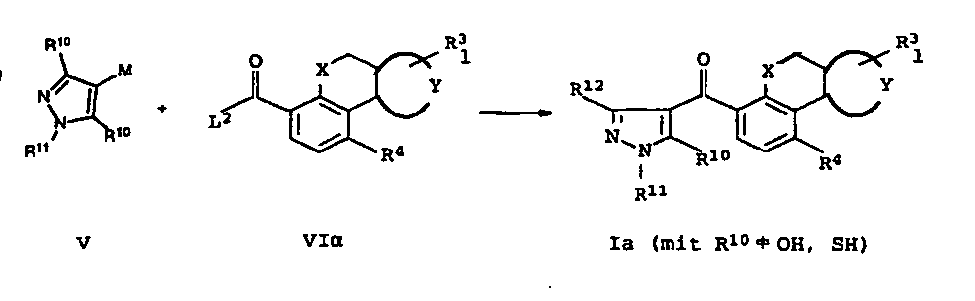

M steht hierbei für ein Metall, insbesondere für ein Alkalimetall wie Lithium oder Natrium, ein Erdalkalimetall wie z.B. Magnesium oder ein Übergangsmetall wie Palladium, Nickel etc. und L² für eine nucleophil verdrängbare Abgangsgruppe wie Halogen, z.B. Chlor oder Brom, Alkylsulfonat wie Mesylat, Halogenalkylsulfonat wie Triflat oder Cyanid.

Die Umsetzung wird in der Regel bei Temperaturen von -100°C bis Rückflußtemperatur des Reaktionsgemisches durchgeführt. Als Lösungsmittel eignen sich inerte aprotische Lösungsmittel, wie Ether, z.B. Diethylether, Tetrahydrofuran. Die Verbindungen der Formel VIα werden in der Regel im Überschuß eingesetzt, es kann aber auch von Vorteil sein, diese in äquimolaren Mengen oder im Unterschuß einzusetzen. Die Aufarbeitung erfolgt zum Produkt hin.

Die metallierten Pyrazol-Derivate der Formel V können auf an sich bekannte Art und weise durch Umsetzung von in 4-Position halogenierten Pyrazolen mit Metallen wie Lithium, Natrium, Magnesium etc. oder mit metallorganischen Verbindungen wie z.B. Butyllithium gebildet werden. Es ist aber auch möglich Pyrazole, die in 4-Position mit Wasserstoff verknüpft sind, direkt zu metallieren, z.B. mit den voranstehend genannten Metallen bzw. metallorganischen Verbindungen. Die Umsetzungen werden in der Regel in einem inerten aprotischen Lösungsmittel durchgeführt, bevorzugt in Ether wie Diethylether, Tetrahydrofuran etc.. Die Reaktionstemperatur liegt im Bereich von -100°C bis zur Höhe des Siedepunktes des Reaktionsgemisches. Die Verbindungen der Formel V werden in der Regel direkt weiter umgesetzt oder in situ erzeugt.

Darstellung von Verbindungen der Formel Iα (≡ I mit R¹⁰ = Hydroxy) durch Umsetzung einer aktivierten tricyclischen Benzoesäure der Formel VIβ oder einer tricyclischen Benzoesäure VIγ, die vorzugsweise in sich aktiviert wird, mit einem Pyrazol der Formel VII zu den Acylierungsprodukt und anschließende Umlagerung.

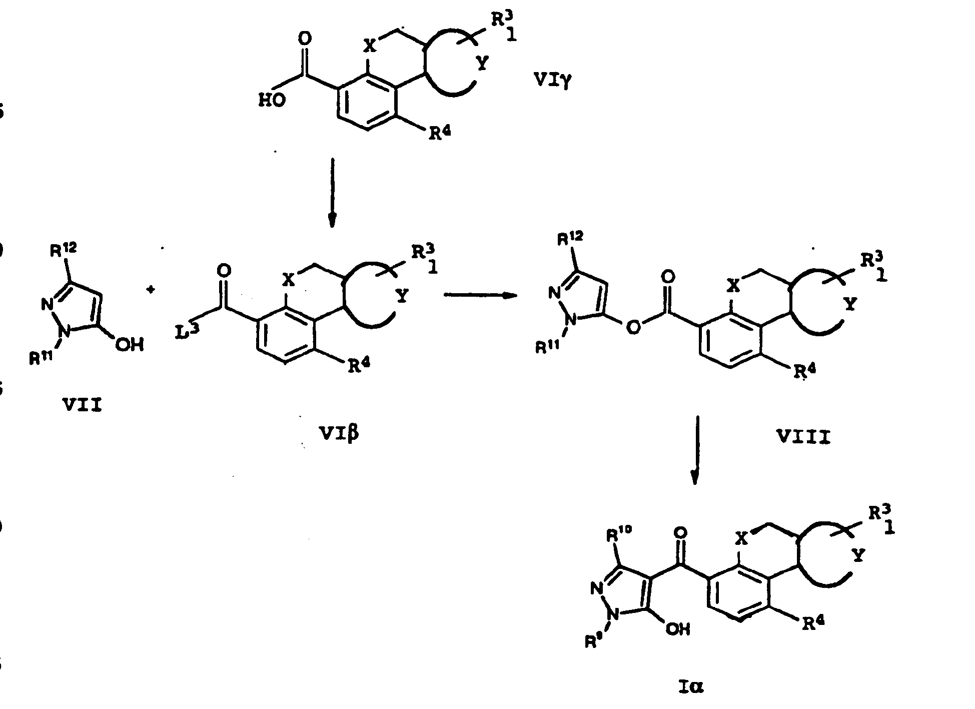

L³ steht für eine nucleophil verdrängbare Abgangsgruppe, wie Halogen z.B. Brom oder Chlor, Hetaryl, z.B. Imidazolyl oder Pyridyl, Carboxylat, z.B. Acetat oder Trifluoracetat etc.

Die aktivierte tricyclische Benzoesäure VIβ kann direkt eingesetzt werden, wie im Fall der tricyclischen Benzoylhalogenide oder in situ erzeugt werden, z.B. mit Dicyclohexylcarbodiimid, Triphenylphosphin/Azodicarbonsäureester, 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf VII, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid oder 1,2-Dichlorethan, aromatische Kohlenwasserstoffe, wie Toluol, Xylol oder Chlorbenzol, Ether, wie Diethylether, Methyl-tert.-butylether, Tetrahydrofuran oder Dioxan, polare aprotische Lösungsmittel, wie

Acetonitril, Dimethylformamid oder Dimethylsulfoxid oder Ester wie Essigsäureethylester oder Gemische hiervon verwendet werden.

Werden tricyclische Benzoylhalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das wertprodukt extrahiert. Als Lösungsmittel eignen sich hierfür besonders Methylenchlorid, Diethylether und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester VIII zu den Verbindungen der Formel Iα erfolgt zweckmäßigerweise bei Temperaturen von 20 bis 100°C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, aromatische Amine wie Pyridin oder Alkalicarbonate, wie Natriumcarbonat oder Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonat verwendet, vorzugsweise in doppelt äquimolarem Verhältnis in Bezug auf den Ester.

Als Cyanoverbindungen kommen anorganische Cyanide, wie Natriumcyanid oder Kaliumcyanid und organische Cyanoverbindungen, wie Acetoncyanhydrin oder Trimethylsilylcyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid oder Essigsäureethylester extrahiert. Der organische Extrakt kann mit 5-10%iger Alkalicarbonatlösung, z.B. Natriumcarbonat- oder Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt.

Es ist aber auch möglich, den Ester VIII in situ zu erzeugen, indem man ein Pyrazol der Formel VII, oder ein Alkalisalz hiervon, mit einem tricyclischen Benzolderivat der Formel IX in Gegenwart von Kohlenmonoxid, eines Katalysators sowie einer Base, umsetzt. 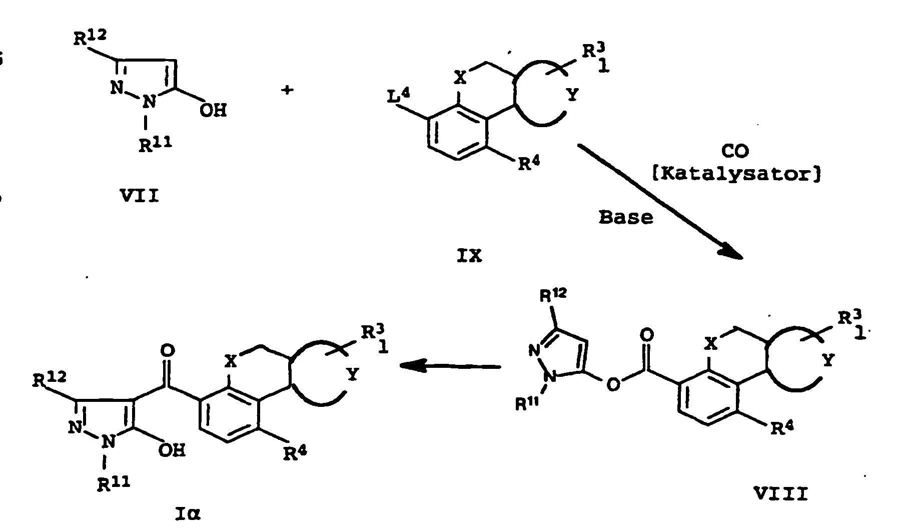 L⁴ steht für eine Abgangsgruppe wie Halogen, z.B. Chlor, Brom oder

Iod, oder Sulfonat wie Mesylat oder Triflat; bevorzugt sind Brom oder Triflat.

Der Ester VIII reagiert ggf. direkt zu dem tricyclischen Benzoylpyrazol-Derivat der Formel Iα ab.

Als Katalysatoren eignen sich Palladiumligandkomplexe, in denen das Palladium in der Oxidationsstufe 0 vorliegt, metallisches Palladium, das gegebenenfalls auf einen Träger aufgezogen wurde, und vorzugsweise Palladium(II)salze. Die Umsetzung mit Palladium(II)salzen und metallischem Palladium wird vorzugsweise in Gegenwart von Komplexliganden durchgeführt.

Als Palladium(0)ligandkomplex kommt beispielsweise Tetrakis(triphenylphosphan)palladium in Frage.

Metallisches Palladium ist vorzugsweise auf einen inerten Träger wie beispielsweise Aktivkohle, Siliciumdioxid, Aluminiumoxid, Bariumsulfat oder Calciumcarbonat aufgezogen. Die Reaktion wird vorzugsweise in Gegenwart von Komplexliganden wie beispielsweise Triphenylphosphan durchgeführt.

Als Palladium(II)salze eigenen sich beispielsweise Palladiumacetat und Palladiumchlorid. Bevorzugt wird in Gegenwart von Komplexliganden wie beispielsweise Triphenylphosphan gearbeitet.

Geeignete Komplexliganden für die Palladiumligandkomplexe, bzw. in deren Gegenwart die Umsetzung mit metallischem Palladium oder Palladium(II)salzen vorzugsweise ausgeführt wird, sind tertiäre Phosphane, deren Struktur durch folgende Formeln wiedergegeben wird: 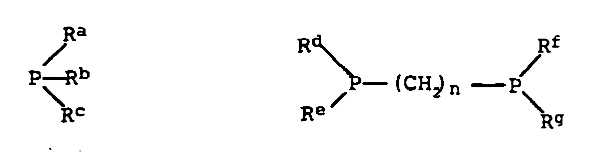 wobei n die Zahlen 1 bis 4 bedeutet und die Reste R^{a} bis R^{g} für C₁-C₆-Alkyl, Aryl-C₁-C₂-alkyl oder vorzugsweise Aryl stehen. Aryl steht beispielsweise für Naphthyl und gegebenenfalls substituiertes Phenyl wie beispielsweise 2-Tolyl und insbesondere für unsubstituiertes Phenyl.

Die Herstellung der komplexen Palladiumsalze kann in an sich bekannter Weise ausgehend von kommerziell erhältlichen Palladiumsalzen wie Palladiumchlorid oder Palladiumacetat und den entsprechenden Phosphanen wie z.B. Triphenylphosphan oder 1,2-Bis(diphenylphosphano)ethan erfolgen. Ein Großteil der komplexierten Palladiumsalze ist auch kommerziell erhältlich. Bevorzugte Palladiumsalze sind [(R)(+)2,2'-Bis(diphenylphosphano)-1,1'-binaphthyl]palladium(II)chlorid, Bis(triphenylphosphan)palladium(II)acetat und insbesondere Bis(triphenylphosphan)palladium(II)chlorid.

Der Palladiumkatalysator wird in der Regel in einer Konzentration von 0,05 bis 5 Mol%, und bevorzugt 1-3 Mol% eingesetzt.

Als Basen kommen tertiäre Amine wie beispielsweise N-Methylpiperidin, Ethyldiisopropylamin, 1,8-Bisdimethylaminonaphthalin oder insbesondere Triethylamin in Betracht. Ebenso eigenen sich

Alkalicarbonat, wie Natriumcarbonat oder Kaliumcarbonat. Aber auch Gemische von Kaliumcarbonat und Triethylamin sind geeignet.

In der Regel werden 2 bis 4 Moläquivalente, insbesondere 2 Moläquivalente, des Alkalicarbonats, sowie 1 bis 4 Moläquivalente, insbesondere 2 Moläquivalente des tertiären Amins bezogen auf das tricyclische Benzolderivat der Formel IX eingesetzt.

Als Lösungsmittel können Nitrile wie Benzonitril und Acetonitril, Amide wie Dimethylformamid, Dimethylacetamid, Tetra-C₁-C₄-alkyl-harnstoffe oder N-Methylpyrrolidon und vorzugsweise Ether wie Tetrahydrofuran, Methyl-tert.-butylether dienen. Insbesondere werden Ether wie 1,4-Dioxan und Dimethoxyethan als Lösungsmittel bevorzugt.

Die tricyclischen Benzoylhalogenide der Formel VIβ mit L³ = Cl, Br können auf an sich bekannte Art und Weise durch Umsetzung der tricyclischen Benzoesäuren der Formel VIγ (≡ VIb) mit Halogenierungsreagentien wie Thionylchlorid, Thionylbromid, Phosgen, Diphosgen, Triphosgen, Oxalylchlorid, Oxalylbromid hergestellt werden.

Die tricyclischen Benzoesäuren der Formel VIγ (≡VIb) können in bekannter Weise durch saure oder basische Hydrolyse aus den entsprechenden Estern VIc hergestellt werden.

Tricyclische Benzoesäurederivate der Formel VI sind neu, 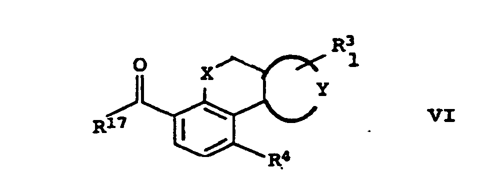 wobei die Variablen folgende Bedeutung haben:
- X: Sauerstoff, Schwefel oder eine Bindung;
- Y: bildet gemeinsam mit den beiden Kohlenstoffen, an die es gebunden ist, nachfogende Heterocyclen aus : 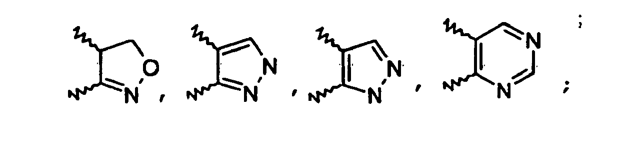
- R³: C₁-C₆-Alkyl;
- R⁴: Halogen, C₁-C₆-Alkoxy, C₁-C₆-Alkylthio, oder C₁-C₆-Alkylsulfonyl;
- l: 0 oder 1 ;
- R¹⁷: Hydroxy oder ein abhydrolysierbarer Rest;

Beispiele für abhydrolysierbare Reste sind Alkoxy-, Phenoxy-, Alkylthio-, Phenylthio-Reste, die ggf. substituiert sein können, Halogenide, Heteroaryl-Reste, die über Stickstoff gebunden sind, Amino-, Imino-Reste, die ggf. substituiert sein können etc.

Bevorzugt sind tricyclische Benzoesäurehalogenide VIa (VI mit R¹⁷ = Halogen) 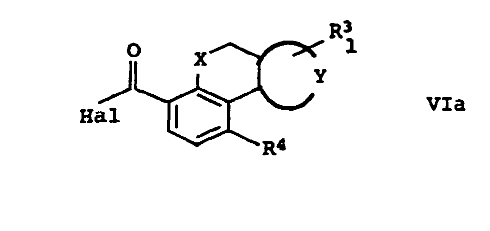 wobei die Variablen X, Y, R³ und R⁴ und l die unter Formel VI genannte Bedeutung haben und
- Hal: Halogen, insbesondere Chlorid oder Bromid, bedeutet.

Ebenso bevorzugt sind tricyclische Benzosäuren der Formel VIb (VI mit R¹⁷ = Hydroxy; ≡ VIγ), 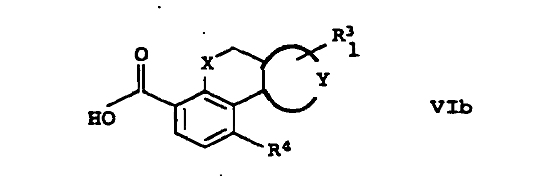 wobei die Variablen X, Y, R³ und R⁴ und l die unter Formel VI genannte Bedeutung haben.

Ebenso bevorzugt sind tricyclische Benzosäureester der Formel VIc (VI mit R¹⁷ = T = C₁-C₆-Alkoxy), 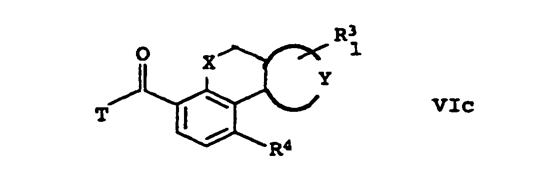 wobei die Variablen X, Y, R³ und R⁴ und 1 die unter Formel VI genannte Bedeutung haben und
- T: C₁-C₆-Alkoxy bedeutet.

Die besonders bevorzugten Ausführungsformen der tricyclischen Benzoesäure-Derivate der Formel VI, VIa, VIb und VIc in Bezug auf die Variablen X, Y, R³ und R⁴ und l entstprechen denen der tricyclischen Benzoylpyrazol-Derivate der Formel I.

Insbesondere bevorzugt sind die Verbindungen VI, VIa, VIb und VIc, wobei Y gemeinsam mit den beiden Kohlenstoffen, an die es gebunden ist, folgende Heterocyclen ausbildet:

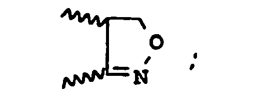

Hierbei sind Verbindungen VI, VIa, VIb und VIc außerordentlich bevorzugt, wobei
- R⁴: Halogen, C₁-C₆-Alkoxy, C₁-C₆-Alkythio oder C₁-C₆-Alkylsulfonyl; insbesondere C₁-C₆-Alkylksulfonyl;
bedeutet.

Die tricyclischen Benzoesäureester VIc sind auf verschiedene Art und Weise erhältlich.

Beispielsweise können Benzosäureester der Formel X, die auf an sich bekannte Art und Weise hergestellt werden (vgl. z.B. Chem. Pharm. Bull. 1985, 33 (8). 3336; Helv. Chim. Acta 1987, 70, 1326; J. Chem. Soc. Perkin Trans. 1972, 2019; J. Chem. Soc. Perkin Trans. 1991, 2763; Tetrahydron Asymmetry 1998, 9, 1137), zu cyclischen Ketonen der Formel XI cyclisiert werden (vgl. z.B. Chem. Ber. 1923, 56, 1819; J. Chem. Soc. Perkin I 1991, 2763; J. Med. Chem. 1988, 31, 230; Tetrahedron 1987, 43, 4549; Synlett 1991, 6, 443; Chem. Pharm. Bull. 1985, 33 (8), 3336). Diese lassen sich in Analogie zu bekannten Verfahren (vgl. z.B. J. Heterocyclic Chem. 1976, 13, 545; J. Heterocyclic Chem. 1972, 9. 1341; J. Org. Chem. 1978, 43, 3015; J. Chem. Soc. Perkin Trans. I 1978, 86; J. Org. Chem. 1986, 51, 2021) in die tricyclischen Benzoesäureester der Formel VIc überführen.

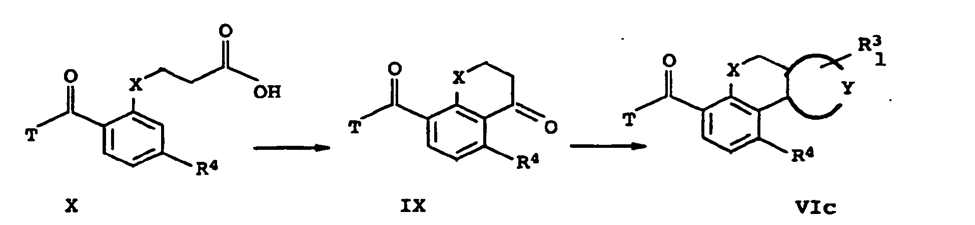

Weiterhin kann es in Betracht kommen das cyclische Keton der Formel XI auf an sich bekannte Weise zu cyclisieren (XII), z.B. mit einem Anhydrid oder Säureanhydrid ggf. in Gegenwart von katalytischen Mengen einer Lewissäure, wie Bortrifluorid (vgl. z.B. Can. J. Chem. 1979. 57, 3292; J. Am. Chem. Soc. 1953, 75, 626) und anschließend mit einem Hydrazin umzusetzen (vgl. A.R. Katritzky et al., Comprehensive Heterocyclic Chemistry, Vol. 5, p. 121, 277 - 280 (1984), Pergamon Press; J. Org. Chem. 1961, 26, 451; Org. Synth. 1949, 29, 54), wobei der resultierende Pyrazolrest nach üblichen Verfahren weiter modifiziert werden kann.

Weiterhin kann das Diketon XII mit Hydroxylamin oder Äquivalenten hiervon umgesetzt werden (vgl. A.R. Katritzky et al., Comprehensive Heterocyclic Chemistry, Vol. 6, p. 61 - 64, 118 (1984), Pergamon Press; Chem. Ber. 1967, 100, 3326). Man erhält entsprechende Isoxazolderivate, die nach üblichen Verfahren weiter modifiziert werden können.

Ebenso ist es möglich, das Diketon XII mit Amidinen umzusetzen (vgl. z.B. A.R. Katritzky et al., Comprehensive Heterocyclic Chemistry, Vol. 3, p. 112 - 114 (1924), Pergamon Press; J. Chem. Soc. C 1967, 1922; Org. Synth. 1963, IV, 182). Die so erhaltenen Pyrimidinderivate können bei Bedarf nach den üblichen Verfahren weiter modifiziert werden.

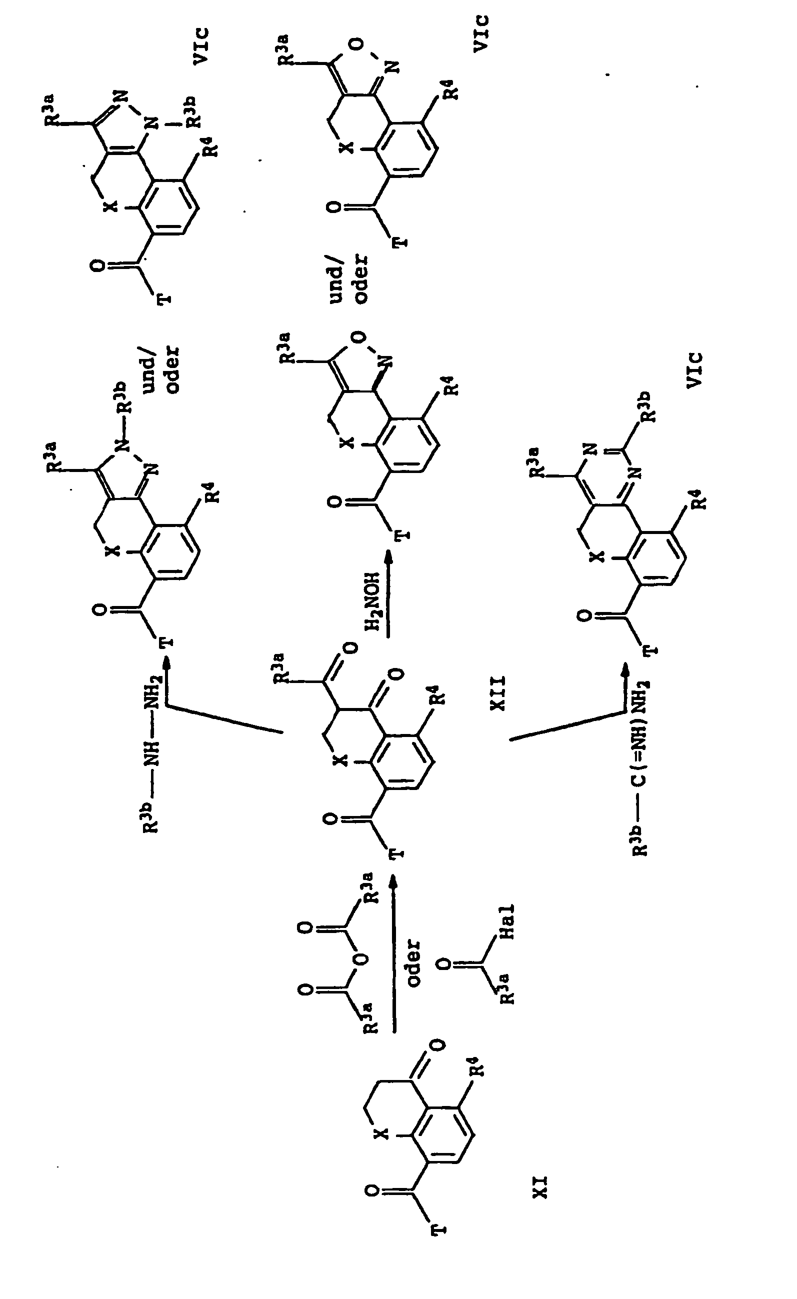

Es ist auch möglich bei den voranstehend genannten Reaktionen anstelle des Diketons XII Äquivalente hiervon, wie Enolether oder Enamine, die in Analogie zu bekannten Verfahren dargestellt werden können, einzusetzen.

Es kann auch in Betracht kommen das cyclische Keton der Formel XI in Analogie zu bekannten Verfahren mit einen Aldehyd oder Keton umzusetzen (XIII) (vgl. z.B. Tetrahedron Lett. 1978, 2111; Tetrahedron Lett. 1981, 5251; Chem. Ber. 1960, 2294; J. Chem. Soc. Perkin Trans. 1, 1991, 1467; Tetrahedron Lett. 1992, 8091). Das resultierende ungesättigte cyclische Keton der Formel XIII kann auf an sich bekannte Weise (vgl. z.B. A.R. Katritzky et al. Comprehensive Heterocyclic Chemistry, Vol. 2, 6 (1984), Pergamon Press; J. Heterocyclic Chem. 1969, 533; J. Heterocyclic Chem. 1968, 853) mit einem Hydrazin umgesetzt werden, wobei das resultierende Pyrazolin nach üblichen Verfahren weiter modifiziert werden kann.

Weiterhin kann das ungesättigte cyclische Keton der Formel XIII mit Hydroxylamin oder Äquivalenten hiervon umgesetzt werden (Z. Chem. 1980, 20, 19). Man erhält entsprechende Isoxazolinderivate, die nach üblichen Verfahren weiter modifiziert werden können.

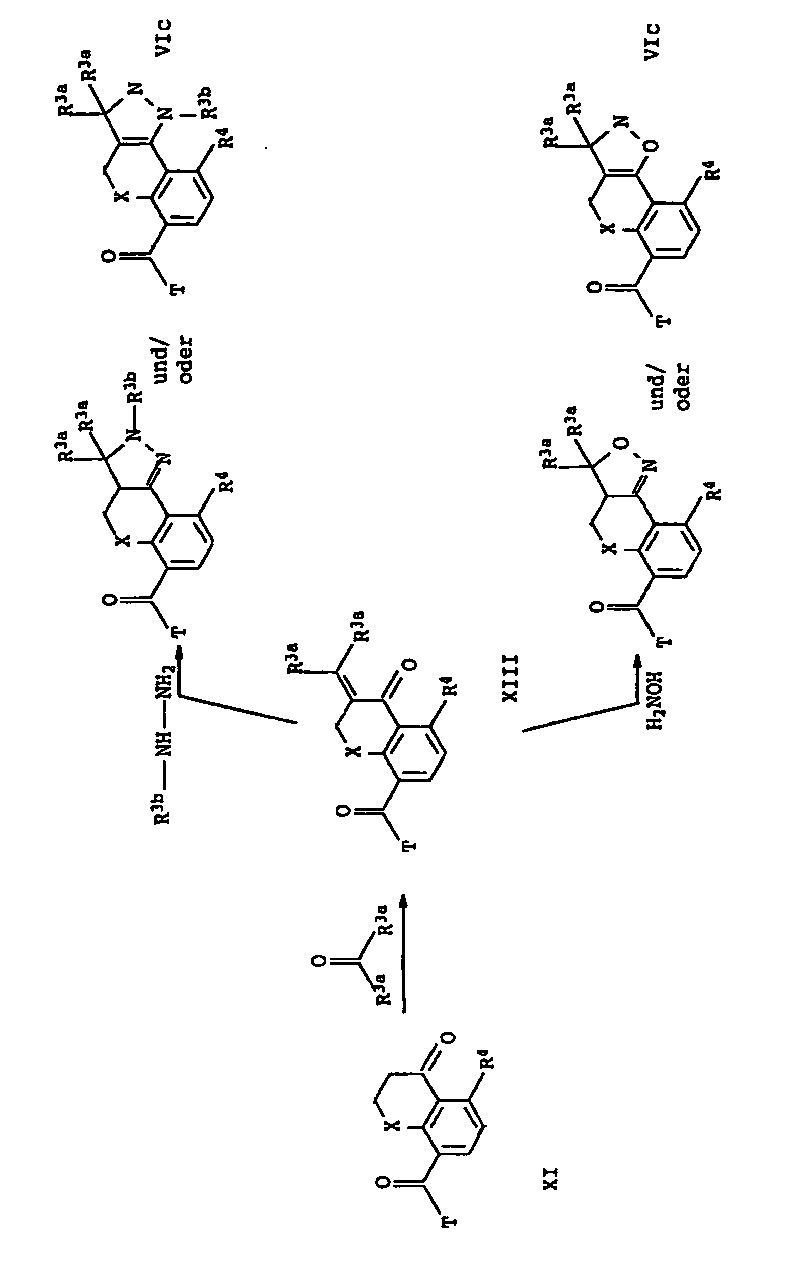

Weiterhin können Aldehyde der Formel XIV, die auf an sich bekannte Art und Weise hergestellt werden können, in Analogie zu literaturbekannten Verfahren durch Umsetzung mit einem Hydrazin oder Hydroxylamin (oder Äquivalenten hiervon) in entsprechende Hydrazone oder Oxime überführt werden (vgl. z.B. Synth. Commun. 1990, 20, 1373; J. Org. Chem. 1980, 45, 3756). Diese können wiederum auf an sich bekannte Art und Weise in die entsprechenden 1,3-Dipole überführt werden, die dann im Rahmen einer [3 + 2]-Cycloaddition zu den Verbindungen VIc abreagieren (vgl. z.B. Synth. Commun. 1990, 20, 1373; EP-A 386 892; J. Org. Chem. 1980, 45, 3756; Tetrahedron Lett. 1981, 22, 1333.)

Die so erhaltenen Pyrazole bzw. Pyrazoline sowie Isoxazole bzw. Isoxazoline können nach üblichen Verfahren weiter modifiziert werden.

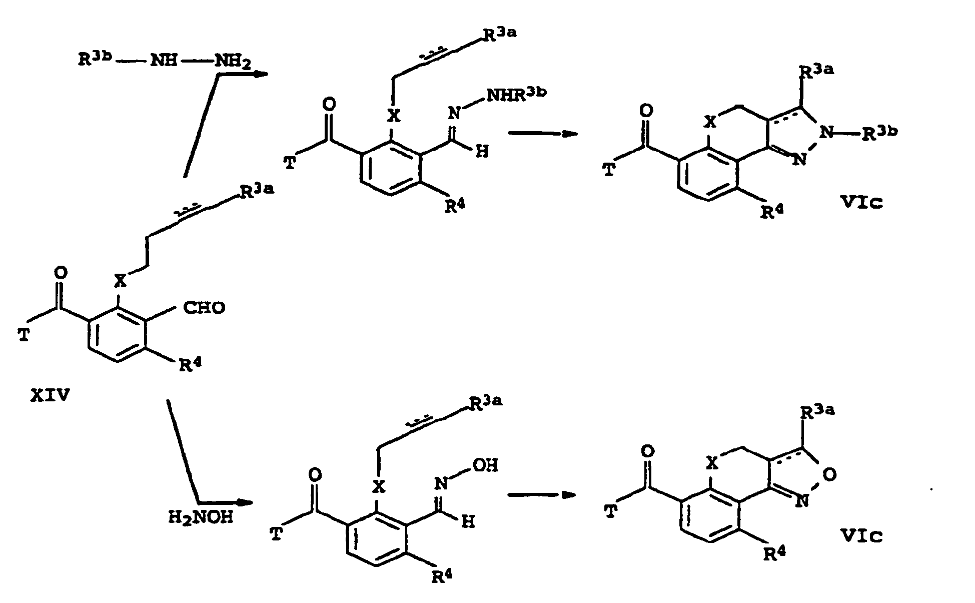

Ebenso ist es möglich das cyclische Keton der Formel XI mit einen Dithiol oder einem "gemischten Alkohol" in Analogie zu literaturbekannten Verfahren (vgl. z.B. T.W. Greene et al., Protective Groups in Organic Synthesis, John Wiley & Sons, 133-140) umzusetzen und anschließend einer Umlagerung in Gegenwart von Brom oder einer geeigneten Lewisssäure wie z.B. Tellurtetrachlorid zu unterwerfen (vgl. Tetrahedron 1991, 47, 4187; Synthesis 1991, 223; J. Chem. Soc. Chem. Commun. 1985, 1645).

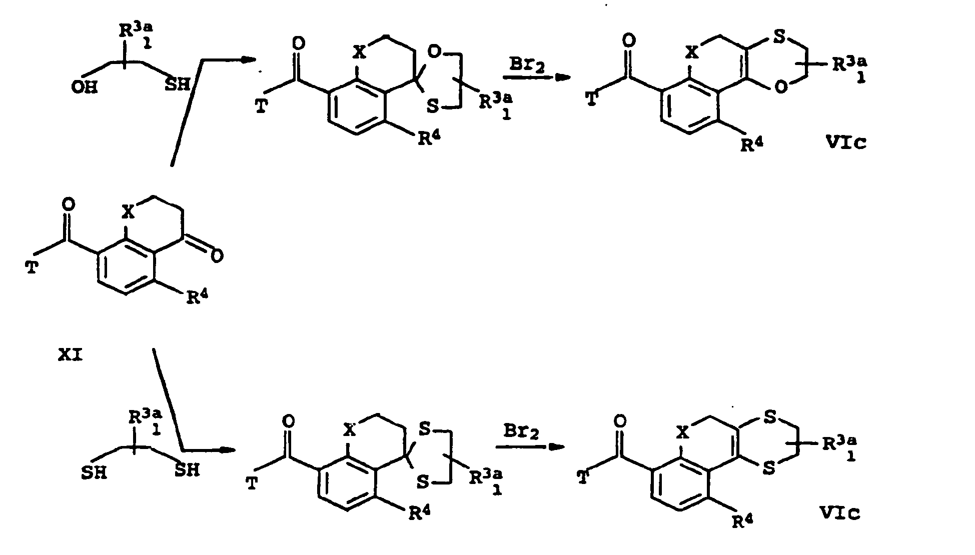

Die so erhaltenen Heterocyclen können nach an sich bekannten Verfahren ggf. weiter modifiziert werden.

Die vorstehend genannten Substituenten R^{3a} stehen für Wasserstoff, C₁-C₆-Alkyl, C₁-C₆-Haloqenalkyl, Hydroxy, C₁-C₆-Alkoxy oder C₁-C₆-Halogenalkoxy; weiterhin stehen die voranstehend genannten Reste R^{3b} für Wasserstoff, C₁-C₆-Alkyl oder C₁-C₆-Halogenalkyl.

Die tricyclischen Benzoesäureester der Formel VIc bzw. die tricyclischen Benzoesäuren der Formel VIb können durch Umsetzung eines tricyclischen Benzolderivats der Formel IX mit einem C₁-C₆-Alkohol bzw. Wasser in Gegenwart von Kohlenmonoxid, eines Katalysators sowie eine Base erhalten werden. Es gelten in der Regel die unter Verfahren F angegebenen Bedingungen.

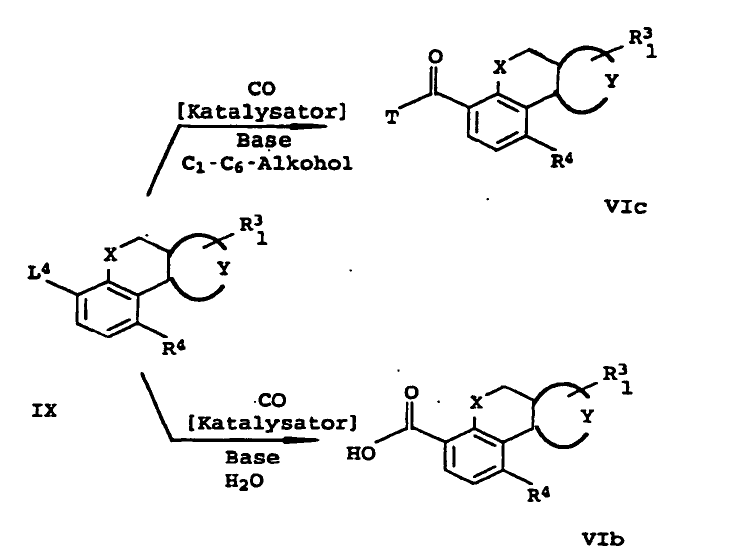

L⁴ steht hierbei für eine Abgangsgruppe wie Halogen, z.B. Chlor, Brom oder Iod, oder Sulfat wie Mesylat oder Triflat; bevorzugt sind Brom oder Triflat.

Weiterhin können die tricyclischen Benzoesäuren der Formel VIb erhalten werden, indem man ein tricyclisches Benzolderivat der Formel IX mit L⁴ Halogen wie Chlor oder Brom, insbesondere Brom, durch Umsetzung mit beispielsweise n-Butyllithium oder Magnesium in das metallierte Derivat überführt und anschließend mit Kohlendioxid quencht (vgl. z.B. J. Org. Chem. 1990, 55, 773; Angew. Chem. Int. Ed. 1969, 8, 68).

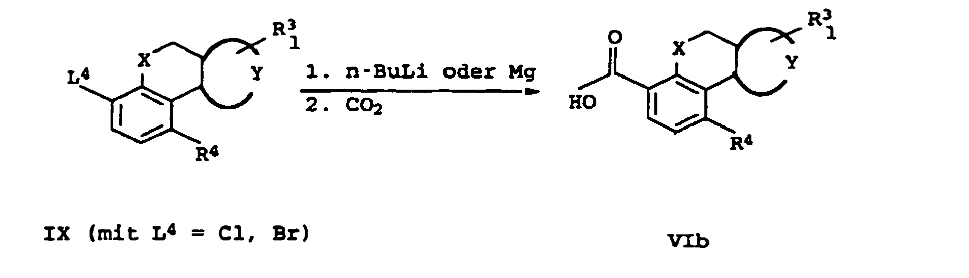

Ebenso können die tricyclischen Benzoesäuren VIb durch Verseifung der entsprechenden Nitrile in Analogie zu literaturbekannten Verfahren erhalten werden. Die Nitrile wiederum können durch.Halogen/Nitril-Austausch erhalten werden oder durch Sandmeyer-Reaktion aus den entsprechenden Anilinen XV.

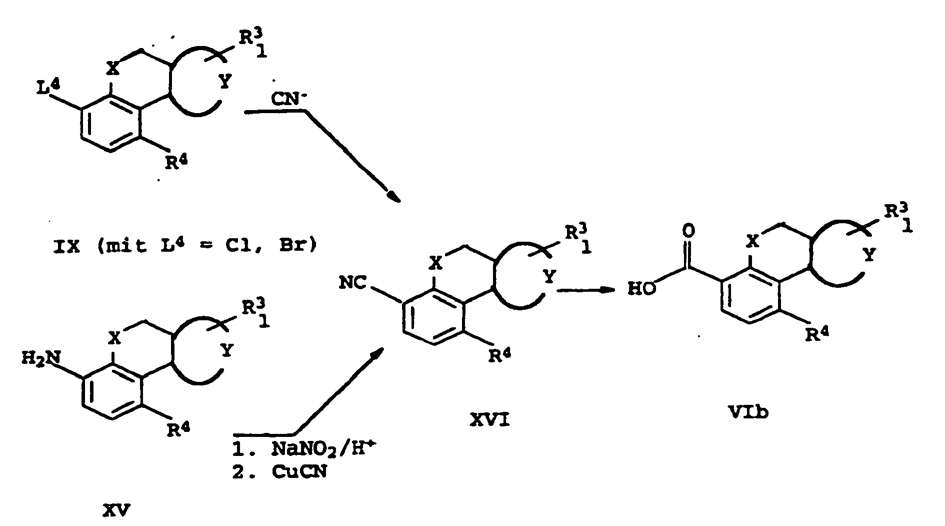

Die Verbindungen der Formel IX sind neu,
wobei die Variablen folgende Bedeutung haben:
- X: Sauerstoff, Schwefel oder eine Bindung;
- Y: bildet gemeinsam mit den beiden Kohlenstoffen, an die es gebunden ist, nachfogende Heterocyclen aus : 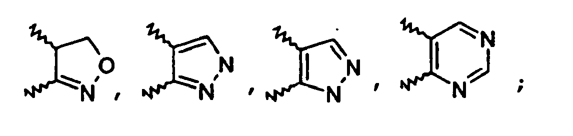
- R³: C₁-C₆-Alkyl;
- R⁴: Halogen, C₁-C₆-Alkoxy, C₁-C₆-Alkylthio oder C₁-C₆-Alkylsulfonyl;
- l: 0 oder 1 ;
- L⁴: Halogen, C₁-C₆-Alkylsulfonyloxy, C₁-C₆-Halogenalkylsulfonyoxy oder Phenylsulfonyloxy, wobei der Phenylring des letztgenannten Rests unsubstituiert sein kann oder partiell oder vollständig halogeniert und/oder einen bis drei der folgenden Reste tragen kann: Nitro, Cyano, C₁-C₆-Alkyl, C₁-C₆-Halogenalkyl, C₁-C₄-Alkoxy- oder C₁-C₄-Halogenalkoxy;

Bevorzugt sind Verbindungen der Formel IX, wobei L⁴ für Halogen, insbesondere Brom steht.

Die besonders bevorzugten Ausführungsformen der Verbindungen der Formel IX in Bezug auf die Variablen X, Y, R³ und R⁴ und l entsprechen denen der tricyclischen Benzoylpyrazol-Derivate der Formel I. Insbesondere bevorzugt sind die Verbindungen der Formel IX, wobei
- Y: gemeinsam mit den beiden Kohlenstoffen, an die es gebunden ist, folgende Heterocyclen ausbildet:

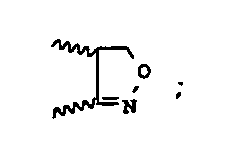

Hierbei sind die Verbindungen IX außerordentlich bevorzugt, wobei
- R⁴: Halogen, C₁-C₆-Alkoxy, C₁-C₆-Alkylthio oder C₁-C₆-Alkylsulfonyl: insbesondere C₁-C₆-Alkylsulfonyl;
bedeutet.

Die Verbindungen der Formel IX können auf verschiedene Art und Weise erhalten werden, beispielsweise kann das anellierte System analog zu den bei den Verbindungen der Formel VIc beschriebenen Verfahren aufgebaut werden.

Es ist aber auch möglich von einem geeigneten Grundkörper ausgehend das anellierte System aufzubauen (in Analogie zu den bei den Verbindungen der Formel VIc beschriebenen Verfahren) und anschließend L⁴ = Halogen durch übliche Halogenierungsreaktion einzuführen.

Ebenso sind die Aniline der Formel XV und die Nitrile der Formel XVI neu, 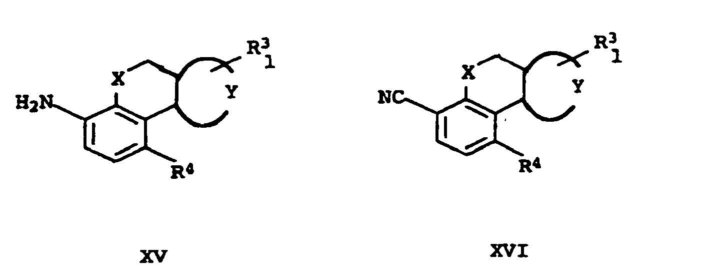 wobei die Variablen folgende Bedeutung haben:
- X: Sauerstoff, Schwefel oder eine Bindung;
- Y: bildet gemeinsam mit den beiden Kohlenstoffen, an die es gebunden ist,
nachfogende Heterocyclen aus : 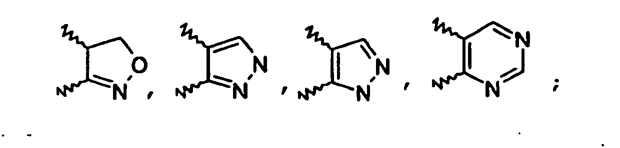
- R³: C₁-C₆-Alkyl,
- R⁴: Halogen, C₁-C₆-Alkoxy, C₁-C₆-Alkylthio, oder C₁-C₆-Alkylsulfonyl,
- l: 0 oder 1 .

Die besonders bevorzugten Ausführungsformen der Verbindungen der Formel XV und XVI in Bezug auf die Variablen X, Y, R³ und R⁴ und 1 entsprechen denen der tricyclischen Benzoylpyrazol-Derivate der Formel I.

Insbesondere bevorzugt sind die Verbindungen der Formel XV bzw. XVI, wobei
- Y: gemeinsam mit den beiden Kohlenstoffen, an die es gebunden ist, folgende Heterocyclen ausbildet:

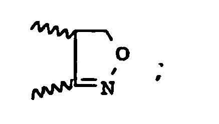

Hierbei sind die Verbindungen XV bzw. XVI außerordentlich bevorzugt, wobei
- R⁴: Halogen, C₁-C₆-Alkoxy, C₁-C₆-Alkylthio oder C₁-C₆-Alkylsulfonyl; insbesondere C₁-C₆-Alkylsulfonyl;

Die Verbindungen der Formel XV können auf verschiedene Art und Weise erhalten werden, beispielsweise kann das anellierte System analog zu den bei den Verbindungen der Formel VIc beschriebenen Verfahren aufgebaut werden.

Es ist aber auch möglich von einem geeigneten Grundkörper ausgehend das anellierte System aufzubauen (in Analogie zu den bei den Verbindungen der Formel VIc beschriebenen Verfahren) und anschließend durch Nitrierung para zu R⁴ in Analogie zu literaturbekannten Verfahren eine Nitrogruppe einzuführen und diese durch Reduktion auf an sich bekannte Art und Weise in die Aminogruppe überzuführen.

Gegenbenenfalls kann es von Vorteil sein, bei den voranstehend beschriebenen Synthesevarianten für bestimmte Funktionalitäten Schutzgruppen einzuführen, wenn die Kompatibilität der Funktionalitäten mit den erforderlichen Rekationsbedingungen nicht gegeben ist.

Die Wahl der Schutzgruppen richtet sich sowohl nach den Reaktionsbedingungen als auch nach der Molekülstruktur. Die Schutzgruppen, ihre Einführung und ihre Abspaltung sind in der Regel literaturbekannt (vgl. z.B. T.W. Greene et al., "Protective Groups in Organic Synthesis", 2^{nd} edition, Wiley, New York, 1991) und sie können in Analogie zu literaturbekannten Verfahren eingesetzt werden.

Weiterhin kann es notwendig sein eine Kombination der voranstehend beschriebenen Synthesevarianten durchzuführen.

Ebenso ist es möglich durch elektrophile, nukleophile, radikalische oder organometallische Rekationen sowie durch Oxidations- oder Reduktionsreaktionen weitere Substituenten einzuführen bzw. vorhandene Substituenten zu modifizieren.

### Herstellungsbeispiele:

### 1. (5-Phenylcarbonyloxy-1-methyl-1H-pyrazol-4-yl)-(8-methylsulfonyl-3a,4-dihydro-3H-indeno[1,2-c]isoxazol-5-yl)methanon (Verbindung 2.2)

### 2-Allyl-6-chlor-benzaldehyd

Eine Lösung von 10,89 g (0,107 mol) Trimethylethylendiamin in 50 ml wasserfreiem Tetrahydrofuran wurde unter Schutzgasatmosphäre auf -10°C gekühlt und mit 66,6 ml 1,6 molarer n-Butyllithium-Lösung in Hexan (0,107 mol) tropfenweise versetzt. Nach 10 Minuten gab man 15 g (0,107 mol) 6-Chlorbenzaldehyd in 70 ml Tetrahydrofuran tropfenweise zu, versetzte mit weiteren 0,214 mol n-Butyllithium in Hexan (146,8 ml) und rührte 2,5 Stunden bei 0°C. Es wurde auf -20°C abgekühlt, 12,42 g (0,139 mol) Kupfer(I)cyanid zugegeben, 30 Minuten bei -10°C gerührt und anschließend 28,42 g Allylbromid in 100 ml Tetrahydrofuran zugetropft. Man rührte noch 2,5 Stunden bei 0°C, ehe 230 ml gesättigte Ammoniumchlorid-Lösung zugetropft wurden. Der dabei anfallende Feststoff wurde abgetrennt und die wäßrige Phase mit Diethylether extrahiert. Die vereinigten organischen Phasen wurden dann mit gesättigter Ammoniumchlorid-Lösung gewaschen, getrocknet und im Vakuum das Lösungsmittel entfernt. Man erhielt 17,0 g 2-Allyl-6-chlorbenzaldehyd (89 %) in Form eines dunklen Öls. ¹H-NMR (CDCl₃, δ in ppm): 3,73 (d, 2H); 5,05 (dd, 2H); 5,96 (m, 1H); 7,05-7,48 (m, 3H); 10,58 (s, 1H).

### 2-Allyl-6-chlor-benzaldehyd-oxim

Zu einer Lösung von 4,62 g Hydroxylamin-Hydrochlorid in 50 ml Wasser wurden 5,58 g Natriumhydrogencarbonat gegeben und auf 0°C gekühlt. Dazu tropfte man eine Lösung von 9,7 g (44,32 mmol) 2-Allyl-6-chlor-benzaldehyd in 50 ml Methanol und rührte bei Raumtemperatur über Nacht. Anschließend wurde das Methanol im Vakuum entfernt und der Rückstand in 300 ml Wasser eingerührt. Die wäßrige Phase extrahierte man mit Diethylether, die vereinigten organischen Phasen wurden mit gesättigter Ammoniumchlorid-Lösung gewaschen, getrocknet und das Lösungmittel entfernt. Man erhielt 8,7 g (quantitativ) 2-Allyl-6-chlorbenzaldehyd-oxim in Form eines zähen Öls. ¹H-NMR (CDCl₃, δ in ppm): 3,58 (d, 2H); 5,02 (2d, 2H); 5,95 (m, 1H); 7,08-7,36 (m, 3H); 8,49 (s, 1H).

### 8-Chlor-3a,4-dihydro-3H-indeno[1,2-c]isoxazol

Zu einer Lösung von 8,4 g (42,9 mmol) 2-Allyl-6-chlorbenzaldehyd-oxim in 100 ml Methylenchlorid wurden bei Raumtemperatur 37,0 ml einer Natriumhypochloritlösung (12,5 % aktives Chlor) getropft und eine Spatelspitze Natriumacetat zugegeben. Man rührte 2 Stunden bei Raumtemperatur, trennte die organische Phase ab, extrahierte die wäßrige Phase mit Methylenchlorid und wusch die vereinigten organischen Phasen mit gesättigter Ammoniumchlorid-Lösung. Es wurde getrocknet und das Lösungsmittel entfernt. Man erhielt 7,0 g (94 %) 8-Chlor-3a,4-dihydro-3H-indeno-[1,2-c]isoxazol in Form eines zähen Öls.
¹H-NMR (CDCl₃, δ in ppm): 2,81 (dd, 1H); 3,24 (dd, 1H); 3,78-4,03 (s, 2H); 4,78 (t, 1H); 7,23-7,41 (m, 3H).

### 8-Methylthio-3a,4-dihydro-3H-indeno[1,2c]isoxazol

Zu einer Lösung von 5,0 g (25,8 mmol) 8-Chlor-3a,4-dihydro-3H-indeno-[1,2-c]isoxazol in 60 ml N-Methylpyrrolidon wurden bei Raumtemperatur 3,6 g (52,0 mmol) Natriumthiomethylat gegeben und über Nacht gerührt. Anschließend rührte man in 800 ml Wasser ein, extrahierte die wäßrige Phase mit Diethylether, wusch die vereinigten organischen Phasen mit gesättigter Ammoniumchlorid-Lösung, trocknete und entfernte das Lösungsmittel. Man erhielt 4,6 g (87 %) 8-Methylthio-3a,4-dihydro-3H-indeno[1,2-c]isoxazol in Form eines dunkelbraunen Feststoffs.
¹H-NMR (CDCl₃, δ in ppm): 2,54 (s, 3H); 2,78 (dd, 1H); 3,21 (dd, 1H); 3,72-3,93 (s, 2H); 4,64 (t, 1H); 7,09-7,38 (m, 3H).

### 5-Hrom-8-methylthio-3a,4-dihydro-3H-indeno[1,2-c]isoxazol

Man kühlte 120 ml Schwefelsäure (98 proz.) auf 0°C und gab portionsweise 11,2 g (54,8 mmol) 8-Methylthio-3a,4-dihydro-3H-indeno[1,2-c]isoxazol zu. Anschließend tropfte man 9,2 g (57,5 mmol) Brom zu und rührte 2 Stunden bei 0°C weiter. Man goß die entstandene Lösung auf 2 1 eines Gemisches von Wasser und Eis, rührte 1,5 Stunden und saugte den ausgefallenen Feststoff ab, der anschließend gewaschen und dann getrocknet wurde. Man erhielt 11,4 g (73 %) 5-Brom-8-methylthio-3a,4-dihydro-3H-indeno[1,2-c]isoxazol eines braunen Feststoffs mit einem Fp. von 127-135°C.
¹H-NMR (CDCl₃, δ in ppm): 2,53 (s, 3H); 2,71 (dd, 1H); 3,24 (dd, 1H); 3,81-4,02 (s, 2H); 4,71 (t, 1H); 7,01 (d, 1H); 7,47 (d, 1H).

### 5-Brom-8-methylsulfonyl-3a,4-dihydro-3H-indeno[1,2-c]isoxazol

Eine Lösung von 11,2 g (39,4 mmol) 5-Brom-8-methylthio-3a,4-dihydro-3H-indeno[1,2-c]isoxazol und 1,55 g Natriumwolframat in 250 ml Toluol und 50 ml Eisessig wurde auf 70°C erhitzt und tropfenweise mit 10,73 g (39 proz., 86,8 mmol) Wasserstoffperoxid versetzt. Man rührte noch 3 Stunden bei 70°C weiter, wobei ein Feststoff ausfiel. Man ließ die Mischung auf Raumtemperatur abkühlen, rührte in 1 1 Wasser ein und saugte den weißen Feststoff ab. Die organische Phase des Filtrats wurde abgetrennt und die wäßrige mit Essigsäureethylester extrahiert. Die vereinigten organischen Phasen wurden mit Wasser gewaschen, getrocknet und das Lösungsmittel entfernt. Man erhielt ein zähes braunes Öl, welches mit Hexan/Essigsäureethylester (4:1) verrührt wurde. Der sich bildende Niederschlag wurde abgesaugt und mit den oben erhaltenen Feststoff vereinigt. Man erhielt 7,3 g (59 %) 5-Brom-8-methylsulfonyl-3a,4-dihydro-3H-indeno[1,2-c]-isoxazol.
¹H-NMR (d⁶-DMSO, δ in ppm): 2,93 (dd, 1H); 3,23 (dd, 1H); 3,41 (s, 3H); 3,94 (dd, 1H); 4,16 (m, 1H); 4,81 (t, 1H); 7,82 (d, 1H); 8, 03 (d, 1H).

### (5-Hydroxy-1-methyl-1H-pyrazol-4-yl)-(8-methylsulfonyl-3a,4-dihydro-3H-indeno[1,2-c]isoxazol-5-yl)methanon (Verbindung 2.1)

Zu einer Suspension von 2,0 g (6,33 mmol) 5-Brom-8-methylsulfonyl-3a,4-dihydro-3H-indeno[1,2-c]isoxazol in 100 ml Dioxan gab man 0,62 g (6,33 mmol) 5-Hydroxy-1-methyl-pyrazol, 1,75 g (12,66 mmol) trockenes Kaliumcarbonat, 1,28 g (12,67 mmol) Triethylamin und 0,22 g (0,30 mmol) Bis-(triphenylphosphan)-palladium-dichlorid. Man presste in einem Miniautoklaven 3-mal 20 bar Kohlenmonoxid auf, rührte jeweils 5 Minuten und entspannte wieder. Anschließend heizte man auf 130°C, presste wiederum 20 bar Kohlenmonoxid auf und rührte 24 Stunden. Nach dem Abkühlen und Entspannen wurde das Lösungsmittel entfernt, der Rückstand in Wasser aufgenommen, auf pH 11 eingestellt und mit Methylenchlorid gewaschen. Anschließend säuerte man mit 10 proz. Salzsäure auf pH 4 an und extrahierte mit Methylenchlorid. Die vereinigten organischen Phasen wurden mit gesättigter Ammoniumchlorid-Lösung gewaschen, getrocknet und das Lösungsmittel entfernt. Man erhielt 0,58 g (25 %) (5-Hydroxy-1-methyl-1H-pyrazol-4-yl)-(8-methylsulfonyl-3a,4-dihydro-3H-indeno[1,2-*c*]isoxazol)methanon in Form eines dunklen Öls.
¹H-NMR (CDCl₃, δ in ppm): 3,03 (dd, 1H); 3,42 (s, 3H); 3,40 (m, 1H); 3,51 (s, 3H); 4,05 (m, 2H); 4,85 (t, 1H); 7,57 (s, 1H); 7,92 (d, 1H); 8,22 (d, 1H).

### (5-Phenylcarbonyloxy-1-methyl-1H-pyrazol-4-yl)-(8-methylsulfonyl-3a,4-dihydro-3H-indeno[1,2-c]isoxazol-5-yl)methanon (Verbindung 2.2)

Zu einer Suspension von 0,55 g (1,52 mmol) (5-Hydroxy-1-methyl-1H-pyrazol-4-yl)-(8-methylsulfonyl-3a,4-dihydro-3H-indeno[1,2-c]-isoxazol-5-yl)methanon in 10 ml Tetrahydrofuran gab man unter Schutzgasatmosphäre bei 0°C 0,18 g Triethylamin und 0,26 g (1,82 mmol) Benzoylchlorid in 10 ml Tetrahydrofuran. Man rührte über Nacht bei Raumtemperatur, entfernte das Lösungsmittel, nahm den Rückstand in Essigsäureethylester auf, wusch mit Wasser, trocknete und entfernte das Lösungsmittel. Das Rohprodukt wurde durch Chromatographie an Kieselgel (Eluent: Essigsäureethylester: Hexan = 1:1) gereinigt. Man erhielt 0,22 g (31 %) (5-Phenylcarbonyloxy-1-methyl-1H-pyrazol-4-yl)-(8-methylsulfonyl-3a,4-dihydro-3H-indeno[1,2-c]isoxazol-5-yl)methanon in Form eines gelben Feststoffes mit einem Fp. von 86-93°C.
¹H-NMR (CDCl₃, δ in ppm): 3,22 (s, 3H); 3,34 (m, 2H): 3,81 (s, 3H); 3,98 (m, 2H); 4,81 (t, 1H) ; 7,20 - 8,21 (m, 8H).

### 4-(2-Methyl-9-chlor-[1]-thiochromano[4,3-c]pyrazol-6-yl) carbonyl-5-hydroxy-1-methyl-1H-pyrazol (Verbindung 3.1)

### 2-Chlorsulfonyl-4-chlor-benzoesäuremethylester

Zu einer Lösung von 139 g (0,75 mol) 2-Amino-4-chlor-benzoesäuremethylester in 400 ml konzentrierter Salzsäure wurden bei 0 bis 5°C eine Lösung von 60,9 g (0,88 mol) Natriumnitrit in 100 ml Wasser zugetropft und noch 1 Stunde bei 0°C nachgerührt.

In einer zweiten Apparatur wurden 3 g Kupfer-(II)-chlorid, 3 g Benzyltriethylammoniumchlorid, 10 ml Wasser und 400 ml 1,2-Dichlorethan vereinigt und 64 g (1 mol) Schwefeldioxid eingeleitet.

Anschließend wurde das Diazoniumsalz wie oben beschrieben bei 10 bis 15°C zugegeben und langsam auf 50°C erwärmt. Dann leitete man weitere 54 g (0,84 mol) Schwefeldioxid ein und rührte noch 30 Minuten bei 50°C. Nach Abkühlen wurden bei Raumtemperatur dann 7,4 g (0,1 mol) Chlor eingegast, 15 Minuten nachgerührt und anschließend die sich bildenden Phasen getrennt. Die organische Phase wurde getrocknet und das Lösungsmittel entfernt. Man erhielt 207 g 2-Chlorsulfonyl-4-Chlor-benzoesäuremethylester.
¹H-NMR (CDCl₃, δ in ppm): 4,00 (s, 3H); 7,75 (m, 2H); 8,18 (m, 1H)

### 2-Mercapto-4-chlor-benzoesäuremethylester

Zu einer Suspension von 205 g (0,75 mol) 2-Chlorsulfonyl-4-chlor-benzoesäuremethylester in 1 1 konzentrierter Salzsäure und 375 g Eis wurde portionsweise innerhalb von 1,5 Stunden 243,5 g (3,7 mol) Zinkpulver gegeben. Man rührte 3 Stunden nach und erhitzte langsam auf 70°C. Nach 2 Stunden bei dieser Temperatur kühlte man ab. Nach 12 Stunden stehen bei Raumtemperatur wurde das Reaktionsgemisch mit Ethylacetat extrahiert, die vereinigten organischen Phasen getrocknet und das Lösungsmittel entfernt. Man erhielt 125,4 g (83 %) 2-Mercapto-4-chlor-benzoesäuremethylester.
¹H-NMR (CDCl₃, δ in ppm): 3, 95 (s, 3H); 4,88 (s, 1H): 7,10 (m, 1H); 7,30 (m, 1H); 7,96 (d, 1H).

### 2-(2-Hydroxycarbonyl-eth-1-yl)-thio-4-chlor-benzoesäuremethylester

Zu einer Lösung von 125,4 g (0,62 mol) 2-Mercapto-4-chlorbenzoesäuremethylester in 1,5 1 Aceton wurde 179,5 g (1,3 mol) Kaliumcarbonat und portionsweise 94,5 g (0,62 mol) 3-Brompropionsäure gegeben und das Reaktionsgemisch 12 Stunden bei Raumtemperatur gerührt. Man destillierte das Lösungsmittel ab, nahm den Rückstand in Wasser auf und extrahierte mit Diethylether. Dann wurde die wäßrige Phase mit konzentrierter Salzsäure sauer gestellt, der ausgefallene Niederschlag abgesaugt und getrocknet. Man erhielt 150 g (88 %) 2-(2-Hydroxycarbonyl-eth-1-yl)-thio-4-chlor-benzosäuremethylester
Fp.: 133 bis 136°C

### 5-Chlor-4-oxo-thiochroman-8-carbonsäuremethylester

50 g (0,18 mol) 2-(2-Hydroxycarbonyl-eth-1-yl)-thio-4-chlorbenzoesäuremethylester wurden bei 70°C zu 500 g Polyphosphorsäure gegeben und noch 30 Minuten nachgerührt. Anschließend wurde das Reaktionsgemisch in Wasser eingerührt, der ausgefallene Niederschlag abgesaugt und getrocknet. Man erhielt 41,1 g (88 %) 5-Chlor-4-oxo-thiochroman-8-carbonsäuremethylester.
¹H-NMR (CDCl₃, δ in ppm): 3,08 (m, 4H); 3,96 (s, 3H); 7,14 (d, 1H); 7,95 (d, 1H).

### 5-chlor-3-(N,N-dimehtylaminomethyliden)-4-oxo-thiochroman-8-carbonsäuremethylester

30 g (0,078 mol) 5-Chlor-4-oxo-thiochroman-8-carbonsäuremethylester wurden in 300 ml N,N-Dimethylformamid-dimethylacetal 6 Stunden unter Rückfluß erhitzt. Dann wurden flüchtige Bestandteile abdestilliert, der Rückstand in Methylenchlorid aufgenommen und die organische Phase mit Wasser gewaschen. Nach Trocknung und Entfernen des Lösungsmittels erhielt man 35,3 g (97 %) 5-Chlor-3-(N,N-dimethylaminomethyliden)-4-oxothiochroman-8-carbonsäuremethylester.
¹H-NMR (CDCl₃, δ in ppm): 3,18 (s, 6H); 3,80 (s, 2H); 3,95 (s, 3H); 7,24 (d, 1H); 7,64 (s, 1H); 7,82 (d, 1H).

### 2-Methyl-6-methoxycarbonyl-9-chlor-[1]-thiochromano[4,3-c] pyrazol

Zu einer Lösung von 7,0 g (22,5 mmol) 5-Chlor-3-(N,N-dimethylaminomethyliden)-4-oxo-thiochroman-8-carbonsäuremethylester in 700 ml Ethanol wurden 1,3 g (29,2 mmol) Methylhydrazin zugetropft und 2 Stunden unter Rückfluß erhitzt. Das Lösungsmittel wurde entfernt und der Rückstand an Kieselgel mit Ethylacetat/Cyclohexan (2:3) als Eluent chromatografiert. Man erhielt 4,0 g (60 %) 2-Methyl-6-methoxycarbonyl-9-chlor-[1]-thiochromano[4,3-c]pyrazol.
¹H-NMR (CDCl₃, δ in ppm): 3,76 (s, 2H); 3,95 (s, 3H); 4,00 (s, 3H); 7,24 (s, 1H): 7,36 (d, 1H); 7,70 (d, 1H).

### 2-Methyl-6-hydroxycarbonyl-9-chlor-[1]-thiochromano[4,3-c] pyrazol

4,0 g (13,6 mmol) 2-Methyl-6-methoxycarbonyl-9-chlor-[1]-thiochromano[4,3-c]pyrazol wurden in 100 ml Methanol/Wasser (1:1) mit 0,8 g (20 mmol) Natriumhydroxid 1 Stunde unter Rückfluß erhitzt. Man entfernte das organische Lösungsmittel am Vakuum und extrahierte den Rückstand mit Ethylacetat. Die wäßrige Phase wurde mit konzentrierter Salzsäure angesäuert, der ausgefallene Niederschlag abgesaugt und getrocknet. Man erhielt 3,5 g (92 %) 2-Methyl-6-hydroxycarbonyl-9-chlor-[1]-thiochromano[4,3-c]pyrazol.
¹H-NMR (CDCl₃, δ in ppm): 3,80 (s, 2H); 3,96 (s, 3H); 7,40 (d, 1H); 7,65 (m, 2H).

### 4-(2-Methyl-9-chlor-[1]-thiochromano[4,3-c]pyrazol-6-yl)-carbonyl-5-hydroxy-1-methyl-1H-pyrazol (Verbindung 3.1)

Ein Gemisch aus 0,60 g (2,1 mmol) 2-Methyl-6-hydroxycarbonyl-9-chlor-[1]-thiochromano[4,3-c]pyrazol, 0,21 g (2,1 mmol) N,N-Dicyclohexylcarbodiimid in 20 ml Acetonitril wurden über Nacht bei Raumtemperatur gerührt. Man versetzte mit je 500 ml Ethylacetat und 2%iger Sodalösung, filtierte den ausgefallenen Niederschlag ab, trocknete die organische Phase und entfernte das Lösungsmittel. Der Rückstand wurde anschließend mit 0,59 g (4,3 mmol) Kaliumcarbonat in 5 ml 1,4-Dioxan 3 Stunden unter Rückfluß erhitzt. Nach dem Abkühlen extrahierte man mit Diethylether und säuerte die wäßrige Phase auf pH 3 an. Der ausgefallene Niederschlag wurde abgesaugt und getrocknet. Man erhielt 0,14 g 4-(2-Methyl-9-chlor-[1]-thiochromano[4,3-c]pyrazol-6-yl)-carbonyl-5-hydroxy-1-methyl-1H-pyrazol
Fp.: 168 bis 171°C

### (5-Hydroxy-1-methyl-1H-pyrazol-4-yl)-(6-methoxy-3a,4-dihydro-3H-chromeno[4,3-c]isoxazolin-9-yl)methanon (Verbindung 2.3)

### 2-Hydroxy-3-formyl-4-methoxy-benzoesäuremethylester

Zu einer Lösung von 50,1 g (0,275 mol) 2-Hydroxy-4-methoxybenzoesäuremethylester und 88 g (0,725 mol) Dichlormethoxymethan in 400 ml Methylenchlorid wurde bei 0 bis 5°C eine Lösung von 209,0 g (1,1 mol) Titantetrachlorid in 150 ml Methylenchlord getropft und über Nacht bei Raumtemperatur gerührt. Anschließend rührte man die Mischung in Eiswasser ein und extrahierte mit Methylenchlorid. Die vereinigten organischen Phasen wurden mit Natriumhydrogencarbonat-Lösung, Wasser und Natriumchlorid-Lösung gewaschen, getrocknet und anschließend das Lösungsmittel entfernt. Nach Chromatographie an Kieselgel mit Cyclohexan/Ethylacetat = 1:1 erhielt man 24,5 g (42 %) 2-Hydroxy-3-formyl-4-methoxy-benzoesäuremethylester in Form eines farblosen Feststoffes vom Fp.: 123 - 124°C.
¹H-NMR (CDCl₃, δ in ppm): 3,92 (s, 3H); 3,98 (s, 3H); 6,49 (d, 1H); 8,19 (d, 1H); 10,39 (s, 1H).

### 2-Allyloxy-3-formyl-4-methoxy-benzoesäuremethylester

Zu einer Mischung von 21,0 g (0,375 mol) Kaliumhydroxid und 20,2 g (0,096 mol) 2-Hydroxy-3-formyl-4-methoxy-benzoesäuremethylester in 500 ml Dimethylsulfoxid wurden bei Raumtemperatur 23,2 g (0,192 mol) Allylbromid getropft und 4 Stunden bei Raumtemperatur gerührt. Anschließend rührte man die Mischung in 1,5 1 3%ige wäßrige Salzsäure ein und extrahierte mit Ethylacetat. Die vereinigten organischen Phasen wurden mit Wasser gewaschen, getrocknet und das Lösungsmittel entfernt. Nach Chromatographie an Kieselgel mit Cyclohexan/ Ethylacetat = 1:2 erhielt man 7,7 g (36 %) 2-Allyloxy-3-formyl-4-methoxy-benzoesäuremethylester in Form eines gelblichen Öls.
¹H-NMR (CDCl₃, δ in ppm): 3,86 (s, 3H); 3,93 (s, 3H); 4,58 (d, 2H); 5,32 (d, 1H); 5,39 (d, 1H); 6,15 (m, 1H); 6,79 (d, 1H); 8,04 (d, 1H); 10,41 (s, 1H).

### 6-Methoxy-9-methoxycarbonyl-3a,4-dihydro-3H-chromeno[4,3-c] isoxazolin

### Stufe a)

Zu einer Lösung von 2,25g (32,3 mmol) Hydroxylammoniumchlorid und 2,7 g Pyridin in 70 ml Wasser wurden bei Raumtemperatur 4,6 g (18,4 mmol) 2-Allyloxy-3-formyl-4-methoxy-benzoesäuremethylester in 70 ml Methanol zugetropft. Man ließ über Nacht bei Raumtemperatur rühren, gab 150 ml Wasser zu, extrahierte mit Methylenchlorid, wusch die vereinigten organischen Phasen mit 3-%iger wäßriger Salzsäure, trocknete und entfernte das Lösungsmittel. Das so erhaltene Oxim hat einen Festpunkt von 126 - 129°C.

### Stufe b)

Dieses Oxim wurde ohne weitere Aufreinigung weiter umgesetzt, indem man es in 40 ml Methylenchlorid löste und 15,0 ml (25,0 mmol) Natriumhypochloridlösung (12,5 % aktives Chlor) zutropfte. Man gab eine Spatenspitze Natriumacetat zu rührte 12 Stunden bei Raumtemperatur. Die organische Phase wurde abgetrennt, die wäßrige Phase mit Methylenchlorid extrahiert und die vereinigten organischen Phasen mit Wasser gewaschen. Man trocknete und entfernte das Lösungsmittel. Nach Chromatographie an Kieselgel mit Cyclohexan/Ethylacetat = 1:1 erhielt man 2,2 g (49 %) 6-Methoxy-9-methoxycarbonyl-3a,4-dihydro-3H-chromeno[4,3-c]isoxazolin in Form eines farblosen Feststoffe vom Fp.: 199 - 203°C.
¹H-NMR (CDCl₃, δ in ppm): 3,84 (s, 3H); 3,98 (s, 3H); 3,8 - 4,0 (m, 2H); 4,16 (dt, 1H); 4,63 (t, 1H); 4,84 (dd, 1H); 6,61 (d, 1H); 7,93 (d, 1H).

### 6-Methoxy-9-hydroxycarbonyl-3a,4-dihydro-3H-chromeno[4,3-c] isoxazolin

Zu einer Lösung von 2,1 g (8,0 mmol) 6-Methoxy-9-methoxycarbonyl-3a,4-dihydro-3H-chromeno[4,3-c]isoxazolin in 40 ml Methanol tropfte man bei Raumtemperatur eine Lösung von 0,8 g (20,0 mmol) Natriumhydroxid in 7 ml Wasser und erhitzte 6 Stunden unter Rückfluß. Nach dem Abkühlen entfernte man das Lösungsmittel, der Rückstand wurde in ca. 50 ml Waser aufgenommen und mit Methylenchlorid gewaschen. Anschließend säuerte man die wäßrige Phase mit 10%-iger Salzsäure an (pH = 1 - 2), saugte den Niederschlag ab, wusch mit Wasser und trocknete bei 60°C. Man erhielt 1,7 g (86 %) 6-Methoxy-9-hydroxycarbonyl-3a,4-dihydro-3H-chromeno[4,3-c]isoxazolin in Form farbloser Kristalle.
¹H-NMR (CDCl₃, δ in ppm): 3,73 (dd, 1H); 3,89 (s, 3H); 3,84-3,95 (m, 1H); 4,11 (dd, 1H); 4,54 (dd, 1H); 4,79 (dd, 1H); 6,61 (d, 1H); 7,81 (d, 1H).

### (5-Hydroxy-1-methyl-1H-pyrazol-4-yl)-(6-methoxy-3a,4-dihydro-3H-chromeno[4,3-c]isoxazolin-9-yl)methanon (Verbindung 2.3)

### Stufe a)

Zu einer Lösung von 0,50 g (2,0 mmol) 6-Methoxy-9-hydroxycarbonyl-3a,4-dihydro-3H-chromeno[(4,3-c)]isoxazolin in 30 ml Tetrachlorkohlenstoff gab man bei Raumtemperatur 0,26 g (2,2 mmol) Thionylchlorid und einen Tropfen Dimethylformamid und rührte 3 Stunden bei 40 - 50°C. Anschließend entfernte man das Lösungsmittel im Vakuum. Man erhielt quantitativ (0,54 g) 6-Methoxy-9-chlorformyl-3a,4-dihydro-3H-chromeno[4,3-c] isoxazolin als bräunliches Öl.

### Stufe b)

Man löste 0,54 g (2 mmol) 6-Methoxy-9-chlorformyl-3a,4-dihydro-3H-chromeno[4,3-c]isoxazolin in 30 ml Acetonitril und tropfte es bei 0°C zu einer Lösung von 0,2 g (2,0 mmol) 1-Methyl-5-hydroxy-pyrazol und 0,6 g (6,0 mmol) Triethylamin in 20 ml Acetonitril. Man rührte über Nacht bei Raumtemperatur, entfernte das Lösungsmittel, nahm in Methylenchlorid auf und wusch mit Wasser. Man trocknete und destillierte das Lösungsmittel ab. Der Rückstand wurde in 30 ml Dioxan gelöst, mt 0,42 g (3,0 mmol) Kaliumcarbonat versetzt und 7 Stunden unter Rückfluß erhitzt. Nach dem Abkühlen destillierte man das Lösungsmittel im Vakuum ab, nahm den Rückstand in Wasser auf und stellte die Lösung mit 10%iger Salzsäure auf pH = 1 ein. Es wurde mit Methylenchlorid extrahiert, die vereinigten organischen Phasen getrocknet und das Lösungsmittel anschließend entfernt. Man erhielt 0,45 g (68 %) (5-Hydroxy-1-methyl-1H-pyrazol-4-yl)-(6-methoxy-3a,4-dihydro-3H-chromeno[4,3-c] isoxazolin-9-yl)methanon mit einem Fp. von 236 - 238^{°C}. ¹H-NMR (CDCl₃, δ in ppm): 3,66 (s, 3H); 3,84 - 4,2 (m, 2H); 4,02 (s, 3H); 4,12 (dd, 1H); 4,63 - 4,77 (m, 2H); 6,68 (d, 1H); 7,24 (s, 1H); 7,61 (d, 1H).

### (5-Hydroxy-1-(1,1-Dimethyleth-1-yl)-1H-pyrazol-4-yl]-[6-methoxy-3a,4-dihydro-3H-chromeno[4,3-c]isoxazolin-9-yl] methanon (Verbindung 2.4)

Man löste 0,54 g (2 mmol) 6-Methoxy-9-chlorformyl-3a,4-dihydro-3H-chromeno[4,3-c]isoxazolin in 30 ml Acetonitril und tropfte es bei 0°C zu einer Lösung von 0,28 g (2,0 mmol) 1-(1,1-Dimethyleth-1-yl)-5-hydroxy-1H-pyrazol und 0,6 g (6,0 mmol) Triethylamin in 20 ml Acetonitril. Man rührte über Nacht bei Raumtemperatur, entfernte das Lösungsmittel, nahm in Methylenchlorid auf und wusch mit Wasser. Man trocknete und destillierte das Lösungsmittel ab. Der Rückstand wurde in 30 ml Dioxan gelöst, mit 0,42 g (3,0 mmol) Kaliumcarbonat versetzt und 7 Stunden unter Rückfluß erhitzt. Nach Abkühlen destillierte man das Lösungsmittel in Vakuum ab, nahm den Rückstand in Wasser auf und stellte die Lösung mit 10%-iger Salzsäure auf pH = 1 ein. Es wurde mit Methylenchlorid extrahiert, die vereinigten organischen Phasen getrocknet und das Lösungsmittel anschließend entfernt. Man erhielt 0,3 g (40 %) [5-Hydroxy-1-(1,1-Dimethyleth-1-yl)-1H-pyrazol-4-yl]-[6-methoxy-3a,4-dihydro-3H-chromeno[4,3-c]isoxazolin-9-yl]methanon mit einem Festpunkt von 223°C - 225°C.
¹H-NMR (CDCl₃, δ in ppm): 1,64 (s, 9H); 3,8 - 4,2 (m, 6H); 4,6 - 4,8 (m, 2H); 6,68 (d, 1H); 7,44 (s, 1H); 7,62 (d, 1H).

In den Tabellen 2 bis 5 sind neben den voranstehenden Verbindungen noch weitere tricyclische Benzoylpyrazol-Derivate der Formel I aufgeführt, die in analoger Weise hergestellt wurden oder herstellbar sind:

**Tabelle 2:**

| 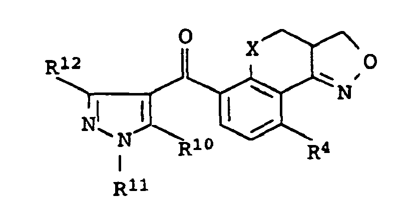 | | | | | | |
|---|---|---|---|---|---|---|
| Ia mit 1 = O, R⁵ = H, Y bildet gemeinsam mit den beiden Kohlenstoffen, an die es gebunden ist, folgendes Isoxazolin aus: | | | | | | 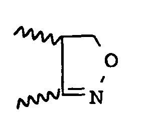 |
| Nr. | X | R⁴ | R¹⁰ | R¹¹ | R¹² | physikalische Daten (Fp. [°C]; ¹H-NMR [ppm] |
| 2.1 | Bindung | SO₂CH₃ | OH | CH₃ | H | 3,03 (dd, 1H); 3,42 (s,3H); 3,51 (s, 3H); 4,05 (m, 2H); 4,85 (t, 1H); 7,57 (s, 1H); 7,92 (d, 1H); 8,22 (d, 1H) |
| 2.2 | Bindung | SO₂CH₃ | OCOC₆H₅ | CH₃ | H | 3,22 (s, 3H); 3,34 (m, 2H); 3,81 (s, 3H); 3,98 (m, 2H); 4,81 (t, 1H); 7,20 - 8,21 (m, 8 H); |
| 2.3 | O | OCH₃ | OH | CH3 | H | 236 - 238 |
| 2.4 | O | OCH₃ | OH | C(CH₃)₃ | H | 223 - 225 |
| 2.5 | O | OCH₃ | OCO(3-F-C₆H₄) | CH₃ | H | Öl |

**Tabelle 3:**

| | | | | | | | |
|---|---|---|---|---|---|---|---|
| 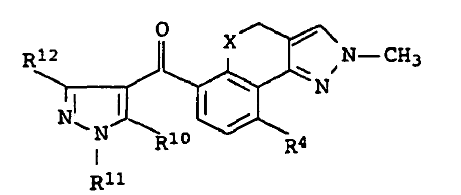 | | | | | | | |
| Ia mit R⁵ = H, Y bildet gemeinsam mit den beiden Kohlenstoffen, an die es gebunden ist, folgendes Methyl-substituierte Pyrazol aus: | | | | | | 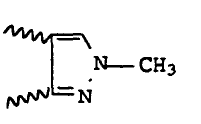 | |

| Nr. | X | R⁴ | R¹⁰ | R¹¹ | R¹² | physikalische Daten (Fp. [°C]) | |
|---|---|---|---|---|---|---|---|
| 3.1 | S | Cl | OH | CH₃ | H | 168 - 171 | |
| 3.2 | S | Cl | OH | CH₂CH₃ | H | 115 | |
| 3.3 | S | SCH₃ | OH | CH3 | H | 245 | |
| 3.4 | S | SCH₃ | OH | CH₂CH₃ | H | 222 | |

**Tabelle 4:**

| | | | | | | | |
|---|---|---|---|---|---|---|---|
| 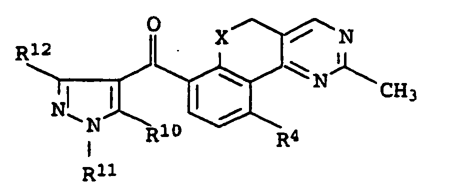 | | | | | | | |
| Ia mit R⁵ = H, Y bildet gemeinsam mit den beiden Kohlenstoffen, an die es gebunden ist, folgendes Methyl-substituierte Pyrimidin aus: | | | | | | | 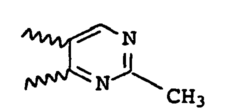 |

| Nr. | X | R⁴ | R¹⁰ | R¹¹ | R¹² | physikalische Daten (Fp. [°C]; ¹H-NMR [ppm] | |
|---|---|---|---|---|---|---|---|
| 4.1 | S | Cl | OH | CH₃ | H | 180°C | |
| 4.2 | S | Cl | OH | CH₂CH₃ | H | 112°C | |

**Tabelle 5:**

| | | | | | | |
|---|---|---|---|---|---|---|
| 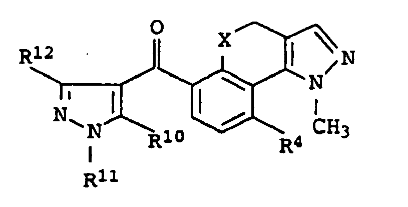 | | | | | | |

| Nr. | X | R⁴ | R¹⁰ | R¹¹ | R¹² | physikalische Daten (Fp. [°C]; ¹H-NMR [ppm] |
|---|---|---|---|---|---|---|
| 5.1 | O | SCH₃ | OH | CH₃ | H | 201 |

Die Verbindungen der Formel I und deren landwirtschaftlich brauchbaren Salze eignen sich sowohl als Isomerengemische als auch in Form der reinen Isomeren - als Herbizide. Die herbiziden Mittel, die Verbindungen der Formel I enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht:

Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline und deren Derivate, alkylierte Benzole oder deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die tricyclischen Benzoylpyrazol-Derivate der Formel I als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
- I.: 20 Gewichtsteile der Verbindung Nr. 2.2 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
- II.: 20 Gewichtsteile der Verbindung Nr. 3.1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
- III.: 20 Gewichtsteile der Verbindung Nr. 2.3 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
- IV.: 20 Gewichtsteile der Verbindung Nr. 2.4 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
- V.: 3 Gewichtsteile der Verbindung Nr. 2.3 werden mit 97 Gewichtsteilen feinteiligem Kaolin vermischt. Man erhält auf diese Weise ein Stäubemittel, das 3 Gew.-% des Wirkstoffs enthält.
- VI.: 20 Gewichtsteile der Verbindung Nr. 2.4 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
- VII.: 1 Gewichtsteil der Verbindung Nr. 2.2 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- VIII.: 1 Gewichtsteil der Verbindung Nr. 3.1 wird in einer Mischung gelöst, die aus 80 Gewichtsteilen Cyclohexanon und 20 Gewichtsteilen Wettol^{R} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0.001 bis 3.0, vorzugsweise 0.01 bis 1.0 kg/ha aktive Substanz (a.S.).

Zur Verbreiterung des Wirkungsspektrums und zur Erzielung synergistischer Effekte können die tricyclischen Benzoylpyrazol-Derivate der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-Aroyl-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der tricyclischen Benzoylpyrazol-Derivate der Formel I ließ sich durch die folgenden Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,5 bzw. 0,25 kg/ha a.S..

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Chenopodium album | Weißer Gänsefuß | lambsquarters (goosefoot) |
| Echinochloa crusgalli | Hühnerhirse | barnyardgrass |
| Setaria viridis | Grüne Borstenhirse | green foxtail |
| Solanum nigrum | Schwarzer Nachtschatten | black nightshade |
| Veronica ssp. | Ehrenpreisarten | speadwell |

Bei Aufwandmengen von 0,5 bzw. 0,25 kg/ha zeigt die Verbindung 2.2 im Nachauflauf eine sehr gute Wirkung gegen die oben genannten unerwünschten Unkräuter und Gräser.

## Patentansprüche

1. Tricyclische Benzoylpyrazol-Derivate der Formel I 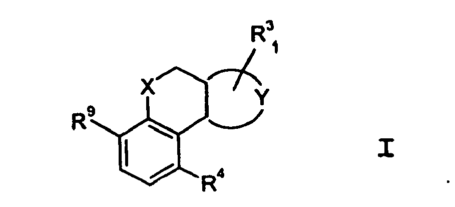 in der die Variablen folgende Bedeutungen haben:
X Sauerstoff, Schwefel oder eine Bindung;
Y bildet gemeinsam mit den beiden Kohlenstoffen, an die es gebunden ist, nachfolgende Heterocyclen aus: 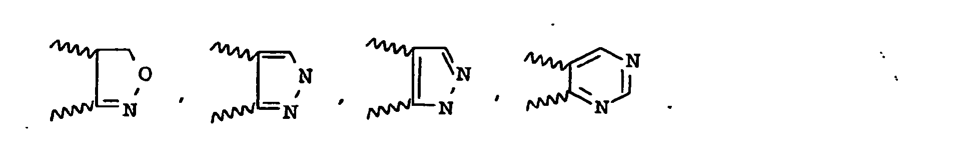
R³ C₁-C₆-Alkyl;
R⁴ Halogen, C₁-C₆-Alkoxy, C₁-C₆-Alkylthio oder C₁-C₆-Alkylsulfonyl;
l 0 oder 1;
R⁹ ein Rest IIa 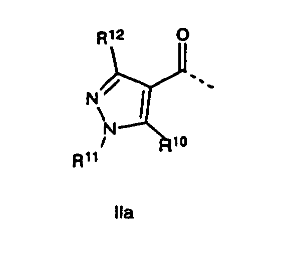 wobei
R¹⁰ Hydroxy oder OR¹³,
R¹¹ C₁-C₆-Alkyl oder C₃-C₆-Cycloalkyl,
R¹² Wasserstoff oder C₁-C₆-Alkyl,
R¹³ Phenylcarbonyl, wobei der Phenyl-Rest partiell oder vollständig halogeniert sein kann und/oder einen bis drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
sowie deren landwirtschaftlich brauchbaren Salze.

2. Tricyclische Benzoylpyrazol-Derivate der Formel I gemäß Anspruch 1 wobei
R¹⁰ Hydroxy;
R¹¹ C₁-C₆-Alkyl oder C₃-C₆-Cycloalkyl;
R¹² Wasserstoff oder C₁-C₆-Alkyl;
bedeuten.

3. Verfahren zur Herstellung von Verbindungen der Formel I mit R¹⁰ = OR¹³, gemäß Anspruch 1 **dadurch gekennzeichnet, daß** man ein tricyclisches Benzoylpyrazol-Derivat der Formel Iα, (= I mit R¹⁰ = Hydroxy), 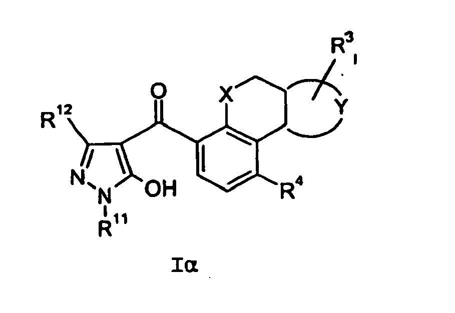 wobei die Variablen R³ und R⁴, R¹¹ und R¹², X, Y und l die in Anspruch 1 genannte Bedeutung haben, mit einer Verbindung der Formel III,
L¹-R¹³ III
wobei die Variable R¹³ die in Anspruch 1 genannte Bedeutung hat und L¹ für eine nucleophil verdrängbare Abgangsgruppe steht, umsetzt.

4. Verfahren zur Herstellung von Verbindungen der Formel I mit R¹⁰ = OR¹³ gemäß Anspruch 1, **dadurch gekennzeichnet, daß** man eine Verbindung der Formel Iβ (≡ I mit R¹⁰ = Halogen), 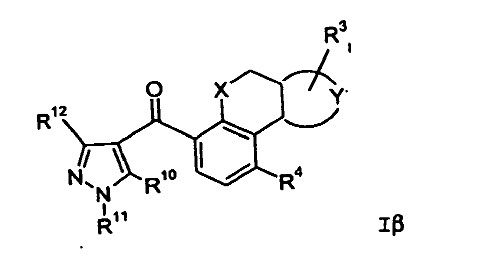 wobei die Variablen R³ und R⁴, R¹¹ und R¹², X, Y und l die in Anspruch 1 genannte Bedeutung haben, mit einer Verbindung der Formel IVα
HOR¹³ IVα
wobei die Variable R¹³ die in Anspruch 1 genannte Bedeutung hat, gegebenenfalls in Gegenwart einer Base, umsetzt.

5. Verfahren zur Herstellung von Verbindungen der Formel I mit gemäß Anspruch 1, **dadurch gekennzeichnet, daß** man ein metalliertes Pyrazol-Derivat der Formel V, wobei M für ein Metall steht und R¹⁰ bis R¹² die in Anspruch 1 genannte Bedeutung haben mit Ausnahme von R¹⁰ = Hydroxy mit einem tricyclischen Benzoesäure-Derivat der Formel VIα, wobei R³ und R⁴ X, Y und l die in Anspruch 1 genannte Bedeutung haben und L² für eine nucleophil verdrängbare Abgangsgruppe steht, umsetzt.

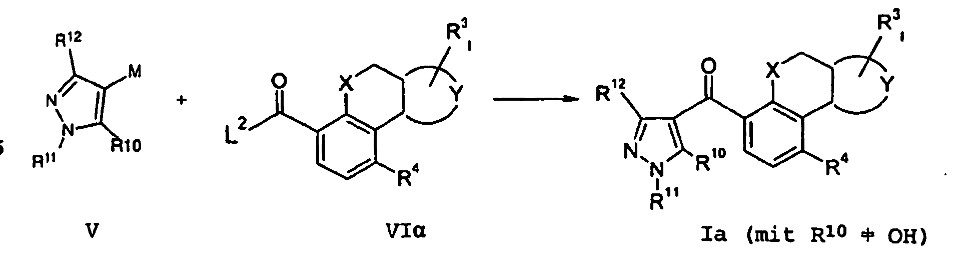

6. Verfahren zur Herstellung von tricyclischen Benzoylpyrazol-Derivaten der Formel Iα (= I mit R¹⁰ = Hydroxy) gemäß Anspruch 1, **dadurch gekennzeichnet, daß** man ein Pyrazol der Formel VII, in der die Variablen R¹¹ und R¹² die unter Anspruch 1 genannte Bedeutung haben, 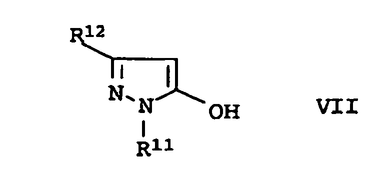 mit einer aktivierten tricyclischen Benzoesäure der Formel VIβ oder mit einer tricyclischen Benzoesäure VIγ, 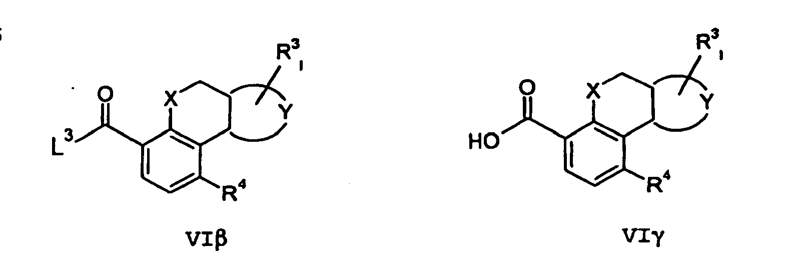 wobei die Variablen R³ und R⁴, X, Y und 1 die unter Anspruch 1 genannte Bedeutung haben und L³ für eine nucleophil verdrängbare Abgangsgruppe steht, acyliert und das Acylierungsprodukt gegebenenfalls in Gegenwart eines Katalysators umlagert.

7. Verfahren zur Herstellung von tricyclischen Benzoylpyrazol-Derivaten der Formel Iα (≡ I mit R¹⁰ = Hydroxy), gemäß Anspruch 1, **dadurch gekennzeichnet, daß** man ein Pyrazol der Formel VII, in der die Variablen R¹¹ und R¹² die in Anspruch 1 genannte Bedeutung haben, oder ein Alkalisalz hiervon, 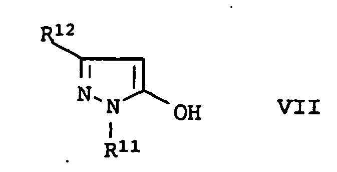 mit einem tricyclischen Benzolderivat der Formel IX, wobei L⁴ für eine Abgangsgruppe steht und die Variablen X, Y, R³ und R⁴ und l die in Anspruch 1 genannte Bedeutung haben, 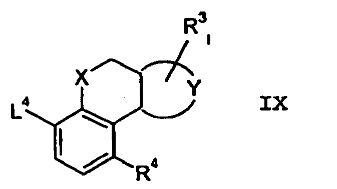 in Gegenwart von Kohlenmonoxid, eines Katalysators sowie einer Base umsetzt.

8. Mittel, enthaltend eine herbizid wirksame Menge mindestens eines tricyclischen Benzoylpyrazol-Derivates der Formel I oder eines landwirtschaftlich brauchbaren Salzes von I gemäß den Ansprüchen 1 oder 2 und für die Formulierung von Pflanzenschutzmitteln übliche Hilfsmittel.

9. Verfahren zur Herstellung von Mitteln gemäß Anspruch 8, **dadurch gekennzeichnet, daß** man eine herbizid wirksame Menge mindestens eines tricyclischen Benzoylpyrazol-Derivates der Formel I oder eines landwirtschaftlich brauchbaren Salzes von I gemäß den Ansprüchen 1 oder 2 und für die Formulierung von Pflanzenschutzmitteln übliche Hilfsmittel mischt.

10. Verfahren zur Bekämpfung von unerwünschtem Pflanzenwuchs, **dadurch gekennzeichnet, daß** man eine herbizid wirksame Menge mindestens eines tricyclischen Benzoylpyrazol-Derivates der Formel I oder eines landwirtschaftlich brauchbaren Salzes von I gemäß den Ansprüchen 1 oder 2 auf Pflanzen, deren Lebensraum und/oder auf Samen einwirken läßt.

11. Verwendung von tricyclischen Benzoylpyrazol-Derivaten der Formel I oder deren landwirtschaftlich brauchbaren Salze gemäß den Ansprüchen 1 oder 2 als Herbizide.

12. Tricyclische Benzosäurederivate der Formel VI 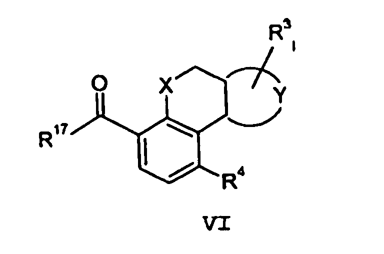 in der die Variablen X, Y, R³ und R⁴ sowie 1 die in Anspruch 1 genannte Bedeutung haben und
R¹⁷ Hydroxy oder abhydrolisierbarer Rest;
bedeutet.

13. Tricyclische Benzolderivate der Formel IX 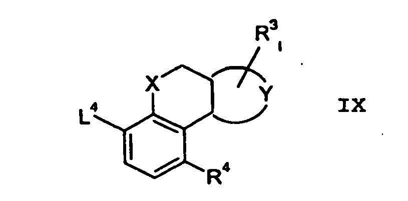 in der die Variablen X, Y, R³ sowie l die in Anspruch 1 genannte Bedeutung haben und
R⁴ Halogen, C₁-C₆-Alkylthio, oder C₁-C₆-Alkylsulfonyl;
L⁴ Halogen, C₁-C₆-Alkylsulfonyloxy, C₁-C₆-Halogenalkylsulfonyloxy oder Phenylsulfonyloxy, wobei der Phenylring des letztgenannten Rests unsubstituiert sein kann oder partiell oder vollständig halogeniert und/oder einen bis drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
bedeutet.

14. Aniline der Formel XV, 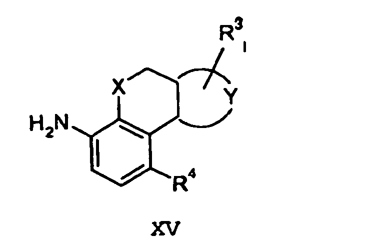 in der die Variablen X, Y, R³ und R⁴ sowie l jeweils die in Anspruch 1 genannte Bedeutung haben.

15. Nitrile der Formel XVI 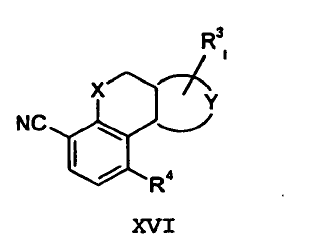 in der die Variablen X, Y, R³ sowie l jeweils die in Anspruch 1 genannte Bedeutung haben und
R⁴ Halogen, C₁-C₆-Alkylthio oder C₁-C₆-Alkylsulfonyl;
bedeuten.

## Claims

1. A tricyclic benzoylpyrazole derivative of the formula I 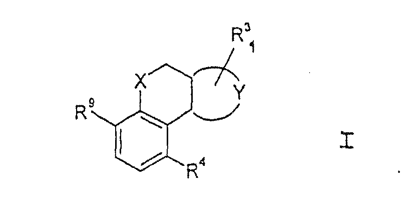 where:
X is oxygen, sulfur or a bond;
Y together with the two carbons to which it is attached forms the following heterocycles: 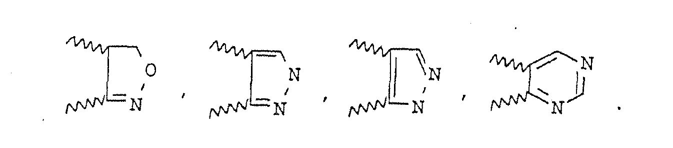
R³ is C₁-C₆-alkyl;
R⁴ is halogen, C₁-C₆-alkoxy, C₁-C₆-alkylthio, or C₁-C₆-alkylsulfonyl;
l is 0 or 1;
R⁹ is a radical IIa 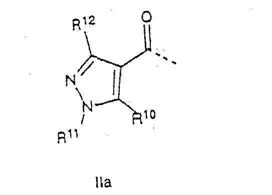 where:
R¹⁰ is hydroxyl or OR¹³,
R¹¹ is C₁-C₆-alkyl or C₃-C₆-cycloalkyl,
R¹² is hydrogen or C₁-C₆-alkyl,
R¹³ is phenylcarbonyl, wherein the phenyl radical may be partially or completely halogenated and/or may carry one to three of the following radicals: nitro, cyano, C₁-C₄-alkyl, C₁-C₄-haloalkyl, C₁-C₄-alkoxy or C₁-C₄-haloalkoxy;
Or an agriculturally useful salt thereof.

2. The tricyclic benzoylpyrazole derivative of the formula I according to claim 1 where
R¹⁰ is hydroxyl;
R¹¹ is C₁-C₆-alkyl or C₃-C₆-cycloalkyl;
R¹² is hydrogen or C₁-C₆-alkyl.

3. A process for preparing compounds of the formula I where R¹⁰ = OR¹³ according to claim 1, which comprises reacting a tricyclic benzoylpyrazole derivative of the formula Iα (= I where R¹⁰ = hydroxyl), 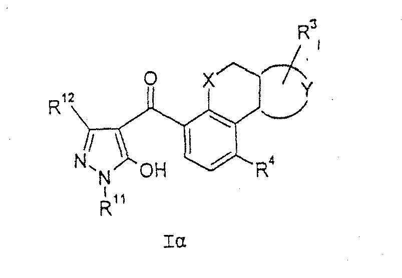 where the variables R³ and R⁴, R¹¹ and R¹², X, Y and 1 are as defined in claim 1, with a compound of the formula III
L¹-R¹³ III
where the variable R¹³ is as defined in claim 1 and L¹ is a nucleophilically replaceable leaving group.

4. A process for preparing compounds of the formula I where R¹⁰ = OR¹³, according to claim 1, which comprises reacting a compound of the formula Iβ (≡ I where R¹⁰ = halogen), 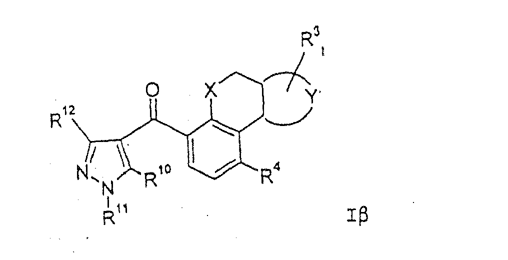 where the variables R³ and R4, R¹¹ and R¹², X, Y and 1 are as defined in claim 1, with a compound of the formula IVα,
HOR¹³ IVα
where the variable R¹³ is as defined in claim 1, if appropriate in the presence of a base.

5. A process for preparing compounds of the formula I where according to claim 1 which comprises reacting a metalated pyrazole derivative of the formula V where M is a metal and R¹⁰ to R¹² are as defined in claim 1, except for R¹⁰ = hydroxyl, with a tricyclic benzoic acid derivative of the formula VIα where R³ and R⁴, X, Y and l are as defined in claim 1 and L² is a nucleophilically replaceable leaving group.

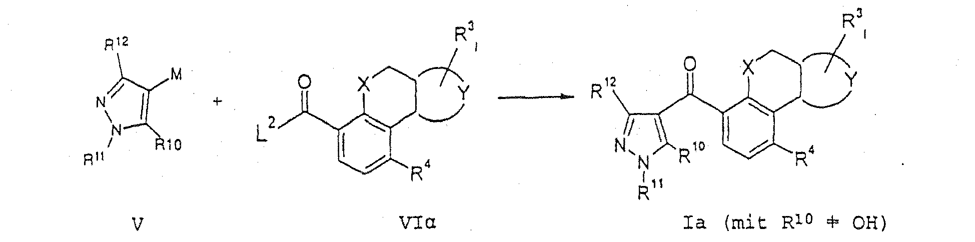

6. A process for preparing tricyclic benzoylpyrazole derivatives of the formula Iα (= I where R¹⁰ = hydroxyl) according to claim 1, which comprises acylating a pyrazole of the formula VII in which the variables R¹¹ and R¹² are as defined in claim 1 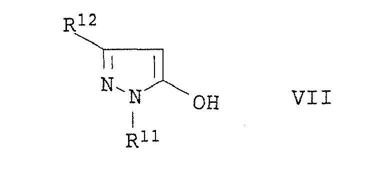 with an activated tricyclic benzoic acid of the formula VIβ or with a tricyclic benzoic acid VIγ, 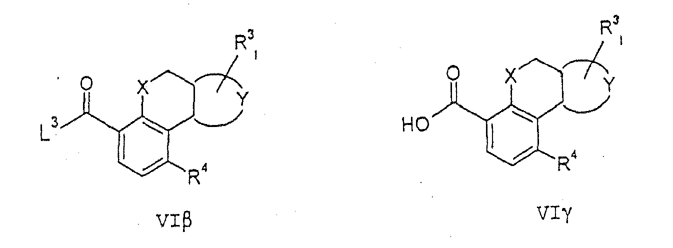 where the variables R³ and R⁴, X, Y and 1 are as defined in claim 1 and L³ is a nucleophilically replaceable leaving group, and rearranging the acylation product, if appropriate in the presence of a catalyst.

7. A process for preparing tricyclic benzoylpyrazole derivatives of the formula Iα (≡ I where R¹⁰ = hydroxyl) according to claim 1, which comprises reacting a pyrazole of the formula VII in which the variables R¹¹ and R¹² are as defined in claim 1, or an alkali metal salt thereof, 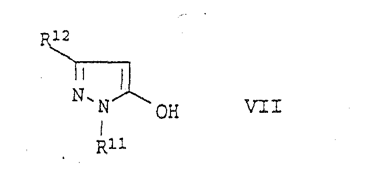 with a tricyclic benzene derivative of the formula IX where L⁴ is a leaving group and the variables X, Y, R³ and R⁴ and l are as defined in claim 1 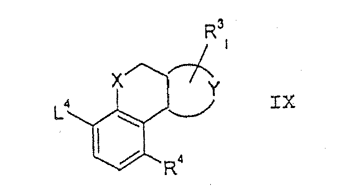 in the presence of carbon monoxide, a catalyst and a base.

8. A composition, comprising a herbicidally effective amount of at least one tricyclic benzoylpyrazole derivative of the formula I or an agriculturally useful salt of I according to claim 1 or 2 and auxiliaries which are customary for formulating crop protection agents.

9. A process for preparing compositions according to claim 8, which comprises mixing a herbicidally effective amount of at least one tricyclic benzoylpyrazole derivative of the formula I or an agriculturally useful salt of I according to claim 1 or 2 and auxiliaries which are customary for formulating crop protection agents.

10. A method for controlling undesirable vegetation, which comprises allowing a herbicidally effective amount of at least one tricyclic benzoylpyrazole derivative of the formula I or an agriculturally useful salt of I according to claim 1 or 2 to act on plants, their habitat and/or on seed.

11. The use of tricyclic benzoylpyrazole derivatives of the formula I or their agriculturally useful salts according to claim 1 or 2 as herbicides.

12. A tricyclic benzoic acid derivative of the formula VI 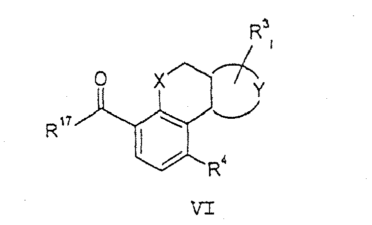 in which the variables X, Y, R³ and R⁴ and l are as defined in claim 1 and
R¹⁷ is hydroxyl or a radical which can be removed by hydrolysis.

13. A tricyclic benzene derivative of the formula IX 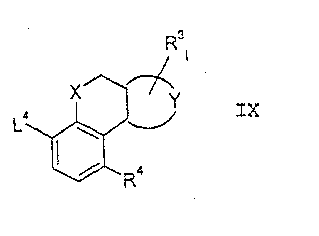 in which the variables X, Y, R³ and l are as defined in claim 1 and
R⁴ is halogen, C₁-C₆-alkylthio or C₁-C₆-alkylsulfonyl;
L⁴ is halogen, C₁-C₆-alkylsulfonyloxy, C₁-C₆-haloalkylsulfonyloxy or phenylsulfonyloxy, where the phenyl ring of the lastmentioned radical may be unsubstituted or partially or fully halogenated and/or may carry one to three of the following radicals: nitro, cyano, C₁-C₄-alkyl, C₁-C₄-haloalkyl, C₁-C₄-alkoxy or C₁-C₄-haloalkoxy.

14. An aniline of the formula XV 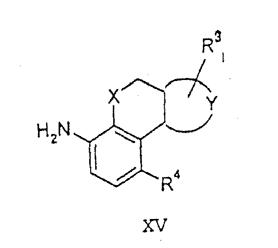 in which the variables X, Y, R³ and R⁴ and l are in each case as defined in claim 1.

15. A nitrile of formula XVI 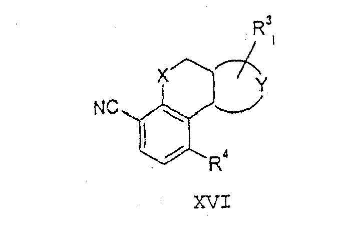 in which the variables X, Y, R³ and l are in each case as defined in claim 1 and
R⁴ is halogen, C₁-C₆-alkylthio, C₁-C₆-alkylsulfonyl.

## Revendications

1. Dérivés tricycliques de benzoylpyrazol de formule I 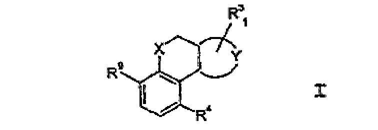 dans laquelle les variables ont les significations suivantes :
X oxygène, soufre ou une liaison ;
Y forme, en association avec les deux hydrocarbures auxquels il est lié, les hétérocycles suivants : 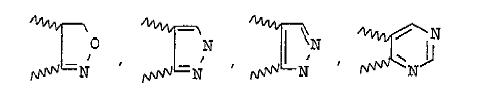
R³ alkyle en C₁-C₆;
R⁴ halogène, alcoxy en C₁-C₆, alkylthio en C₁-C₆ ou alkylsulfonyle en C₁-C₆;
l 0 ou 1;
R⁹ un radical IIa dans lequel
R¹⁰ hydroxy ou OR¹³,
R¹¹ alkyle en C₁-C₆ ou cycloalkyle en C₃-C₆,
R¹² hydrogène ou alkyle en C₁-C₆,
R¹³ phénylcarbonyle, le radical phényle pouvant être partiellement ou complètement halogéné et/ou pouvant porter un à trois des radicaux suivants : nitro, cyano, alkyle en C₁-C₄, halogénoalkyle en C₁-C₄, alcoxy en C₁-C₄ ou halogénoalcoxy en C₁-C₄ ;
ainsi que leurs sels utilisables en agriculture.

2. Dérivés tricycliques de benzoylpyrazol de formule I selon la revendication 1 dans laquelle
R¹⁰ signifie hydroxy;
R¹¹ signifie alkyle en C₁-C₆ ou cycloalkyle en C₃-C₆;
R¹² signifie hydrogène ou alkyle en C₁-C₆.

3. Procédé de préparation de composés de formule I avec R¹⁰ = OR¹³, selon la revendication 1 **caractérisé en ce qu'**un dérivé tricyclique de benzoylpyrazol de formule Iα, (= I avec R¹⁰ = hydroxy), 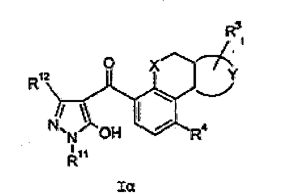 dans laquelle les variables R³ et R⁴, R¹¹ et R¹², X, Y et l ont la signification mentionnée dans la revendication 1, réagit avec un composé de formule III,
L¹-R¹³ III
dans laquelle la variable R¹³ a la signification mentionnée dans la revendication 1 et L¹ est un groupe de départ nucléophile déplaçable.

4. Procédé de préparation de composés de formule I avec R¹⁰ = OR¹³ selon la revendication 1, **caractérisé en ce qu'**un composé de formule Iβ (≡ I avec R¹⁰ = halogène), 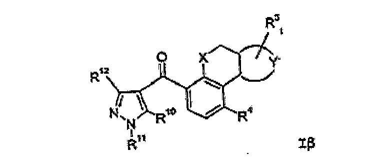 dans laquelle les variables R³ et R⁴, R¹¹ et R¹², X, Y et l ont la signification mentionnée dans la revendication 1, réagit avec un composé de formule IVα
HOR¹³ IVα
dans laquelle la variable R¹³ a la signification mentionnée dans la revendication 1, le cas échéant en présence d'une base.

5. Procédé de préparation de composés de formule I selon la revendication 1, **caractérisé en ce qu'**un dérivé métallisé de pyrazole de formule V, dans laquelle M est un métal et R¹⁰ à R¹² ont la signification mentionnée dans la revendication 1 avec l'exception de R¹⁰ = hydroxy, réagit avec un dérivé tricyclique d'acide benzoïque de formule VIα, dans laquelle R³ et R⁴, X, Y et lont la signification mentionnée dans la revendication 1 et L² est un groupe de départ nucléophile déplaçable.

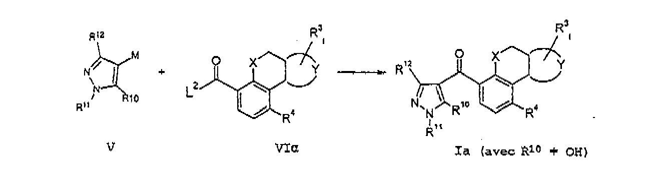

6. Procédé de préparation de dérivés tricycliques de benzoylpyrazol de formule Iα (= I avec R¹⁰ = hydroxy) selon la revendication 1, **caractérisé en ce qu'**un pyrazole de formule VII, dans laquelle les variables R¹¹ et R¹² ont la signification mentionnée dans la revendication 1, 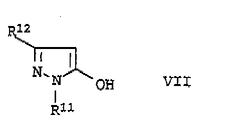 est acylé avec un acide benzoïque tricyclique activé de formule VIβ ou avec un acide benzoïque tricyclique de formule VIγ, 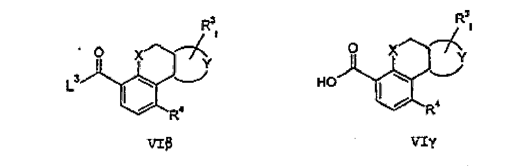 dans lesquelles les variables R³ et R⁴, X, Y et l ont la signification mentionnée dans la revendication 1 et L³ est un groupe de départ nucléophile déplaçable, et **en ce que** le produit d'acylation est réarrangé le cas échéant en présence d'un catalyseur.

7. Procédé de préparation de dérivés tricycliques de benzoylpyrazol de formule Iα (= I avec R¹⁰ = hydroxy), selon la revendication 1, **caractérisé en ce qu'**un pyrazole de formule VII, dans laquelle les variables R¹¹ et R¹² ont la signification mentionnée dans la revendication 1, ou un sel alcalin de celui-ci, 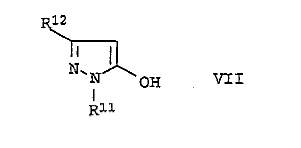 réagit avec un dérivé tricyclique de benzol de formule IX, dans laquelle L⁴ est un groupe de départ et les variables X, Y, R³ et R⁴ et l ont 1a signification mentionnée dans la revendication 1, 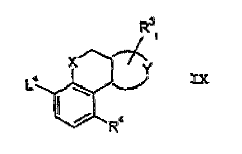 en présence de l'oxyde de carbone, d'un catalyseur ainsi que d'une base.

8. Agent, contenant une quantité active de manière herbicide d'au moins un dérivé tricyclique de benzoylpyrazol de formule I ou un sel de la formule I selon les revendications 1 ou 2 utilisable en agriculture et des adjuvants usuels pour la formulation d'agents phytosanitaires.

9. Procédé de préparation d'agents selon la revendication 8, **caractérisé en ce qu'**une quantité active de manière herbicide d'au moins un dérivé tricyclique de benzoylpyrazol de formule I ou un sel de formule I selon les revendications 1 ou 2 utilisable en agriculture et des adjuvants usuels pour la formulation d'agents phytosanitaires sont mélangés.

10. Procédé de lutte contre la croissance indésirable de plantes, **caractérisé en ce qu'**une quantité active de manière herbicide d'au moins un dérivé tricyclique de benzoylpyrazol de formule I ou un sel de formule I selon les revendications 1 ou 2 utilisable en agriculture est laissé à agir sur des plantes, leur espace vital et/ou sur des semences.

11. Utilisation de dérivés tricycliques de benzoylpyrazol de formule I ou leurs sels selon les revendications 1 ou 2 utilisables en agriculture en tant qu'herbicides.

12. Dérivés tricycliques d'acide benzoïque de formule VI 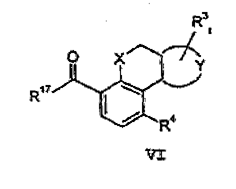 dans laquelle les variables X, Y, R³ et R⁴ ainsi que l ont la signification mentionnée dans la revendication 1 et
R¹⁷ signifie hydroxy ou radical pouvant être éliminé par hydrolyse.

13. Dérivés tricycliques de benzol de formule IX 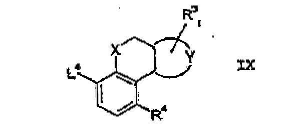 dans laquelle les variables X, Y, R³ ainsi que l ont la signification mentionnée dans la revendication 1 et
R⁴ signifie halogène, alkylthio en C₁-C₆, ou alkylsulfonyle en C₁-C₆;
L⁴ signifie halogène, alkylsulfonyloxy en C₁-C₆, halogénoalkylsulfonyloxy en C₁-C₆ ou phénylsulfonyloxy, le cycle phényle de ce dernier radical pouvant être non substitué ou partiellement ou complètement halogéné et/ou pouvant porter un à trois des radicaux suivants : nitro, cyano, alkyle en C₁-C₄, halogénoalkyle en C₁-C₄, alcoxy en C₁-C₄ ou halogénoalcoxy en C₁-C₄.

14. Anilines de formule XV, 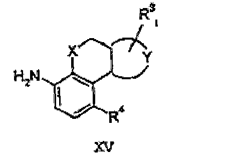 dans laquelle les variables X, Y, R³ et R⁴ ainsi que l ont respectivement la signification mentionnée dans la revendication 1.

15. Nitriles de formule XVI 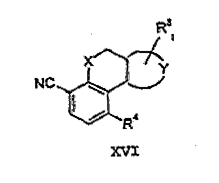 dans laquelle les variables X, Y, R³ ainsi que l ont respectivement la signification mentionnée dans la revendication 1 et
R⁴ signifie halogène, alkylthio en C₁-C₆ ou alkylsulfonyle en C₁-C₆.
